(19) Europäisches Patentamt
European Patent Office
Office européen des brevets

(11) Publication number: 0 021 883
A2

(12) # EUROPEAN PATENT APPLICATION

(21) Application number: 80400738.3

(22) Date of filing: 27.05.80

(51) Int.Cl.$^3$: C 07 D 207/16
C 07 D 209/48, C 07 C 103/46
C 07 D 409/06, A 61 K 31/40
A 61 K 31/195
//C07C69/34, C07C69/63,
C07C69/38, C07C69/734,
C07C101/34, C07C153/00,
C07C59/58, C07C121/407,
C07F7/08, C07D339/08

(30) Priority: 30.05.79 US 43864

(43) Date of publication of application:
07.01.81 Bulletin 81/1

(84) Designated Contracting States:
BE CH DE FR IT LI NL

(71) Applicant: MERRELL TORAUDE ET COMPAGNIE
16 Rue d'Ankara
F-67084 Strasbourg(FR)

(72) Inventor: Bey, Philippe
8 rue du Stockholm
F-67000 Strasbourg(FR)

(72) Inventor: Metcalf, Brian Walter
481 Clubhouse Drive
Mason Ohio 45040(US)

(72) Inventor: Wiseman, Jeffrey Stewart
12177 Cedar Drive
Loveland Ohio 45140(US)

(74) Representative: Burford, Anthony Frederick et al,
W.H. Beck, Greener & Co. 7 Stone Buildings Lincoln's Inn
London WC2A 3SZ(GB)

(54) **N-acyl-amino acid derivatives and pharmaceutical compositions containing them, intermediates and starting compounds.**

(57) Novel N-acyl amino acid derivatives are irreversible inhibitors of angiotensin-converting enzyme and have the following general Formula 1:-

```
    X    R1   O
    |    |    ||
Z - CH - CH - C - R2        Formula 1
```

wherein:-

Z represents an electron-withdrawing group, preferably cyano or a carbonyl-terminated functional group;

X represents a group which, in combination with Z, can form a Michael acceptor after abstraction of the proton from the carbon atom adjacent to X, and preferably X is ethynyl or a fluoromethyl group;

$R_1$ represents hydrogen, alkyl or phenylalkyl; and

$R_2$ represents an aminocarboxylic acid residue obtained by removal of a hydrogen atom from the amino group thereof.

Novel intermediates include compounds of the following general Formula 21:-

$$C_6H_5CH_2O_2CCH_2CO_2A$$

wherein A represents *tert*-butyl, dibenzyl or trityl, and compounds of the following general Formula 36 (being the combination of Formulae 29 and 35 herein):-

```
      R10       X1   R1
      |         |    |
H2N - CH.OC -  CH -  CH - COR9
                 Formula 36
```

wherein $X_1$ represents ethynyl or mono-, di- or tri-fluoromethyl;

$R_1$ is a defined above;

$R_9$ represents hydroxy, alkoxy or amino; and

$R_{10}$ represents hydrogen, alkyl, phenylalkyl, phenyl or *p*-methoxyphenyl.

EP 0 021 883 A2

TITLE MODIFIED

see front page

N-Acyl-Amino Acid Derivatives

The invention relates to novel pharmaceutically useful N-acyl amino acid derivatives which are inhibitors of angiotensin converting enzyme. The invention provides said compounds *per se*, pharmaceutical compositions comprising said compounds, methods of medical treatment using said compounds, processes for preparing said compounds and novel intermediates formed in said processes.

## BACKGROUND OF THE INVENTION

Angiotensin II is an active pressor octapeptide which is implicated as a causative agent in various forms of mammalian hypertension. It is formed by the action of the protease angiotensin-converting enzyme (ACE) on the decapeptide Angiotensin I which is produced by the action of renin on the pseudoglobulin Angiotensinogen. ACE is not specific in its cleavage of Angiotensin I but acts on a number of peptides, including the vasodepressor nonapeptide bradykinin, to cleave a dipeptide residue from the C-terminal end of the peptide.

It has been proposed to treat angiotensin-dependent hypertension in mammals, especially man, by administering an inhibitor of angiotensin-converting enzyme. In particular, U.S. Patent No. 4046889 to Ondetti and Cushman issued September 6th 1977,

discloses the use of certain proline derivatives and related compounds as ACE-inhibitors. These proline derivatives and related compounds have the following general formula:-

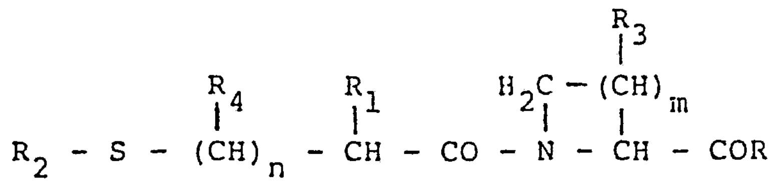

wherein:-

R represents hydroxy, $NH_2$ or alkoxy of 1 to 7 carbon atoms;

$R_1$ and $R_4$ each represent hydrogen, alkyl of 1 to 7 carbon atoms or phenylalkyl in which the alkyl moiety has 1 to 7 carbon atoms;

$R_2$ represents hydrogen or $R_5CO$;

$R_3$ represents hydrogen, hydroxy or alkyl of 1 to 7 carbon atoms;

$R_5$ represents alkyl of 1 to 7 carbon atoms, phenyl or phenylalkyl in which the alkyl moiety has 1 to 7 carbon atoms;

<u>m</u> represents 1 to 3; and

<u>n</u> represents 0 to 2.

The reportedly most preferred of said compounds of U.S. Patent No. 4046889 is the compound "Captopril" or "SQ 14225" which is the compound of the above general formula wherein:-

R represents hydroxy;

$R_1$ represents methyl;

$R_2$, $R_3$ and $R_4$ each represent hydrogen; and

m and n each represent 1.

Other ACE-inhibitors have also been reported including venom peptides (see, for example, Cushman et al, Experientia 1973, 29, 1032) and certain carboxyalkanoyl analogues of the mercaptoalkanoylprolines of U.S. Patent No. 4046889 (see, for example, Cushman et al, Biochemistry 1977, 16, 5484, and Ondetti et al, Science 1977, 196, 441); carboxy-alkylacylamino acids (Cushman et al, U.S. Patents No. 4,052,511 issued October 4, 1977, and 4,128,653 issued December 5, 1978); thio-acylamino acids (Ondetti et al, U.S. Patent 4,116,962 issued September 26, 1978); halogen substituted mercaptoacylamino acids (Ondetti et al, U.S. Patent No. 4,154,935 issued May 15, 1979); mercaptoacyl heterocyclic carboxylic acids (Bernstein et al, U.S. Patent No. 4,154,934 issued May 15, 1979); 1-[(1,2-dithiolane-4-yl) carbonyl]-L-proline (Ondetti et al, U.S. Patent No. 4,154,936 issued May 15, 1979); and hydroxycarbamoylalkylacyl pipecolic acid (Cushman et al, U.S. Patent No. 4,154,937 issued May 15, 1979).

The previously proposed ACE-inhibitors are competitive inhibitors in that they compete with the

natural substrates for binding to an active site of ACE. Whilst the inhibitors can be bound more tightly to said active site than the natural substrates, there is no covalent bond formation between the inhibitor and the enzyme and hence the inhibition is reversible.

In recent years the concept of enzyme-activated irreversible inhibitors (otherwise known as suicide or $k_{cat}$ inhibitors) has been developed (see, for example, "Enzyme-Activated Irreversible Inhibitors", Ed. Seiler _et al_, Elsevier/North Holland Biomedical Press, 1978, ISBN 0-444-80080-8). This concept is that an enzyme can be irreversibly inhibited by a substrate which contains a latent reactive group which is activated by the enzyme to form a reactive specie which eventually reacts with the enzyme or the cofactor to form a covalent bond therewith and thereby irreversibly inactivates the enzyme.

It is an object of the present invention to provide an enzyme-activated irreversible inhibitor for angiotensin-converting enzyme.

It is another object of the invention to provide an efficacious angiotensin-converting enzyme inhibitor.

A further objection of the invention is to provide an efficacious method of treating hypertension.

Additional objects will be apparent from the following description of the invention.

## SUMMARY OF THE INVENTION

According to a first aspect of the present invention, there are provided novel N-acyl amino acid derivatives of the following general Formula 1:-

$$\begin{array}{ccccccccc} & & X & & R_1 & & O & & \\ & & | & & | & & \| & & \\ Z & - & CH & - & CH & - & C & - & R_2 \end{array} \qquad \text{Formula 1}$$

wherein:-

Z represents an electron-withdrawing group, preferably cyano or a carbonyl-terminated functional group;

X represents a group which, in combination with Z, can form a Michael acceptor after abstraction of the proton from the carbon atom adjacent to X, and preferably X is ethynyl or a fluoromethyl group;

$R_1$ represents hydrogen, alkyl of 1 to 7 carbon atoms, or phenylalkyl in which the alkyl moiety has 1 to 7 carbon atoms; and

$R_2$ represents an aminocarboxylic acid residue obtained by removal of a hydrogen atom from the amino group thereof, and preferably an $\alpha$-amino acid residue; and derivatives thereof, especially non-toxic hydrolysable derivatives, such as for example pharmaceutically acceptable salts, amides and esters, which derivatives are transformed <u>in vivo</u> into compounds of Formula 1.

The invention includes individual optical isomers as well as racemates and other optical isomer mixtures of said compounds of Formula 1 and of the said prodrugs thereof.

Compounds of Formula 1 are pharmacologically useful in that they inhibit angiotensin-converting enzyme and can be used to treat angiotensin-dependent hypertension in man and other mammals.

## DETAILED DESCRIPTION OF THE INVENTION

The symbol Z in Formula 1 represents an electron-withdrawing group and preferably is cyano (i.e. $-C\equiv N$) or, more especially, a carbonyl-terminated functional group. Suitable electron-withdrawing groups Z include cyano and carbonyl-terminated groups of the formula -COR wherein:-

R represents

$$-R_{10},\ -OR_{10},\ -N\genfrac{}{}{0pt}{}{\diagup R_{10}}{\diagdown R_{10}},\ -NHOR_{10},\ -SR_{10},\ -R_{2},$$

$$-\overset{R_{10}}{\overset{|}{C}}H-R_{11},\quad \text{or } -CO_2H$$

$R_2$ is as defined in connection with Formula 1;

$R_{10}$ represents hydrogen, alkyl of 1 to 7 carbon atoms, phenylalkyl in which the alkyl moiety has 1 to 7 carbon atoms, phenyl or p-methoxyphenyl;

$R_{11}$ represents

$$\text{halogen},\ -OR_{10},\ -N\genfrac{}{}{0pt}{}{\diagup R_{10}}{\diagdown R_{10}},\ -O.CO.R_{10},$$

$$-SR_{10},\ \text{tosyloxy, or } -NH.CO.R_{12};$$

$R_{12}$ represents alkyl of 1 to 7 carbon atoms, phenylalkyl in which the alkyl moiety has 1 to 7 carbon atoms, phenyl, $-\underset{\underset{R_{10}}{|}}{CH}-NH.CO.R_{12}$, $-\underset{\underset{NH_2}{|}}{CH}(CH_2)_nNH_2$ or $-\underset{\underset{NHCOR_{12}}{|}}{CH}(CH_2)_nNH_2$ ; and

n represents 3 or 4.

Examples of suitable electron-withdrawing groups Z of the formula -COR as defined above are the following:-

| | |
|---|---|
| formyl | (R = $R_{10}$ = hydrogen) |
| carboxy | (R = $OR_{10}$, $R_{10}$ = hydrogen) |
| carboxycarbonyl | (R = $CO_2H$) |
| alkanoyl | (R = $R_{10}$ = alkyl) |
| phenylalkanoyl | (R = $R_{10}$ = phenylalkyl) |
| benzoyl | (R = $R_{10}$ = phenyl) |
| p-methoxybenzoyl | (R = $R_{10}$ = p-methoxyphenyl) |
| alkoxycarbonyl | (R = $OR_{10}$, $R_{10}$ = alkyl) |
| phenylalkoxy-carbonyl | (R = $OR_{10}$, $R_{10}$ = phenylalkyl) |
| phenoxycarbonyl | (R = $OR_{10}$, $R_{10}$ = phenyl) |
| carbamoyl | (R = $N\begin{smallmatrix}R_{10}\\R_{10}\end{smallmatrix}$, both $R_{10}$ = hydrogen) |
| alkylcarbamoyl | (R = $N\begin{smallmatrix}R_{10}\\R_{10}\end{smallmatrix}$, $R_{10}$ = hydrogen and alkyl) |
| dialkylcarbamoyl | (R = $N\begin{smallmatrix}R_{10}\\R_{10}\end{smallmatrix}$, both $R_{10}$ = alkyl) |
| phenylcarbamoyl | (R = $N\begin{smallmatrix}R_{10}\\R_{10}\end{smallmatrix}$, $R_{10}$ = hydrogen and phenyl) |

| | |
|---|---|
| hydroxycarbamoyl | (R = $NHOR_{10}$, $R_{10}$ = hydrogen) |
| alkoxycarbamoyl | (R = $NHOR_{10}$, $R_{10}$ = alkyl) |
| mercaptocarbonyl | (R = $SR_{10}$, $R_{10}$ = hydrogen) |
| alkylthiocarbonyl | (R = $SR_{10}$, $R_{10}$ = alkyl) |
| phenylthiocarbonyl | (R = $SR_{10}$, $R_{10}$ = phenyl) |
| glycoloyl | (R= $-\overset{R_{10}}{\overset{\vert}{C}}H-R_{11}$, $R_{10}$ = hydrogen, $R_{11}$ = hydroxy) |
| halogenoacetyl | (R = $-\overset{R_{10}}{\overset{\vert}{C}}H-R_{11}$, $R_{10}$ = hydrogen, $R_{11}$ = halogen) |
| glycyl | (R = $-\overset{R_{10}}{\overset{\vert}{C}}H-R_{11}$, $R_{10}$ = hydrogen, $R_{11}$ = $-NH_2$) |
| N-alkanoylglycyl | (R = $-\overset{R_{10}}{\overset{\vert}{C}}H-R_{11}$, $R_{10}$ = hydrogen, $R_{11}$ = $-NHCOR_{12}$, $R_{12}$ = alkyl) |
| N-benzoylglycyl | (R = $-\overset{R_{10}}{\overset{\vert}{C}}H-R_{11}$, $R_{10}$ = hydrogen, $R_{11}$ = $NHCOR_{12}$, $R_{12}$ = phenyl) |
| N-phenylalkanoyl-glycyl | (R = $-\overset{R_{10}}{\overset{\vert}{C}}H-R_{11}$, $R_{10}$ = hydrogen, $R_{11}$ = $NHCOR_{12}$, $R_{12}$ = phenyl-alkyl) |
| N-alkanoyl-α-aminoalkanoyl | (R = $-\overset{R_{10}}{\overset{\vert}{C}}H-R_{11}$, $R_{10}$ = alkyl, $R_{11}$ = $-NHCOR_{12}$, $R_{12}$ = alkyl) |

| | |
|---|---|
| N-benzoyl-α-aminoalkanoyl | (R = $-\overset{R_{10}}{\overset{\vert}{C}}H-R_{11}$, $R_{10}$ = alkyl, $R_{11}$ = $-NHCOR_{12}$, $R_{12}$ = phenyl) |
| N-phenylalkanoyl-α-aminoalkanoyl | (R = $-\overset{R_{10}}{\overset{\vert}{C}}H-R_{11}$, $R_{10}$ = alkyl, $R_{11}$ = $NHCOR_{12}$, $R_{12}$ =phenyl-alkyl) |
| N-alkanoyl-phenylalanyl | (R = $-\overset{R_{10}}{\overset{\vert}{C}}H-R_{11}$, $R_{10}$ = benzyl, $R_{11}$ = $-NHCOR_{12}$, $R_{12}$ = alkyl) |
| N-benzoyl-phenyl-alanyl | (R = $-\overset{R_{10}}{\overset{\vert}{C}}H-R_{11}$, $R_{10}$ = benzyl, $R_{11}$ = $-NHCOR_{12}$, $R_{12}$ = phenyl) |
| N-phenylalkanoyl-phenylalanyl | (R = $-\overset{R_{10}}{\overset{\vert}{C}}H-R_{11}$, $R_{10}$ = benzyl, $R_{11}$ = $-NHCOR_{12}$, $R_{12}$ = phenyl-alkyl) |
| alkoxyacetyl | (R = $-\overset{R_{10}}{\overset{\vert}{C}}H-R_{11}$, $R_{10}$ = hydrogen, $R_{11}$ = $OR_{10}$, $R_{10}$ = alkyl |
| tosyloxyacetyl | (R = $-\overset{R_{10}}{\overset{\vert}{C}}H-R_{11}$, $R_{10}$ = hydrogen, $R_{11}$ = Otosyl) |
| alkanoyloxyacetyl | (R = $-\overset{R_{10}}{\overset{\vert}{C}}H-R_{11}$, $R_{10}$ = hydrogen, $R_{11}$ = $-O.CO.R_{10}$, $R_{10}$ = alkyl) |

| | |
|---|---|
| N-(lysyl or ornithyl)glycyl | (R = $-\overset{R_{10}}{\overset{\mid}{C}}H-R_{11}$, $R_{10}$ = hydrogen, $R_{11}$ = $-NHCOR_{12}$, $R_{12}$ = $-\underset{NH_2}{\underset{\mid}{C}}H(CH_2)_nNH_2$) |
| N-[N$^{\alpha}$-alkanoyl-lysyl or ornithyl)glycyl | (R = $-\overset{R_{10}}{\overset{\mid}{C}}H-R_{11}$, $R_{10}$ = hydrogen, $R_{11}$ = $-\underset{NHCOR_{12}}{\underset{\mid}{C}}H(CH_2)_nNH_2$, $R_{12}$ = alkyl) |
| thioglycoloyl | (R = $-\overset{R_{10}}{\overset{\mid}{C}}H-R_{11}$, $R_{10}$ = hydrogen, $R_{11}$ = mercapto) |
| (2-carboxy-1-pyrrolidinyl)carbonyl | (R = $R_2$ = 2-carboxy-1-pyrrolidinyl) |

Preferred electron-withdrawing groups Z of the formula -COR as defined above include $-COR_{10}'$, -CONHOH and $-COCH(R_{10}'')NHCOR_{10}'$, wherein $R_{10}'$ represents $C_1$-$C_7$ alkyl, phenyl or phenyl-$C_1$-$C_7$ alkyl and $R_{10}''$ represents $C_1$-$C_7$ alkyl or phenyl-$C_1$-$C_7$ alkyl. It is particularly preferred that $R_{10}'$ represents $C_1$-$C_4$ alkyl, phenyl or benzyl and that $R_{10}''$ represents $C_1$-$C_4$ alkyl, especially s-butyl, or benzyl.

References in this Specification, including the Claims, to an alkyl group or moiety mean a straight or branched chain alkyl group or moiety of 1 to 7 carbon atoms unless some further limitation is stated or clearly implied by the context. Further, reference to a specific alkyl group or moiety having structural isomers includes all of those isomers and mixtures thereof unless a particular isomer is specified or clearly implied by the context. Thus, the alkyl groups or moieties generally referred to in connection with the definition and exemplification of the electron-withdrawing group Z and elsewhere in the

Specification are methyl; ethyl; n-propyl; isopropyl; butyl, for example n-butyl, sec-butyl, and tert-butyl; pentyl, for example n-pentyl, neo-pentyl and tert-pentyl; hexyl, for example isohexyl; and heptyl, for example 5-methylhexyl. Unless, otherwise stated, the alkyl groups and moieties preferably have 1 to 4 carbon atoms, i.e. methyl, ethyl, propyl and butyl.

Illustrative examples of preferred phenylalkyl groups are benzyl, phenethyl, 1-phenylethyl, 3-phenylpropyl, and 4-phenylbutyl.

Illustrative examples of preferred alkoxy groups are methoxy, ethoxy, n-propoxy, n-butoxy, iso-propoxy, and tert-butoxy.

Illustrative examples of preferred alkanoyl groups are acetyl, propionyl, butyryl, and isobutyryl.

Illustrative examples of preferred phenylalkanoyl groups are phenylacetyl, 3-phenylpropionyl, 4-phenylbutyryl and 3-phenylisobutyryl.

Illustrative examples of preferred alkoxycarbonyl groups are methoxycarbonyl (otherwise "carbomethoxy"), ethoxycarbonyl (otherwise "carboethoxy"), n-propoxycarbonyl, n-butoxycarbonyl, tert-butoxycarbonyl and sec-butoxycarbonyl.

Illustrative examples of preferred phenylalkoxycarbonyl groups are benzyloxycarbonyl,2-phenylethoxycarbonyl, 1-phenylethoxycarbonyl, 3-phenylpropoxycarbonyl, and 4-phenylbutoxycarbonyl.

Illustrative examples of preferred alkylcarbamoyl groups are methylcarbamoyl, ethylcarbamoyl, n-propylcarbamoyl, iso-propylcarbamoyl, n-butylcarbamoyl and sec-butylcarbamoyl.

Illustrative examples of dialkylcarbamoyl groups are dimethylcarbamoyl, methylethylcarbamoyl, diethylcarbamoyl and di(n-propyl)carbamoyl.

Illustrative examples of alkoxycarbamoyl groups are methoxycarbamoyl, ethoxycarbamoyl, n-propoxycarbamoyl, isopropoxycarbamoyl and sec-butoxycarbamoyl.

Illustrative examples of alkylthiocarbonyl groups are methylthiocarbonyl, ethylthiocarbonyl, n-propylthiocarbonyl, iso-propylthiocarbonyl, n-butylthiocarbonyl and iso-butylthiocarbonyl.

When, as in connection with the exemplification above of the electron-withdrawing group Z, the same symbol, for example $R_{10}$, is used more than once in the same formula, said symbols can represent identical or different groups within the definition of the relevant symbol.

Unless stated or clearly implied by the context, the term "halogen" used in this Specification, including the Claims, means bromine, chlorine, fluorine and iodine and the preferred halogens are chlorine and fluorine. Illustrative examples of halogenoacetyl

groups are chloroacetyl and fluoroacetyl.

The symbol X in Formula 1 represents a group which, in combination with the electron-withdrawing group Z can form a Michael acceptor after abstraction of the proton from the carbon atom adjacent to the group X. Michael acceptors have a double bond conjugated with an electron-withdrawing group and are good substrates for nucleophilic addition at the double bond. The formation of Michael acceptors and conjugate addition of nucleophiles to Michael acceptors are so well known per se as not to require detailed discussion at this time. Without restriction to any particular theory as to the mechanism by which the compounds of Formula 1 inhibit ACE, it is believed that ACE catalyses removal of the proton from the carbon atom adjacent to the group X whereby the Michael acceptor is formed and subsequently reacts with a nucleophilic site of the enzyme to form a covalent bond therewith by conjugate addition. This proposed mechanism can be represented as follows:-

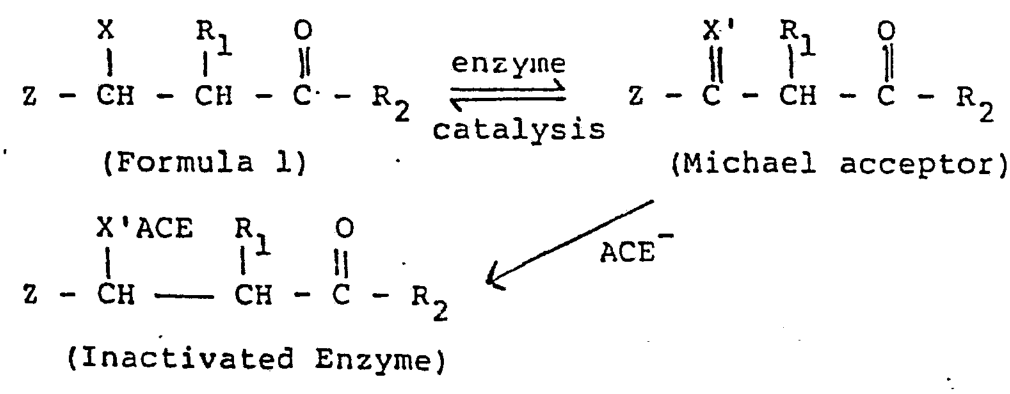


(Formula 1)

(Michael acceptor)

(Inactivated Enzyme)

In the above scheme Z, X, $R_1$ and $R_2$ are as defined in connection with Formula 1, X' is the residue of X upon formation of the Michael acceptor, and ACE is angiotensin-converting enzyme.

The preferred groups X are ethynyl (i.e. $-C\equiv CH$) and fluoromethyl of the formula $-CF_pH_{3-p}$ wherein p represents 1, 2 or 3 (i.e. mono-, di- and tri-fluoromethyl).

The symbol $R_1$ in Formula 1 represents hydrogen, alkyl of 1 to 7 carbon atoms or phenylalkyl in which the alkyl moiety has 1 to 7 carbon atoms. It is preferred that $R_1$ represents hydrogen or alkyl. When $R_1$ represents alkyl or phenylalkyl, it is intended that reference should be made to the definition of "alkyl" and the exemplification of alkyl and phenylalkyl provided hereinbefore. It is preferred that the alkyl group has 1 to 4 carbon atoms and that the alkyl moiety of the phenylalkyl group also has 1 to 4 carbon atoms. Methyl is the most preferred alkyl group and benzyl is the most preferred phenylalkyl group.

The symbol $R_2$ in Formula 1 represents an aminocarboxylic acid residue obtained by removal of a hydrogen from the amino group thereof. Preferably, the amino acid residue has the amino group attached to an $\alpha$-carbon atom relative to the carboxylic acid group

and suitably, but not necessarily, is the residue of a naturally occurring mammalian amino acid. The carboxylic acid group of the residue can be a free carboxyl or a non-toxic hydrolysable derivative thereof which is transformed *in vivo* into the carboxyl group. In particular, the carboxylic acid group can be present in Formula 1 in the form of a pharmaceutically acceptable salt, amide, such as primary amide or ester, such as alkyl ester.

In one preferred embodiment, the group $R_2$ has the formula:-

$$-\overset{R_3}{\overset{|}{N}}-\overset{R_4}{\overset{|}{CH}}-COR_5$$

wherein:-

$R_3$ and $R_4$ represent hydrogen or the side chain of a naturally occurring mammalian amino acid of the formula

$$H\overset{R_3}{\overset{|}{N}}-\overset{R_4}{\overset{|}{CH}}-CO_2H,$$ and

$R_5$ represents hydroxy, alkoxy of 1 to 7 carbon atoms, primary amino (i.e. $-NH_2$) or

$$-\overset{R_3}{\overset{|}{N}}-\overset{R_4}{\overset{|}{CH}}-COR_5$$ in which $R_3$, $R_4$ and $R_5$ are as defined above.

In another preferred embodiment, the group $R_2$ has the formula:-

$$-\overset{R_3'}{\overset{|}{N}}-\overset{R_4'}{\overset{|}{CH}}-COR_5'$$

wherein

$R_3'$ represents hydrogen and $R_4'$ represents hydrogen or alkyl of 1 to 4 carbon atoms and optionally substituted by indole, by imidazole or by phenyl, or

$R_3'$ and $R_4'$ together represent alkylene or alkenylene, said alkylene and alkenylene having 2, 3 or 4 chain carbon atoms and being optionally substituted by hydroxy, by alkyl of 1 to 7 carbon atoms or, in the hydrocarbon chain, by a sulfur atom; and

$R_5'$ represents hydroxy, alkoxy of 1 to 7 carbon atoms, primary amino, or $-\overset{\overset{R_3'}{|}}{N}-\overset{\overset{R_4'}{|}}{CH}-COR_5'$ in which $R_3'$, $R_4'$ and $R_5'$ are as defined above.

It will readily be appreciated that the aforementioned two preferred embodiments of the group $R_2$ are not mutually exclusive and that there are numerous aminoacid residues which fall within the scope of both of said embodiments.

Illustrative examples of naturally occurring mammalian amino acid residues within both embodiments are the following:-

| | |
|---|---|
| carboxymethylamino | ($R_3 = R_4 = H$ ) (i.e. glycine residue) |
| 1-carboxy-1-ethylamino | ($R_3 = H$, $R_4$ = methyl) (i.e. alanine residue) |

| | |
|---|---|
| 1-carboxy-2-methyl-1--propylamino | ($R_3$ = H, $R_4$ = isopropyl) (i.e. valine residue) |
| 1-carboxy-3-methyl-1--butylamino | ($R_3$ = H, $R_4$ = isobutyl (i.e. leucine residue) |
| 1-carboxy-2-methyl-1--butylamino | ($R_3$ = H, $R_4$ = secbutyl) (i.e. isoleucine residue) |
| 1-carboxy-2-phenyl-1--ethylamino | ($R_3$ = H, $R_4$ = benzyl) (i.e. phenylalanine residue) |
| 2-carboxy-1-pyrrolidinyl | ($R_3$ + $R_4$ = trimethylene) (i.e. proline residue) |
| 2-carboxy-4-hydroxy--1-pyrrolidinyl | ($R_3$ + $R_4$ = 2-hydroxy-trimethylene) (i.e. hydroxy-proline residue) |
| 1-carboxy-2-(3-indolyl)-1-ethylamino | ($R_3$ = H, $R_4$ = 3-indolyl-methyl) (i.e. tryptophan residue), and |
| 1-carboxy-2-(5-imidazolyl-1-ethyl-amino | ($R_3$ = H, $R_4$ = imidazolyl-methyl) (i.e. histidine residue). |

Other illustrative examples of aminoacid residues are the following:-

2-carboxy-piperidino ($R_3$' + $R_4$' = tetramethylene)

2-carboxy-3-pyrrolin-1-yl ($R_3$' + $R_4$' = propenylene)

4-carboxy-1,3-thiazolidin-3-yl ($R_3$' + $R_4$' = methylenethiomethyl)

2-carboxy-1-azetidinyl ($R_3$' + $R_4$' = ethylene), and 1(1'-carboxy-3'-methyl-butylcarbamoyl)-2(4"-imidazolyl)-ethylamino ($R_3$' = H, $R_4$' = 4-imidazolylmethyl, R'$_5$= 1-carboxy-3-methyl-1-butylamino).

It is particularly preferred that the group $R_2$ has the formula:

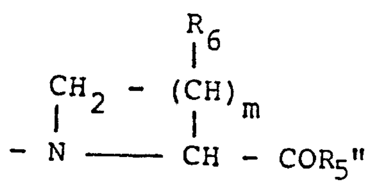

wherein

m represents 1, 2 or 3,

$R_5$" represents hydroxy, alkoxy of 1 to 7 carbon atoms, or amino, and

$R_6$ represents hydrogen, hydroxy, alkyl of 1 to 7 carbon atoms or $R_6$ on each of two adjacent carbon atoms together represent a second valency bond joining those carbon atoms.

When m is 2 or 3, each $R_6$ radical is independently selected from those defined above and can be the same as or different from another $R_6$ radical in the molecule. The especially preferred groups $R_2$ are the proline residues in which m represents 2 and the most preferred group $R_2$ is 2-carboxy-1-pyrrolidinyl where m represents 2, $R_5$" represents hydroxy and $R_6$ represents hydrogen.

When $R_5$, $R_5$' or $R_5$" represent alkoxy and/or $R_6$ represents alkyl, it is intended that reference should be made to the definition of "alkyl" and the exemplification of alkyl and alkoxy provided hereinbefore. It is preferred that when $R_6$ represents alkyl, the alkyl group has 1 to 4 carbon atoms and that when $R_5$, $R_5$' or $R_5$" represents alkoxy, the alkoxy group has 1 to 4 carbon atoms.

The compounds of Formula 1 exist as at least two enantiomers because the α carbon atom relative to the ______________________

electron-withdrawing group Z is asymmetrical. If $R_1$ represents alkyl or phenylalkyl, a further asymmetrical centre is present in the molecule. Moreover, another asymmetrical centre can be provided by the aminoacid residue. As mentioned previously, the present invention includes not only racemates and other mixtures of the optical isomers but also the individual optical isomers. Methods of selecting reactants and/or optically resolving intermediates to provide a desired individual optical isomer are well known *per se* to those skilled in the art. See, for example, "Stereochemistry of Chemical Compounds", Eliel, McGraw-Hill, N.Y. 1962, pp. 49-63.

The invention also includes derivatives which are transformed *in vivo* into the compounds of Formula 1. Said derivatives include especially non-toxic hydrolysable derivatives such as salts, amides and esters. Examples of amides and esters are those compounds referred to above in which $R_5$ represents amino or alkoxy. The salts can be acid addition salts of basic centres in the molecule or base salts of acid centres in the molecule. Illustrative of acid addition salts are those with inorganic acids, such as hydrochloric, hydrobromic, sulfuric and phosphoric acids, and with organic acids, such as salicylic, maleic, malonic, tartaric, citric, ascorbic, cyclamic or methane sulfonic acids. Illustrative of basic

BAD ORIGINAL

salts are those with inorganic bases, such as ammonium, alkali metals, for example sodium, potassium and lithium, alkaline earth metals, for example calcium and magnesium, and light metals of Group IIIA, for example aluminium; with organic bases such as cyclohexylamine, ethylamine, ethylaminoethanol, ethanolamine, piperazine, benzathine, N-methyl-D-glucamine, and with amino acids such as arginine and lysine.

In addition to pharmaceutically acceptable salts, other salts such as those acid addition salts with picric or oxalic acid and those basic salts with dicyclohexamine are useful as intermediates in the purification of compounds of Formula 1, in the preparation of pharmaceutically acceptable salts, or for the characterisation and identification of compounds of Formula 1.

The salts, amides and esters can be prepared from corresponding compounds of Formula 1 by conventional methods well known in the art.

One broadly preferred class of compounds of Formula 1 are those of the following general Formula 2:-

$$Z_1 - \overset{X_1}{\overset{|}{CH}} - \overset{R_1}{\overset{|}{CH}} - \overset{O}{\overset{||}{C}} - \overset{R_3}{\overset{|}{N}} - \overset{R_4}{\overset{|}{CH}} - COR_5 \qquad \text{Formula 2}$$

wherein:-

$Z_1$ represents cyano or an electron-withdrawing carbonyl-terminated functional group;

$X_1$ represents ethynyl or mono-, di- or tri-fluoromethyl;

$R_1$ represents hydrogen, alkyl of 1 to 7, preferably 1 to 4, carbon atoms or phenylalkyl in which the alkyl moiety has 1 to 7, preferably 1 to 4, carbon atoms;

$R_3$ and $R_4$ represent hydrogen or the side chain of a naturally occurring mammalian amino acid of the formula

$$H\overset{R_3}{\overset{|}{N}} - \overset{R_4}{\overset{|}{CH}} - CO_2H;$$ and

$R_5$ represents hydroxy, alkoxy of 1 to 7, preferably 1 to 4, carbon atoms, primary amino or

$$- \overset{R_3}{\overset{|}{N}} - \overset{R_4}{\overset{|}{CH}} - COR_5$$ in which $R_3$, $R_4$ and $R_5$ are as defined above,

and pharmaceutically acceptable salts thereof.

One preferred sub-class of compounds of Formula 2

are those of the following general Formula 3:-

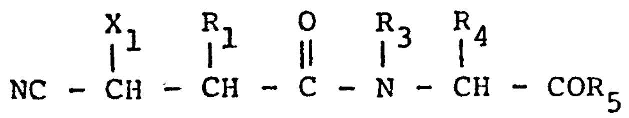

Formula 3

wherein $X_1$, $R_1$, $R_3$, $R_4$ and $R_5$ are as defined in connection with Formula 2,

and pharmaceutically acceptable salts thereof.

One genus of compounds of Formula 3 are those of the following general Formula 3A:-

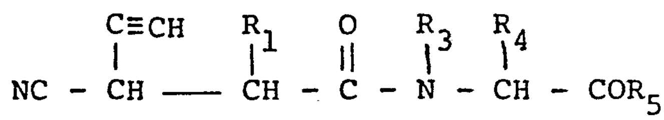

Formula 3A

wherein $R_1$, $R_3$, $R_4$ and $R_5$ are as defined in connection with Formula 2,

and pharmaceutically acceptable salts thereof.

The other genus of compounds of Formula 3 are those of the following general Formula 3B:-

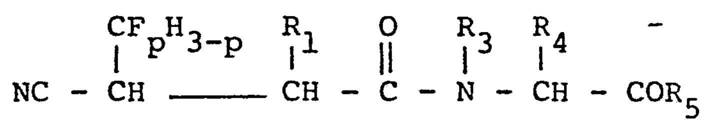

Formula 3B

wherein:-

p represents 1, 2 or 3; and

$R_1$, $R_3$, $R_4$ and $R_5$ are as defined in connection with Formula 2,

and pharmaceutically acceptable salts thereof.

Another preferred sub-class of compounds of Formula 2 are those of the following general Formula 4:-

$$RCO - \overset{X_1}{\overset{|}{CH}} - \overset{R_1}{\overset{|}{CH}} - \overset{O}{\overset{\|}{C}} - \overset{R_3}{\overset{|}{N}} - \overset{R_4}{\overset{|}{CH}} - COR_5 \qquad \text{Formula 4}$$

wherein:-

$X_1$, $R_1$, $R_3$, $R_4$ and $R_5$ are as defined in connection with Formula 2;

R represents $-R_{10}$, $-OR_{10}$, $-N\begin{matrix}R_{10}\\R_{10}\end{matrix}$, $-NHOR_{10}$, $-SR_{10}$, $-\overset{R_{10}}{\overset{|}{CH}} - R_{11}$, $-COOH$, or $-\overset{R_3}{\overset{|}{N}} - \overset{R_4}{\overset{|}{N}} - COR_5$

$R_{10}$ represents hydrogen, alkyl of 1 to 7, preferably 1 to 4, carbon atoms, phenylalkyl in which the alkyl moiety has 1 to 7, preferably 1 to 4, carbon atoms, phenyl or p-methoxyphenyl;

$R_{11}$ represents halogen, $-OR_{10}$, $-N\begin{matrix}R_{10}\\R_{10}\end{matrix}$, $-SR_{10}$ $-O.CO.R_{10}$, tosyloxy or $-NH.CO.R_{12}$;

$R_{12}$ represents alkyl of 1 to 7, preferably 1 to 4, carbon atoms, phenylalkyl in which the alkyl moiety has 1 to 7, preferably 1 to 4, carbon atoms, phenyl, $-\underset{NH_2}{\underset{|}{CH}}(CH_2)_nNH_2$, $-\underset{NHCOR_{12}}{\underset{|}{CH}}(CH_2)_nNH_2$ or $-\underset{R_{10}}{\underset{|}{CH}} - NH.CO.R_{12}$;

n represents 3 or 4, preferably 4,

and pharmaceutically acceptable salts thereof.

One genus of compounds of Formula 4 are those of the following general Formula 4A:-

$$RCO - \overset{C\equiv CH}{\overset{|}{CH}} —— \overset{R_1}{\overset{|}{CH}} - \overset{O}{\overset{\|}{C}} - \overset{R_3}{\overset{|}{N}} - \overset{R_4}{\overset{|}{CH}} - COR_5 \qquad \text{Formula 4A}$$

wherein:-

$R_1$, $R_3$, $R_4$ and $R_5$ are as defined in connection with Formula 2, and

R is as defined in connection with Formula 4, and pharmaceutically acceptable salts thereof.

The other genus of compounds of Formula 4 are those of the following general Formula 4B:-

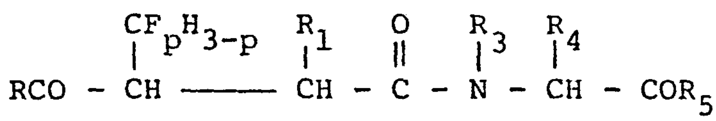
Formula 4B

wherein:-

p represents 1, 2 or 3;

$R_1$, $R_3$, $R_4$ and $R_5$ are as defined in connection with Formula 2; and

R is as defined in connection with Formula 4, and pharmaceutically acceptable salts thereof.

Another broadly preferred class of compounds of Formula 1 are those of the following general Formula 5, which also includes many of the compounds of the Formula 2:-

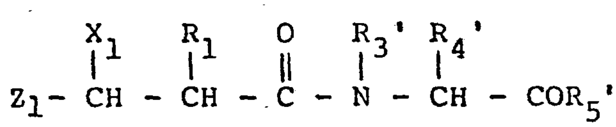
Formula 5

wherein:-

$Z_1$ represents cyano or an electron-withdrawing carbonyl-terminated functional group;

$X_1$ represents ethynyl or mono-, di- or trifluoromethyl;

$R_1$ represents hydrogen, alkyl of 1 to 7, preferably 1 to 4, carbon atoms or phenylalkyl in which the alkyl moiety has 1 to 7, preferably 1 to 4, carbon atoms;

$R_3'$ represents hydrogen and $R_4'$ represents hydrogen or alkyl of 1 to 4 carbon atoms and optionally substituted by indole, by imidazole or by phenyl, or

$R_3'$ and $R_4'$ together represent alkylene or alkenylene, said alkylene and alkenylene having 2, 3 or 4 chain carbon atoms and being optionally substituted by hydroxy, by alkyl of 1 to 7, preferably 1 to 4, carbon atoms or, in the hydrocarbon chain, by a sulfur atom; and

$R_5'$ represents hydroxy, alkoxy of 1 to 7, preferably 1 to 4, carbon atoms, primary amino, or

$-\overset{R_3'}{\overset{|}{N}}-\overset{R_4'}{\overset{|}{CH}}-COR_5'$ in which $R_3'$, $R_4'$ and $R_5'$ are as defined above,

and pharmaceutically acceptable salts thereof.

One preferred sub-class of compounds of Formula 5 are those of the following general Formula 6:-

$$NC-\overset{X_1}{\overset{|}{CH}}-\overset{R_1}{\overset{|}{CH}}-\overset{O}{\overset{\|}{C}}-\overset{R_3'}{\overset{|}{N}}-\overset{R_4'}{\overset{|}{CH}}-COR_5' \qquad \text{Formula 6}$$

wherein:-

$X_1$, $R_1$, $R_3'$, $R_4'$ and $R_5'$ are as defined in connection with Formula 5,

and pharmaceutically acceptable salts thereof.

One genus of compounds of Formula 6 are those of the following general Formula 6A:-

$$NC-\overset{C\equiv CH}{\overset{|}{CH}}-\overset{R_1}{\overset{|}{CH}}-\overset{O}{\overset{\|}{C}}-\overset{R_3'}{\overset{|}{N}}-\overset{R_4'}{\overset{|}{CH}}-COR_5' \qquad \text{Formula 6A}$$

wherein $R_1$, $R_3'$, $R_4'$ and $R_5'$ are as defined in connection with Formula 5,

and pharmaceutically acceptable salts thereof.

The other genus of compounds of Formula 6 are those of the following general Formula 6B:-

$$NC-\overset{CF_pH_{3-p}}{\overset{|}{CH}}-\overset{R_1}{\overset{|}{CH}}-\overset{O}{\overset{\|}{C}}-\overset{R_3'}{\overset{|}{N}}-\overset{R_4'}{\overset{|}{CH}}-COR_5' \qquad \text{Formula 6B}$$

wherein $R_1$, $R_3'$, $R_4'$ and $R_5'$ are as defined in connection with Formula 5,

and pharmaceutically acceptable salts thereof.

Another preferred sub-class of compounds of Formula 5 are those of the following general Formula 7:-

$$RCO-\overset{X_1}{\overset{|}{CH}}-\overset{R_1}{\overset{|}{CH}}-\overset{O}{\overset{\|}{C}}-\overset{R_3'}{\overset{|}{N}}-\overset{R_4'}{\overset{|}{CH}}-COR_5' \qquad \text{Formula 7}$$

wherein:-

$X_1$, $R_1$, $R_3'$, $R_4'$ and $R_5'$ are as defined in connection with Formula 5;

R represents $-R_{10}$, $-OR_{10}$, $-N\begin{smallmatrix}R_{10}\\R_{10}\end{smallmatrix}$, $-NHOR_{10}$,

$-SR_{10}$, $-\overset{R_{10}}{\overset{|}{CH}}-R_{11}$, $-COOH$ or $-\overset{R_3'}{\overset{|}{N}}-\overset{R_4'}{\overset{|}{CH}}-COR_5'$;

$R_{10}$ represents hydrogen, alkyl of 1 to 7, preferably 1 to 4, carbon atoms, phenylalkyl in which the alkyl moiety has 1 to 7, preferably 1 to 4, carbon atoms, phenyl or p-methoxyphenyl;

$R_{11}$ represents halogen, $-OR_{10}$, $-N\begin{matrix}R_{10}\\R_{10}\end{matrix}$, $-SR_{10}$, $-O.CO.R_{10}$, tosyloxy or $-NH.CO.R_{12}$,

$R_{12}$ represents alkyl of 1 to 7, preferably 1 to 4, carbon atoms, phenylalkyl in which the alkyl moiety has 1 to 7, preferably 1 to 4, carbon atoms, phenyl, $-\underset{\displaystyle NH_2}{\underset{|}{CH}}(CH_2)_nNH_2$, $-\underset{\displaystyle NHCOR_{12}}{\underset{|}{CH}}(CH_2)_nNH_2$ or $-\underset{\displaystyle R_{10}}{\underset{|}{CH}}-NH.CO.R_{12}$; and

n represents 3 or 4, preferably 4,

and pharmaceutically acceptable salts thereof.

One genus of compounds of Formula 7 are those of the following general Formula 7A:-

$$\begin{array}{ccccccccccccc} & & C\equiv CH & & R_1 & & O & & R_3' & & R_4' & & \\ & & | & & | & & \| & & | & & | & & \\ RCO & - & CH & — & CH & - & C & - & N & - & CH & - & COR_5' \end{array} \qquad \text{Formula 7A}$$

wherein:-

$R_1$, $R_3'$, $R_4'$ and $R_5'$ are as defined in connection with Formula 5, and

R is as defined in connection with Formula 7, and pharmaceutically acceptable salts thereof.

The other genus of compounds of Formula 7 are

those of the following Formula 7B:-

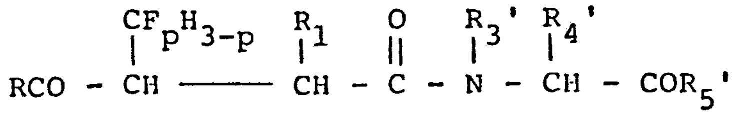 Formula 7B

wherein:-

$\underline{p}$ represents 1, 2 or 3;

$R_1$, $R_3'$, $R_4'$ and $R_5'$ are as defined in connection with Formula 5, and

R is as defined in connection with Formula 7, and pharmaceutically acceptable salts thereof.

In Formulae 4, 4A, 4B, 7, 7A and 7B, it is preferred that R represents $R_{10}'$, -NHOH or

$$-\overset{R_{10}''}{\overset{|}{CH}}-NHCOR_{10}'$$

in which $R_{10}'$ represents $C_1$-$C_7$ alkyl, preferably $C_1$-$C_4$, phenyl-$C_1$-$C_7$ alkyl, preferably benzyl, or phenyl and $R_{10}''$ represents $C_1$-$C_7$ alkyl, preferably $C_1$-$C_4$ alkyl and especially s-butyl, or phenyl-$C_1$-$C_7$ alkyl, preferably benzyl.

Particularly preferred compounds of Formula 5 include those of the following general Formula 8:-

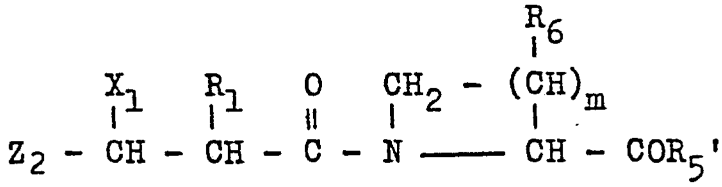 Formula 8

wherein:-

$Z_2$ represents cyano, $-COR_{10}'$,

-CO.NH.OH or 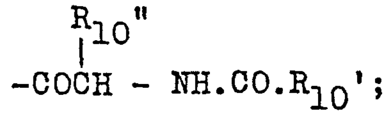;

$X_1$, $R_1$, $R_{10}'$ and $R_{10}''$ are as defined above in connection with Formula 5;

$R_5''$ represents hydroxy, alkoxy of 1 to 7, preferably 1 to 4, carbon atoms or primary amino;

*m* represents 1, 2 or 3; and

each $R_6$ independently represents hydrogen, hydroxy or alkyl of 1 to 7, preferably 1 to 4, carbon atoms or, when *m* represents 2 or 3, $R_6$ in each of two adjacent carbon atoms together represent a second valency bond between those carbon atoms;

and pharmaceutically acceptable salts thereof.

Preferably $R_5''$ represents hydroxy, *m* represents 2 and each $R_6$ represents hydrogen.

Particularly preferred compounds of Formula 6A include the compounds of the following general Formula 9A:-

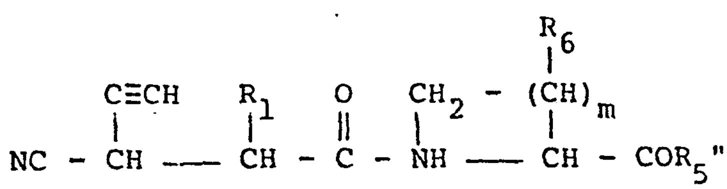 Formula 9A

wherein:-

*m*, $R_1$, $R_5''$ and $R_6$ are as defined in connection

with Formula 8,

and pharmaceutically acceptable salts thereof.

Particularly preferred compounds of Formula 6B include the compounds of the following general Formula 9B:-

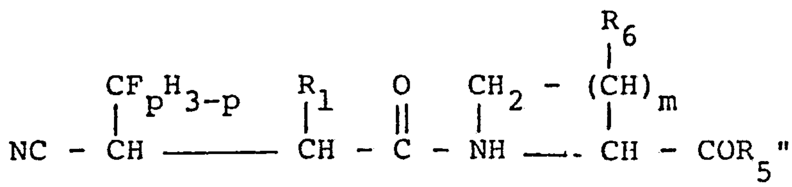

Formula 9B

wherein:-

p represents 1, 2 or 3, and

m, $R_1$, $R_5''$ and $R_6$ are as defined in connection with Formula 8,

and pharmaceutically acceptable salts thereof.

Particularly preferred compounds of Formula 7A include the compounds of the following general Formula 10A:-

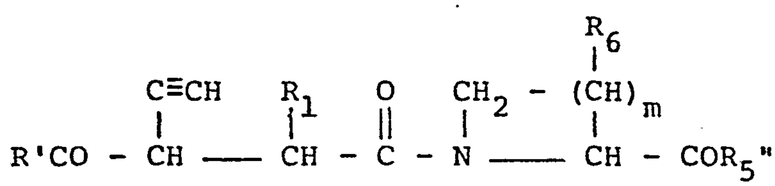

Formula 10A

wherein:-

R' represents $-R_{10}'$, -NHOH or

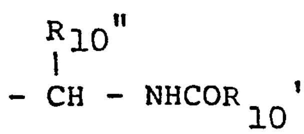 , and

m, $R_1$, $R_5''$, $R_6$, $R_{10}'$ and $R_{10}''$ are as defined in

connection with Formula 8,

and pharmaceutically acceptable salts thereof.

Particularly preferred compounds of Formula 7B include the compounds of the following general Formula 10B:-

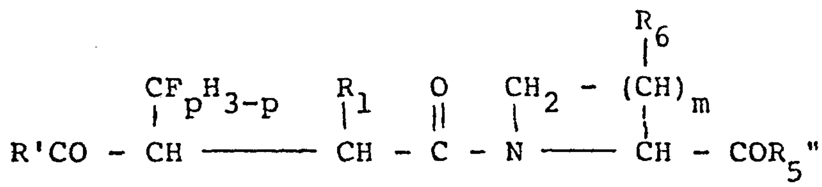

Formula 10B

wherein:-

p represents 1, 2 or 3;

R' represents $-R_{10}'$, -NHOH or

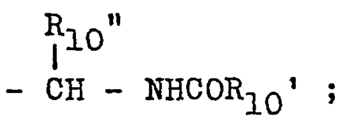 ; and

m, $R_1$, $R_5$", $R_6$, $R_{10}$' and $R_{10}$" are as defined in connection with Formula 8,

and pharmaceutically acceptable salts thereof.

The presently particularly preferred compounds of the invention include those of the following general Formula 11:-

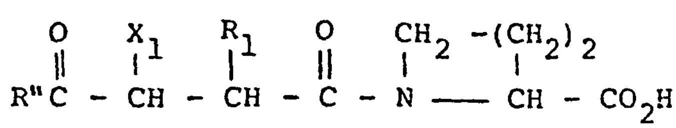 Formula 11

wherein:-

$X_1$ represents ethynyl or mono-, di- or tri-fluoromethyl;

R" represents 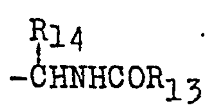 or -NHOH

$R_1$ represents hydrogen, alkyl of 1 to 4 carbon atoms or phenylalkyl in which the alkyl moiety has 1 to 4 carbon atoms,

$R_{13}$ represents alkyl of 1 to 4 carbon atoms or phenyl, and

$R_{14}$ represents benzyl or <u>sec</u>.butyl (i.e. $-CH(CH_3)CH_2CH_3$)

and pharmaceutically acceptable salts thereof.

The presently most preferred compounds of Formula 10A are the compounds of the following general Formula 12A:-

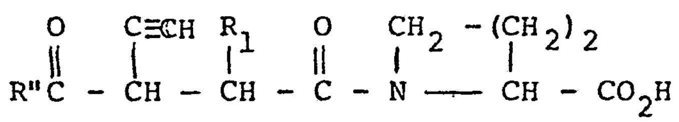 Formula 12A

wherein:-

R" and $R_1$ are as defined in connection with Formula 11,

and pharmaceutically acceptable salts thereof.

The presently most preferred compounds of Formula 10B are the compounds of the following general Formula 12B:-

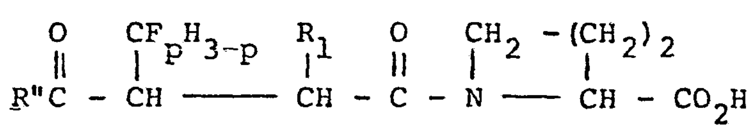 Formula 12B

wherein:-

<u>p</u> represents 1, 2 or 3, and

R" and $R_1$ are as defined in connection with Formula 11,

and pharmaceutically acceptable salts thereof.

Examples of compounds of the invention are the following:-

N-(3-cyano-3-monofluoromethyl-propionyl)-L-proline;

N-(3-cyano-3-difluoromethyl-propionyl)-L-alanine;

N-(3-cyano-3-trifluoromethyl-propionyl)-L-proline;

N-(3-cyano-pent-4-ynoyl)-L-phenylalanine;

N-(3-carboxy-3-monofluoromethyl-propionyl)-L-alanine;

N-(3-carboxy-3-difluoromethyl-propionyl)-L-proline;

N-(3-carboxy-3-trifluoromethyl-propionyl)-L-proline;

N-(3-carboxy-pent-4-ynoyl)-L-proline;

N-(3-formyl-3-trifluoromethyl-propionyl)-L-proline;

N-(3-formyl-3-monofluoromethyl-2-ethyl-propionyl)-L-phenylalanine;

N-(3-acetyl-3-trifluoromethyl-propionyl)-L-proline;

N-(3-acetyl-pent-4-ynoyl)-L-alanine;

N-(3-propionyl-3-difluoromethyl-propionyl)-L-proline;

N-(3-propionyl-3-trifluoromethyl-2-methyl-propionyl)-L-phenylalanine;

N-(3-butyryl-3-trifluoromethyl-propionyl)-L-proline;

N-(3-butyryl-3-difluoromethyl-2-methyl-propionyl)-L-phenylalanine;

N-(3-pivaloyl-pent-4-ynoyl)-L-proline;

N-(3-pivaloyl-3-monofluoromethyl-propionyl)-L-proline;

N-(3-benzoyl-3-difluoromethyl-propionyl)-L-alanine;

N-(3-benzoyl-3-trifluoromethyl-propionyl)-L-proline;

N-(3-phenylacetyl-3-difluoromethyl-propionyl)-L-alanine;

N-(3-(3-phenylpropionyl)-3-difluoromethyl-propionyl)-L-phenylalanine;

N-(3-(p-methoxybenzoyl)-3-trifluoromethyl-propionyl)-L-alanine;

N-(3-(p-methoxybenzoyl)-3-difluoromethyl-2-ethyl-propionyl)-L-proline;

N-(3-benzoyloxyacetyl-3-difluoromethyl-propionyl)-L-proline;

N-(3-methoxycarbonyl-3-trifluoromethyl-propionyl)-L-proline;

N-(3-methoxycarbonyl-pent-4-ynoyl)-L-proline;

N-(3-ethoxycarbonyl-3-difluoromethyl-2-methyl-propionyl)-L-phenylalanine;

N-(3-ethoxycarbonyl-3-trifluoromethyl-propionyl)-L-proline;

N-(3-propoxy-pent-4-ynoyl)-L-proline;

N-(3-iso-propoxy-3-trifluoromethyl-propionyl)-L-proline;

N-(3-butoxy-3-trifluoromethyl-2-methyl-propionyl)-L-phenylalanine;

N-(3-sec.-butoxy-3-trifluoromethyl-propionyl)-L-proline;

N-(3-tert-butoxy-3-monofluoromethyl-propionyl)-L-proline;

N-(3-tert-butoxy-pent-4-ynoyl)-L-proline;

N-(3-phenoxycarbonyl-3-difluoromethyl-propionyl)-L-proline;

N-(3-phenoxycarbonyl-3-trifluoromethyl-2-methyl-propionyl)-L- alanine;

N-(3-benzyloxy-3-trifluoromethyl-propionyl)-L-alanine;

N-(3-(2-phenylethoxy)-3-difluoromethyl-propionyl)-L-proline;

N-(3-carbamoyl-pent-4-ynoyl)-L-proline;

N-(3-carbamoyl-3-trifluoromethyl-propionyl)-L-proline;

N-(3-methylcarbamoyl-pent-4-ynoyl)-L-proline;

N-(3-propylcarbamoyl-3-difluoromethyl-propionyl)-L-proline;

N-(3-diethylcarbamoyl-pent-4-ynoyl)-L-proline;

N-(3-phenylcarbamoyl-3-trifluoromethyl-propionyl)-L-alanine;

N-(3-hydroxycarbamoyl-3-difluoromethyl-propionyl)-L-proline;

N-(3-hydroxycarbamoyl-3-trifluoromethyl-propionyl)-L-phenylalanine;

N-(3-methoxycarbamoyl-3-trifluoromethyl-propionyl)-L-phenylalanine;

N-(3-mercaptocarbonyl-3-difluoromethyl-propionyl)-L-proline;

N-(3-methylthiocarbonyl-3-trifluoromethyl-propionyl)-L-phenylalanine;

N-(3-phenylthiocarbonyl-3-trifluoromethyl-propionyl)-L-proline;

N-(3-glycoloyl-3-difluoromethyl-propionyl)-L-proline;

N-(3-chloroacetyl-3-difluoromethyl-propionyl)-L-alanine;

N-(3-bromoacetyl-3-trifluoromethyl-propionyl)-L-proline;

N-(3-iodoacetyl-3-difluoromethyl-propionyl)-L-alanine;

N-(3-fluoroacetyl-3-difluoromethyl-propionyl)-L-proline;

N-(3-hydroxyacetyl-3-difluoromethyl-propionyl)-L-proline;

N-(3-glycyl-3-difluoromethyl-2-methyl-propionyl)-L-proline;

N-(3-thioglycoloyl-3-difluoromethyl-propionyl)-L-alanine;

N-(3-(N-acetyl-glycyl)-3-difluoromethyl-propionyl)-L-alanine;

N-(3-(N-benzoyl-glycyl)-3-trifluoromethyl-propionyl)-L-proline;

N-(3-(N-phenylacetyl-glycyl)-3-difluoromethyl-propionyl)-L-proline;

N-(3-(N-acetyl-α-aminopropionyl)-3-difluoromethyl-propionyl)-L-alanine;

N-(3-(N-benzoyl-α-aminopropionyl)-3-difluoromethyl-propionyl)-L-phenylalanine;

N-(3-(N-phenylacetyl-α-aminopropionyl)-3-trifluoromethyl-propionyl)-L-phenylalanine;

N-(3-(N-acetyl-phenylalanyl)-3-difluoromethyl-propionyl)-L-proline;

N-(3-(N-benzoyl-phenylalanyl)-3-difluoromethyl-propionyl)-L-proline;

N-(3-(N-phenylacetyl-phenylalanyl)-3-trifluoromethyl-propionyl)-L-proline;

N-(3-methoxyacetyl-3-difluoromethyl-propionyl)-L-alanine;

N-(3-tosyloxyacetyl-3-difluoromethyl-propionyl)-L-proline;

N-(3-formyloxyacetyl-3-difluoromethyl-propionyl)-L-proline;

BAD ORIGINAL

N-(3-acetoxyacetyl-3-difluoromethyl-propionyl)-L-phenylalanine;

N-(3-(N-lysyl-glycyl)-3-trifluoromethyl-propionyl)-L-proline;

N-(3-(N-ornithyl-glycyl)-3-difluoromethyl-propionyl)-L-phenylalanine;

N-(3-(N-(N$^{\alpha}$-acetyl-lysyl)-glycyl)-3-difluoromethyl-propionyl)-L-proline;

N-(3-(N-(N$^{\alpha}$-propionyl-ornithyl)-glycyl)-3-difluoro-methyl-propionyl)-L-alanine;

N-(3-(2-carboxy-1-pyrrolidinyl)-carbonyl-3-monofluoromethyl-propionyl)-L-proline;

N-(3-(N-acetyl-isoleucyl)-3-trifluoromethyl-propionyl)-L-alanine;

N-(3-(N-propionyl-isoleucyl)-3-difluoromethyl-propionyl)-L-proline;

N-(3-(N-benzoyl-isoleucyl-3-difluoromethyl-2-methyl-propionyl)-L-alanine;

N-(3-(N-phenylacetyl-isoleucyl)-3-difluoromethyl-propionyl)-L-phenylalanine;

N-(3-carboxycarbonyl-3-trifluoromethyl-propionyl)-L-proline;

N-(3-carboxy-3-monofluoromethyl-2-methyl-propionyl)-L-alanine;

N-(3-cyano-2-methyl-pent-4-ynoyl)-L-proline;

N-(3-cyano-pent-4-ynoyl)-glycine;

N-(3-N-benzoylglycyl-3-diflucromethyl-propionyl)-L-proline;

N-(3-carboxy-3-trifluoromethyl-propionyl)-alanine;

N-(3-acetyl-3-difluoromethyl-propionyl)-valine;

N-(3-pivaloyl-3-trifluoromethyl-2-methyl-propionyl)-leucine;

N-(3-carboxy-3-difluoromethyl-propionyl)-isoleucine;

N-(3-carboxy-3-difluoromethyl-propionyl)-phenylalanine;

N-(3-carboxy-3-difluoromethyl-propionyl)-L-hydroxyproline;

N-(3-cyano-3-trifluoromethyl-propionyl)-tryptophan;

N-(3-cyano-3-trifluoromethyl-propionyl)-histidine;

N-(3-carboxy-3-trifluoromethyl-propionyl)-pipecolic acid;

N-(3-carboxy-pent-4-ynoyl)-2-pyrroline carboxylic acid;

N-(3-carboxy-pent-4-ynoyl)-heptalidine;

N-(3-carboxy-3-monofluoromethyl-propionyl)-2-azetidine carboxylic acid.

The compounds of Formula 1 in which X represents a fluoromethyl group (see following general Formula 13) can be prepared by decarboxylation of the corresponding β-carboxy-propionamide derivative of the following general Formula 14.

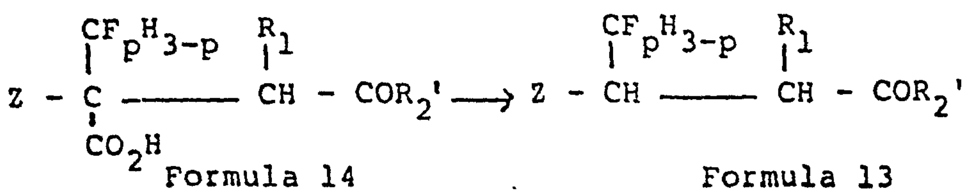

Formula 14 Formula 13

BAD ORIGINAL

In Formulae 13 and 14, Z and $R_1$ are as defined in connection with Formula 1, p represents 1, 2 or 3, and $R_2'$ is as defined for $R_2$ in connection with Formula 1 but the carboxyl group of the amino acid residue $R_2$ can be in the form of, for example, an ester or amide, in which case the carboxyl group can be freed in manner known *per se* after the decarboxylation. Conveniently, the decarboxylation can be carried out in manner known *per se* at a temperature in the range 0° to 200°C, preferably about 110°C in the presence of an acid such as trifluoroacetic acid, acetic acid, hydrochloric acid or sulfuric acid for a period in the range of 1 to 48 hours.

The β-carboxy-propionamide derivatives of Formula 14 can be prepared by acid catalysed hydrolysis or hydrogenolysis of the corresponding β-ester-propionamide derivative of the following general Formula 15.

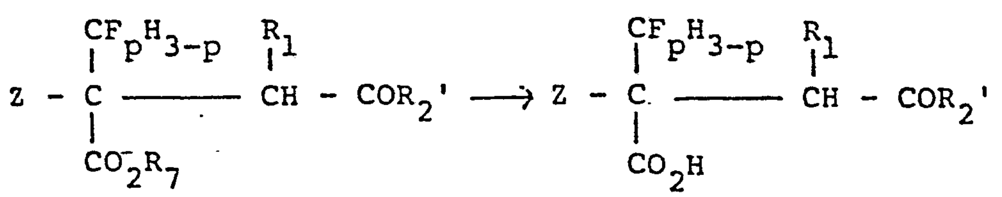

Formula 15 Formula 14

In Formula 15, p, Z, $R_1$ and $R_2'$ are as defined in connection with Formula 14 and $-CO_2R_7$ represents a

hydrolysable ester group. Suitably $R_7$ represents alkyl of 1 to 7 carbon atoms, phenylalkyl in which the alkyl moiety has 1 to 7 carbon atoms, phenyl, p-methoxyphenyl, dibenzyl, trityl or 2,2,2-trichloroethyl. Preferably, $R_7$ is alkyl, especially tert-butyl, benzyl, dibenzyl or trityl. Conveniently, the acid hydrolysis can be carried out by treatment in manner known per se with acid at room temperature for about 1 hour. Said hydrolysis can be effected as a preliminary stage in the decarboxylation supra without separation of the β-carboxy-propionamide of Formula 14. If the ester of Formula 15 is subjected to hydrogenolysis, conventional conditions can be used, for example, in an ethanol solution in the presence of a platinum or palladium catalyst, such as $PtO_2$ or Pd/C, under a hydrogen atmosphere at about 25°C for 1 to 48 hours.

If Z in Formula 15 is an ester group, the hydrolysable ester group $-CO_2R_7$ can be selected so that only the desired ester group $-CO_2R_7$ is converted into a carboxyl group thereby providing the required hemiester. It is preferred in such circumstances that $R_7$ is tert-butyl, dibenzyl or trityl because these groups are hydrolysed under relatively mild conditions which will not hydrolyse a number of other esters such as benzyl, straight chain alkyl or phenyl esters. In the case of hydrogenolysis, it is

preferred that $R_7$ is benzyl because hydrogenolysis to remove benzyl will not remove an alkyl ester constituting the group Z. However, reaction conditions can be employed which will convert an ester group Z in Formula 15 to a free acid group Z in Formula 14 or 13.

When Z in Formula 15 is a cyano group or an ester group, the $\beta$-ester propionamide derivatives of Formula 15 can be prepared by acylation of the corresponding aminoacid, in which any free carboxyl group is protected, by the corresponding butanedioic hemiester derivative of the following general Formula 16.

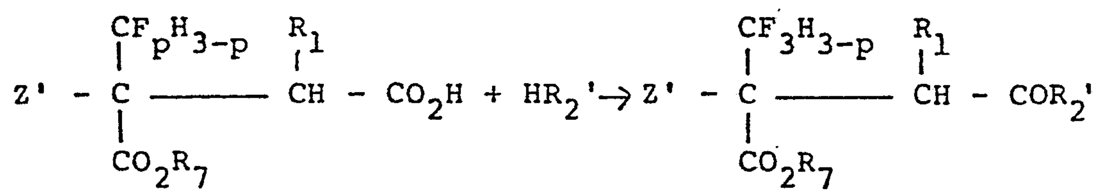

Formula 16 Formula 15A

In Formulae 15A and 16, p, $R_1$, $R_2$' and $R_7$ are as defined in connection with Formula 15 and Z' represents a cyano or ester group (i.e. $-CO_2R_{10}$ and $CO.SR_{10}$ where $R_{10}$ is as defined in connection with Formula 4 except that it does not represent hydrogen.

The acylation can be performed in manner known *per se* for example in the presence of a dehydrating agent such as dicyclohexyldiimide and in an inert solvent such as ethyl acetate, methylene chloride or acetonitrile at a temperature in the range $-70^{\circ}$ to $+100^{\circ}C$ for a period of 0.5 to ______

8 days. Usually, the acid and aminoacid reactants and the dehydrating agent will be used in equimolar amounts.

The butanedioic hemiester derivatives of Formula 16 can be prepared by selective hydrolysis of the corresponding butanedioic ester derivatives of the following general Formula 17.

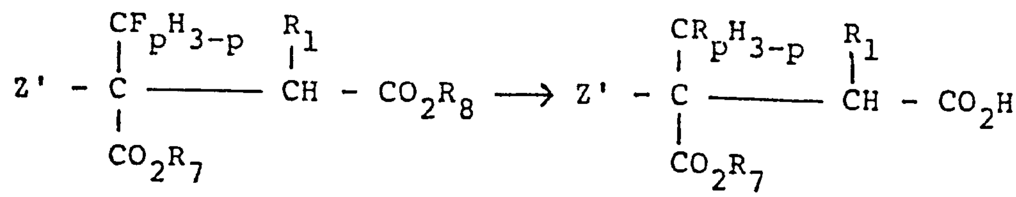

Formula 17 Formula 16

In Formula 17, p, Z', $R_1$ and $R_7$ are as defined in connection with Formula 16 and $-CO_2R_8$ represents a hydrolysable ester group. Suitably $R_8$ represents alkyl of 1 to 4 carbon atoms, benzyl, phenyl or trimethylsilylethyl. The hydrolysis is conducted under basic or neutral conditions in manner known *per se*. For example, 1 to 1.5 equivalents of a base, such as sodium hydroxide or tetraalkylammonium hydroxide, is employed in an aqueous solvent, such as aqueous alcohol or aqueous tetrahydrofuran at a temperature in the range $0^o$ to $100^oC$ for a period of 1 to 24 hours. In an alternative hydrolysis, an alkali thiolate such as lithium thiolate in an aprotic solvent such as dimethylsulfoxide or hexamethylphosphoric acid triamide can be used in manner known *per se*. In a further alternative, trimethylsilyl iodide optionally in an aprotic

solvent such as carbontetrachloride can be used in manner known per se.

It is convenient to resolve optical isomerism in the production of compounds for Formula 1 at the intermediates of Formula 16. In particular, the extracted butanedioic hemiester product obtained as above can be optically resolved using conventional techniques well known to those skilled in the art.

The butanedioic ester derivatives of Formula 17 can be prepared by fluoromethylation of the corresponding butanedioic ester derivatives of the following Formula 18.

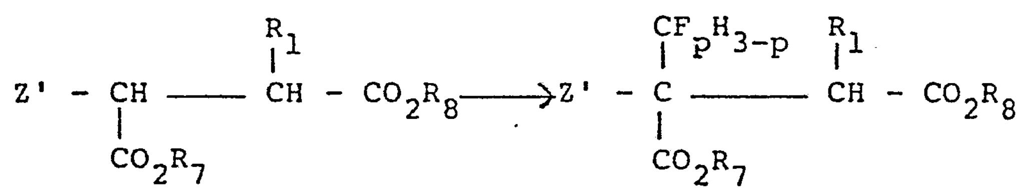

Formula 18 Formula 17

In Formula 18, Z', $R_1$, $R_7$ and $R_8$ are as defined in connection with Formula 17. The fluoromethylation can be carried out in manner known per se by adding an excess of a fluoromethylating agent of the formula $CF_pH_{3-p}W$, where p represents 1, 2 or 3 and W represents chlorine, bromine or iodine, to a solution in an aprotic solvent of a carbanion of the compound of Formula 18. The reaction can be performed by stirring at a temperature in the range -70° to +80°C for a period of 1 to 48 hours and the fluoromethylated

product of Formula 17 can be extracted from the reacted mixture by an organic solvent, such as diethylether or methylene chloride. The carbanion can be obtained in manner known *per se* by reacting the compound of Formula 18 in the aprotic solvent with a base, such as sodium hydride, potassium hydride, lithium acetylide, lithium carbide, sodamide, potassium *tert* butoxide, and lithium di*iso*propylamide at a temperature in the range 0° to 70°C for a period of 1 to 24 hours. The aprotic solvent can be, for example, diethylether, tetrahydrofuran, hexamethylphosphoric acid triamide, dimethylsulfoxide, dioxane, benzene and mixtures thereof.

The esters of Formula 18 can be prepared by alkylation with an ester derivative of the following general Formula 19 of a malonate of the following general Formula 20.

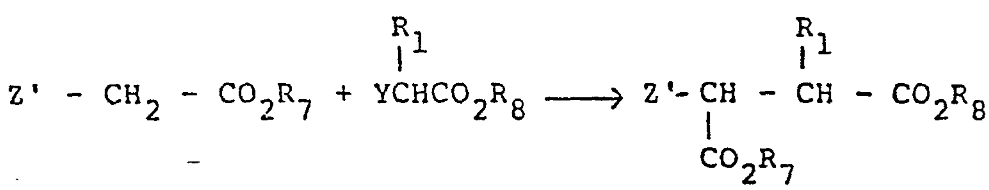

Formula 20 Formula 19 Formula 18

In Formulae 19 and 20, Z', $R_1$, $R_7$ and $R_8$ are as defined in connection with Formula 18 and Y is a leaving group, preferably chlorine, bromine, tosyloxy, or methylsulfonyloxy. The malonate alkylation can be carried out in manner known *per se* in a protic or aprotic solvent at a temperature in the range 0° to 100°C for a period

of 1 to 24 hours using any strong base which will abstract a proton from the methylene group of the malonate reactant. Suitable bases include sodium hydride, sodium ethoxide or potassium tertbutoxide. Suitable solvents include the aprotic solvents hexamethylphosphorus triamide or tetrahydrofuran and the protic solvents ethanol and tert-butanol. As well known in the art, the actual base employed is dependent upon the nature of the solvent. Usually the malonate reactant, ester reactant and base will be used in the molar ratio of 1:1 to 1.1:1 to 1.5. An optical isomer of the ester reactant can be selected to provide the required optical isomer of the product of Formula 18.

Many malonates of Formula 20 are known and some are available commercially. In general, those malonates in which Z' represents cyano can be obtained by esterification of cyanoacetic acid whilst those malonates in which Z' represents an ester group can be obtained by esterification of malonic or mercaptocarbonylacetic acid or a hemiester thereof.

The malonates of Formula 20 in which $R_7$ represents tertbutyl, dibenzyl or trityl, Z' represents $-CO_2R_7'$ and $R_7'$ represents benzyl are novel compounds which are particularly useful in preparing compounds of Formula 1. Accordingly, the present invention also

provides compounds of the following general Formula 21.

$$C_6H_5CH_2O_2C - CH_2 - CO_2 - A$$

Formula 21

wherein A represents tert-butyl, dibenzyl or trityl. Thus, the three compounds within the scope of Formula 21 are the following:-

1-tert-butyl-3-benzyl malonate;

1-dibenzyl-3-benzyl malonate; and

1-trityl-3-benzyl malonate.

The malonates of Formula 21 can be prepared in manner known *per se* by treating monobenzyl malonate with isobutylene in the presence of sulfuric acid as a catalyst at room temperature for a period of 1 to 24 hours or with diphenyl diazomethane or by treating a metal salt of monobenzyl malonate with tritylchloride or tritylbromide.

Compounds of Formula 15 in which Z represents an electron-withdrawing group other than cyano or ester, can be prepared from the compounds of Formula 15A in which Z' represents a carboxyester group $-CO_2R_{10}$ often via the corresponding acid chloride or anhydride. In particular, a compound of the following general Formula 15B can be converted into an acid of the following general Formula 22 which is then converted into an acid chloride of the following general Formula 23.

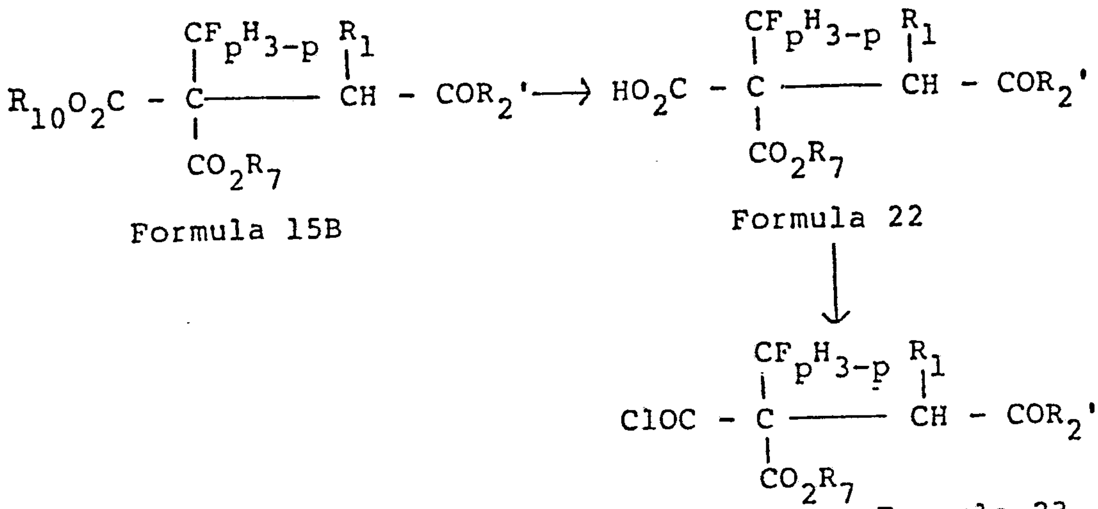

Formula 15B

Formula 22

Formula 23

In Formulae 15B, 22 and 23, p, $R_1$, $R_2$', $R_7$ and $R_{10}$ are as defined in connection with Formula 15A.

The conversion of the compounds of Formula 15B to the compounds of Formula 22 can be carried out in manner known *per se* by selective acid hydrolysis or by hydrogenolysis of one of the ester functions. Conveniently, the acid hydrolysis is achieved by treatment with trifluoroacetic acid at a temperature in the range 0° to 25°C for a period of 1 to 10 hours. Suitably, the hydrogenolysis is carried out in ethanol in the presence of a palladium catalyst, such as Pd/C, under a hydrogen atmosphere at about 25°C for a period of 1 to 48 hours. In accordance with known procedures, the ester functions and reaction conditions are chosen so that only the desired ester group is converted into the free carboxyl group. Thus, in the case of acid hydrolysis it is preferred that $R_7$ and any protecting ester group in the aminoacid residue $R_2$' are straight chain alkyl of 1 to 4 carbon atoms and that $R_{10}$ is *tert*.

butyl, dibenzyl or trityl. In the case of hydrogenolysis, it is preferred that $R_7$ and any protecting ester group in the aminoacid residue $R_2'$ is straight or branched chain alkyl of 1 to 4 carbon atoms and that $R_{10}$ is benzyl.

The formation of the acid chlorides of Formula 23 can be carried out in manner known *per se* by treatment with thionyl chloride or, preferably with the acid in the form of an alkali salt, with oxalyl chloride at -30° to +130°C for a period of 1 to 24 hours. When thionyl chloride is employed, an excess of thionyl chloride usually will be used as a solvent for the reaction. Suitably, the oxalyl chloride can be used in an equimolar amount relative to the acid reactant or in an excess of up to 3 moles per mole of acid reactant and in inert solvent, such as benzene or toluene.

The subsequent reaction of the acid chlorides of Formula 23 to obtain the desired compound of Formula 15 will depend upon the nature of the desired electron-withdrawing group Z. In general terms however said subsequent reaction will be an acylation in manner known *per se*.

When it is desired to obtain a compound of Formula 15 where Z represents $-COR_{10}$ and $R_{10}$ is as defined in connection with Formula 15A, the acid chloride of Formula 23 can be reacted in manner known *per se* with an appropriate organo-metallic reagent.

Suitable organometallic reagents include $(R_{10})_2CuLi$, $(R_{10})_2Cu$, $(R_{10})_2Cd$, $(R_{10})_2Zn$, $R_{10}ZnI$, $R_{10}MnI$, $R_{10}MgI$, $R_{10}MgBr$ and $R_{10}MgCl$. The reaction can be carried out by adding, preferably under an inert atmosphere, a solution of the acid chloride in an aprotic solvent, such a tetrahydrofuran, ether, benzene or aliphatic hydrocarbon, at a temperature in the range -78°C to room temperature to a solution of the organimetallic reagent in the same solvent at a temperature between -78°C and the boiling point of the solvent. The reaction mixture is stirred at a temperature in the range -78°C to solvent boiling point for a period of 1 to 48 hours.

When it is desired to obtain a compound of Formula 15 in which Z represents $-CONHOR_{10}$ where $R_{10}$ is as defined in connection with Formula 2, the acid chloride of Formula 23 can be treated in manner known per se with hydroxylamine or a derivative thereof of the formula $H_2NOR_{10}$. Conveniently, the reaction is carried out in a solvent such as benzene, toluene, diethylether, chloroform or carbon tetrachloride, at a temperature in the range -60° to +200°C for a period of 0.5 to 24 hours with equimolar amounts of the reactants or an excess of up to 10 molar of the hydroxylamine reactant.

When it is desired to obtain a compound of Formula 15 in which Z represents $-COCH_2-R'_{11}$ wherein $R'_{11}$ represents halogen, $-OR_{10}$, 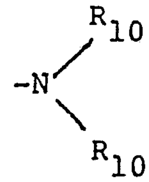, $-O.CO.R_{10}$, $-SR_{10}$, or -tosyloxy and $R_{10}$ is as defined in connection with Formula 4, the acid chloride of Formula 23 can be converted in manner known per se into a corresponding diazoketone of the following general Formula 24.

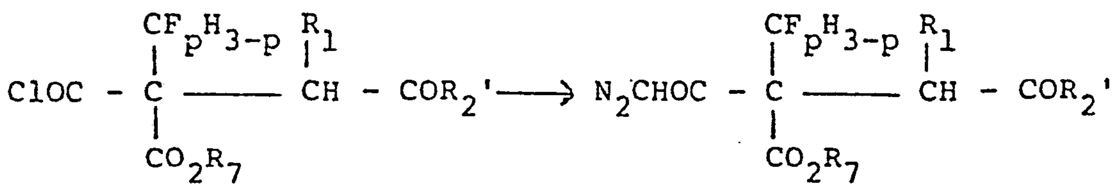

Formula 23 Formula 24

In Formula 24, p, $R_1$, $R_2'$ and $R_7$ are as defined in connection with Formula 23. Conveniently, the reaction is carried out by adding a solution of diazomethane in an ether, such as diethylether, to a solution of the acid chloride in the same solvent at a temperature in the range -30°C to +45°C, preferably room temperature. Usually an excess up to 4 molar of diazomethane will be used. The reaction mixture is stirred at a temperature in the range -30°C to +45°C, preferably room temperature, for a period of 0.5 to 24 hours, preferably about 2 hours.

If the diazoketone of Formula 24 is treated in manner known per se with an excess of hydrogen chloride,

hydrogen bromide or hydrogen fluoride in pyridine, the corresponding halogenomethylketone compound of Formula 15 is obtained in which Z represents $-COCH_2Cl$, $-COCH_2Br$ or $-COCH_2F$ respectively.

If the diazoketone of Formula 24 is treated in manner known per se with aqueous trifluoroacetic acid, followed by ethanolysis or with aqueous sulfuric acid, the corresponding hydroxymethyl ketone compound of Formula 15 is obtained in which Z represents $-COCH_2OH$.

Products of Formula 15 where Z represents $-COCO_2H$ can be obtained from corresponding compounds where Z represents $-COCH_2OH$ by methods known per se, for example Jones oxidation with pyridinium dichromate at $0^o$ to $25^oC$.

Compounds of Formula 15 in which Z represents $-COCH_2$ tosyloxy can be obtained by treating in manner known per se the corresponding hydroxymethyl ketone (Formula 15, Z = $-COCH_2OH$) with tosyl chloride in the presence of a base such as pyridine. Usually 1 to 2 moles of tosylchloride will be used per mole of the ketone reactant.

Compounds of Formula 15 in which Z represents $-CO.CH_2OCR_{10}$ where $R_{10}$ is as defined in connection with Formula 15A can be obtained by treating in manner known per se the corresponding hydroxymethyl ketone

(Formula 15, Z = $-COCH_2OH$) with an acid chloride of the formula $R_{10}COCl$ or an anhydride of the formula $(R_{10}CO)_2O$ in the presence of base e.g. 4-dimethylamino pyridine. Usually 1 to 3 moles of acid chloride or anhydride will be used per mole of the ketone reactant.

Compounds of Formula 15 in which Z represents $-COCH_2I$ can be obtained by treating in manner known *per se* the corresponding monobromo- or chloro-methyl ketone, or monomesyloxymethylketone with sodium iodide in an excess of a solvent such as acetone, or tetrahydrofuran. Conveniently, the reaction is carried out at a temperature in the range 25° to 70°C for a period of 1 to 48 hours.

Compounds of Formula 15 in which Z represents $-COCH_2OR_{10}$, where $R_{10}$ is as defined in connection with Formula 15A, can be obtained by treating in manner known *per se* the corresponding monobromo- or chloro-methyl ketone or monomesyloxymethylketone with an alkoxide or phenoxide of the formula $R_{10}O^-$ in a solvent such as diethylether, benzene or $R_{10}OH$. Conveniently, the anion $R_{10}O^-$ can be provided by the alkali metal salt such as sodium or potassium alkoxide or phenoxide and the reactants used in equimolar amounts.

Compounds of Formula 15 in which Z represents $-COCH_2SR_{10}$ can be obtained by treating in manner known *per se* the corresponding monobromo-, chloro- or iodomethyl ketone with hydrogen sulfide, sodium bisulfide or an alkylsulfide or phenylsulfide of the formula $R_{10}S^-$, where $R_{10}$ is as defined in connection with Formula 15A, in a solvent. Conveniently, the solvent can be methanol, ethanol, diethylether, dimethylsulfoxide, hexamethylphosphoric triamide or dimethylformamide. If an anion $R_{10}S^-$ is employed, it suitably can be provided by the alkali metal salt such as sodium or potassium alkylsulfide or phenylsulfide.

Compounds of Formula 15 in which Z represents

$-COCH_2NHR_{10}$ or $-COCH_2N(R_{10})_2$

wherein $R_{10}$ is as defined in connection with Formula 15A can be obtained by treating in manner known *per se* the corresponding monochloromethyl ketone, monobromomethyl ketone or monoiodomethyl ketone (Formula 15, Z = $-COCH_2Cl$, $-COCH_2Br$ or $-COCH_2I$) with the corresponding amine of the formula

$H_2NR_{10}$ or $HN(R_{10})_2$

Conveniently, the reaction can be carried out at a temperature in the range 0° to 100°C for a period of 0.5 to 24 hours in dimethylformamide, acetone, hexamethylphosphoric triamide, dimethylsulfoxide, tetrahydrofuran or ethylacetate as a solvent.

Compounds of Formula 15 in which Z represents $-COCH_2NH_2$ can be obtained in manner known *per se* from the corresponding monochloromethyl ketone, monobromomethyl ketone or monoiodomethyl ketone or monomesyloxymethylketone via an intermediate phthalimide or azido derivative. The phthalimide or azido intermediate can be obtained by treating the monohalogenoketone with sodium or potassium phthalimide or tetramethyl guanidium azide, conveniently under the reaction conditions outlined above for reaction of the monohalogenoketone with a primary or secondary amine. The phthalimide derivative can be treated with hydrazine or monomethylamine in an alkanol of 1 to 4 carbon atoms at a temperature of 0° to 80°C for a period of 1 to 24 hours to yield the desired glycyl derivative of Formula 15. Conveniently, the molar ratio of phthalimide to hydrazine is 1:1 and of phthalimide to methylamine is 1:1 to 2. The azido intermediate is reduced under conditions known *per se* to the desired glycyl derivative.

Compounds of Formula 15 in which Z represents $-COCH_2NHCOR_{12}$ can be obtained by treatment in manner known *per se* of the corresponding glycyl derivative (Formula 15, Z = $-COCH_2NH_2$) with an acid chloride of the formula $R_{12}COCl$ or an acid anhydride of the formula $(R_{12}CO)_2O$ in the presence of a base such as triethylamine. Conveniently 1 to 2 moles of acid chloride or anhydride will be used per mole of glycyl reactant and suitable solvents include ethyl acetate, benzene and diethylether. When $R_{12}$ represents $-\underset{\displaystyle NH_2}{\underset{|}{CH}}(CH_2)_nNH_2$ or $-\underset{\displaystyle NHCOR_{12}}{\underset{|}{CH}}(CH_2)_nNH_2$, the free amino group or

groups of $R_{12}$ are protected by, for example, benzyloxycarbonyl or <u>tert</u>-butoxycarbonyl before the reaction with the acid chloride or anhydride and subsequently freed by acid hydrolysis or hydrogenolysis of the reaction product. The corresponding amino-protected aminoacid can be used instead of said amino-protected acid chloride or anhydride. In said latter case, the reaction will be carried out in a solvent such a diethyl ether, dioxan, tetrahydrofuran, methylene chloride or chloroform and in the presence of a dehydrating agent such as dicyclohexylcarbodiimide.

Compounds of Formula 15 in which Z represents

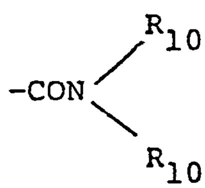 or $-COR'_2$ can be obtained by treating in manner known *per se* the corresponding acid chlorides of Formula 23 with the corresponding amine of the general formula 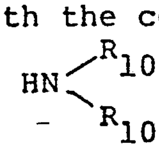 or $HR'_2$ in the presence of a base such as triethylamine. Suitable solvents for the reaction include ethyl acetate, benzene and diethyl ether.

Compounds of Formula 15 in which Z represents

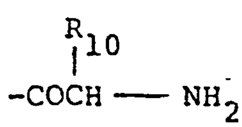 can be obtained in manner known *per se* by treating the corresponding acid chloride of Formula

23 with a Schiffs base or an isonitrile of the general Formula 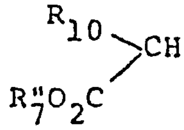 in which $-CO_2R''_7$ is a readily hydrolysable ester group, especially <u>tert</u>-butoxycarbonyl, and G represents $-N{=}C$ or $-N{=}CH.C_6H_5$. The resultant intermediate compound of the following general Formula 25 is then subjected to acid hydrolysis in manner known <u>per</u> <u>se</u> at a temperature in the range 0° to 100°C for 1 to 24 hours and then heated to cause rearrangement to the desired compound of Formula 15.

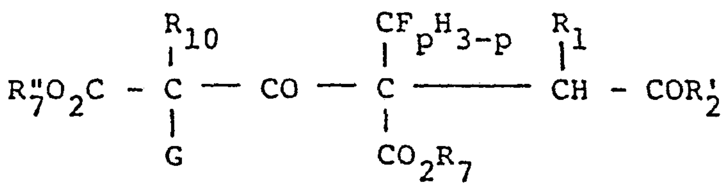

Formula 25

Compounds of Formula 15 in which Z represents $-CO\overset{R_{10}}{\overset{|}{C}}H-R_{11}$ where $R_{10}$ is as defined in connection with Formula 15A and $R_{11}$ is as defined in connection with Formula 1 can be prepared in manner known <u>per</u> <u>se</u> by diazotizing the corresponding glycyl compound using, for example, sodium nitrite in the presence of an acid. When the acid is hydrochloric, hydrobromic or sulfuric acid, the product is the corresponding monochloroalkyl-, monobromoalkyl-, or hydroxyalkyl-ketone respectively. These derivatives can be reacted in the manner described

above with reference to Formula 24 and its derivatives to provide other derivatives of Formula 15 in which Z represents $\overset{R_{10}}{\overset{|}{-COCHR_{11}}}$ .

Compounds of Formula 1 in which Z represents an electron-withdrawing group other than cyano or ester, can also be prepared from corresponding compounds of Formula 1 in which Z represents $-CO_2R_{10}$ often via the corresponding acid chloride or anhydride. In particular, they can be obtained via an acid chloride of the following general Formula 23A using the methods described above in connection with the preparation of the corresponding compounds of Formula 15.

$$ClOC - \overset{CF_pH_{3-p}}{\overset{|}{CH}} \text{———} \overset{R_1}{\overset{|}{CH}} - COR'_2$$ Formula 23A.

In Formula 23A p, $R_1$ and $R'_2$ are as defined in connection with Formula 15.

In general terms however, it is preferred to use the routes via the acid chloride of Formula 23 because of the risk of dehydrofluorination when proceeding via the acid chloride of Formula 23A. In particular, the action in manner known *per se* of a strong base on the compounds of Formula 15 can provide the corresponding fluoromethylene derivative of the following general Formula 26.

$$Z - \overset{CF_{p-1}H_{3-p}}{\overset{||}{C}} \text{———} \overset{R_1}{\overset{|}{CH}} - COR_2$$ Formula 26.

Use of said dehydrofluorination can be made to convert trifluoromethyl and difluoromethyl derivatives of Formula 1 into the corresponding difluoromethyl and monofluoromethyl derivatives respectively by subsequently catalytically hydrogenating in manner known *per se* the fluoromethylene derivatives. The dehydrofluorination can be carried out in a protic or aprotic solvent, such as water, methanol, ethanol, tetrahydrofuran, diethylether, dioxane, dimethylsulfoxide, ethyl acetate, hexamethylphosphoric triamide, benzene, carbon tetrachloride or a mixture thereof, using an equimolar amount or, when Z represents $CO_2H$, two equivalents of a base, such as sodium hydroxide, potassium hydroxide, lithium hydroxide, sodamide, sodium acetylide, lithium carbide, triethylamine or pyridine, at a temperature in the range of -20°C to solvent boiling point for a period of 0.1 to 24 hours. The catalytic hydrogenation can be carried out under a hydrogen atmosphere of 1 to 150 atmospheres at a temperature in the range 25° to 150°C for a period of 1 to 48 hours using, for example $PtO_2$, Pd/C or $RhCl(P(C_6H_5)_3)_3$ as catalyst.

The actual sequence of the stages in the reaction schemes discussed above can be varied as will be apparent to those skilled in chemical synthesis. In particular, when Z' in the butanedioic ester of Formula 17 represents a carboxylic ester group (i.e. $-CO_2R_{10}$ where $R_{10}$ is as defined in connection with

BAD ORIGINAL

Formula 15A), said group can be converted into the group Z of the required compound of Formula 1 or into a precursor of said group Z before formation of the amide function (i.e. $CO_2R_2$ or $-CO_2R_2'$) at the other end of the molecule. The appropriate selection of the ester groups $-CO_2R_7$, $-CO_2R_8$ and $-CO_2R_{10}$ and of the hydrolysis conditions employed will allow intermediates in the reaction scheme, such as compounds of Formula 17, to be selectively hydrolysed enabling the required reaction sequence to be followed.

In a particularly preferred alternative sequence to those previously discussed, the pentanetrioic triester of Formula 17A is selectively hydrolysed to the ester acid of the Formula 27.

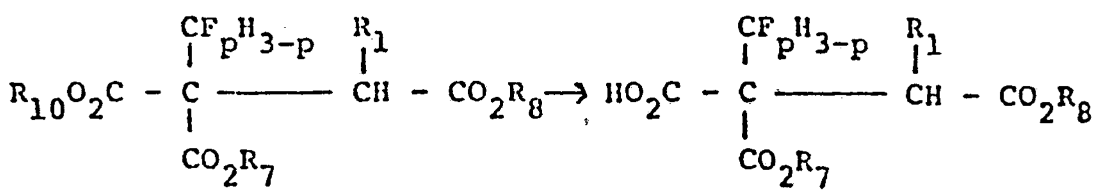

Formula 17A                Formula 27

wherein p, $R_1$, $R_7$ and $R_{10}$ are as defined in connection with Formula 15 and $R_8$ is as defined in connection with Formula 17. Preferably $R_{10}$ is tert-butyl and $R_7$ and $R_8$ are straight chain alkyl of 1 to 4 carbons, or benzyl, in which case the hydrolysis can readily be carried out using acid conditions, especially trifluoroacetic acid. The esteracid of Formula 27 can be converted into the acid chloride of Formula 28 by, for example, treatment

with thionylchloride and said acid chloride converted into the desired electron-withdrawing group Z.

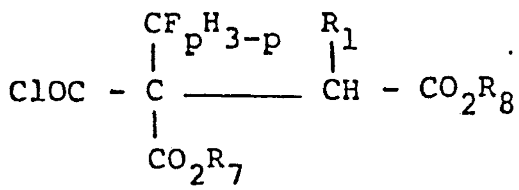

Formula 28.

Particularly preferred intermediates obtainable from the acid chlorides of Formula 28 are the 5-amino-3-fluorinated methyl-4-oxo-pentanoic acids of the following general Formula 29 and functional derivatives thereof.

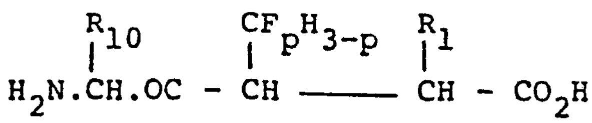

Formula 29

wherein

$R_1$ and $R_{10}$ are as defined in connection with Formula 4 and p represents 1, 2 or 3.

The compounds of Formula 29 in which $R_{10}$ represents hydrogen can be obtained by converting the acid chloride of Formula 28 into the corresponding bromomethyl ketone, converting said bromomethyl ketone into the corresponding phthalimide and hydrolysing the phthalimide. When $R_{10}$ does not represent hydrogen, the compounds of Formula 29 can be obtained by reacting the acid chloride of Formula 28 with a Schiffs base or isonitrile, subsequent hydrolysis and heating. The individual reaction stages have been discussed above in connection with the preparation of compounds of Formula 15 and the

BAD ORIGINAL

reaction sequences are shown below.

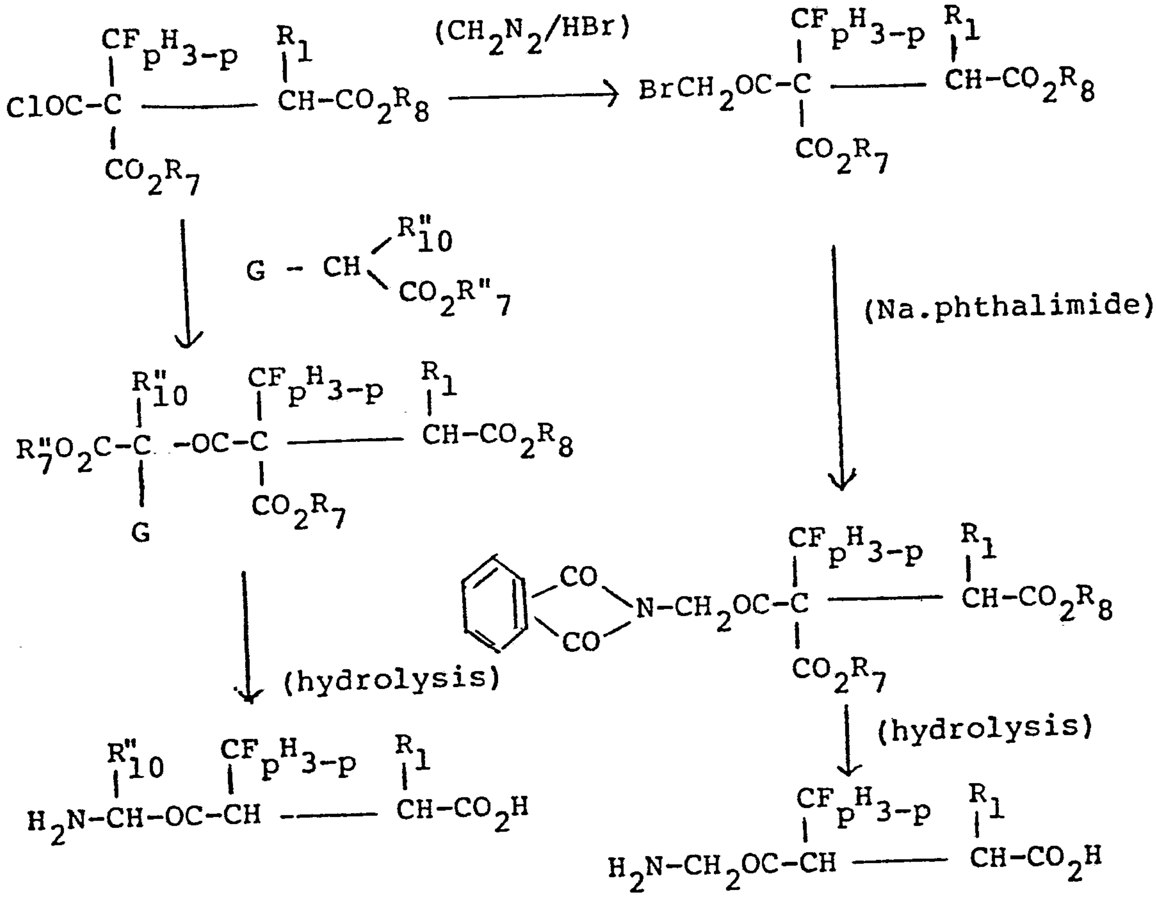

The compounds of Formula 29 are irreversible inhibitors of delta-aminolevulinic acid dehydrase and as such of use in medicine in, for example, the treatment of porphyria.

In another method of preparation of compounds of Formula 13, the corresponding protected aminoacid of the formula $HR_2'$ (where $R_2'$ is as defined in connection with Formula 13) can be acylated in manner known <u>per se</u> with the corresponding fluoro-butanoic acid derivative of the following general Formula 30 to provide an N-acyl aminoacid derivative of the following general Formula 31 which subsequently can be converted into a compound of Formula 13

$$Z''-CH(CF_pH_{3-p})-CH(R_1)-CO_2H + HR_2' \rightarrow Z''-CH(CF_pH_{3-p})-CH(R_1)-COR_2'$$

Formula 30 Formula 31.

In Formulae 30 and 31, $p$, $R_1$ and $R_2'$ are as defined in connection with Formula 13 and Z" represents the electron-withdrawing group Z or a protected form of said group Z. If Z" represents a protected form of the group Z, it subsequently will be converted in manner known <u>per se</u> into the group Z and said conversion can be carried out before or after the carboxyl group of the aminoacid is freed.

The acylation can be carried out in the manner described above in connection with the acylation of compounds of Formula 16. A further example of a suitable dehydrating agent for said acylations is N-ethoxycarbonyl-2-ethoxy-1,2-dihydroquinoline.

The compounds of Formula 30 can be prepared using the appropriate reaction steps described above in connection with the preparation of compounds of Formula 13. However, when p is 3, the preparations described below are preferred.

The cyano trifluoro compound of Formula 30 can be prepared———————————————— in manner known *per se* by treating the corresponding *tert*-butyl-trifluorocrotonate with potassium or sodium cyanide in the presence of acetic acid or ammonium chloride at about 0°C in ethanol solution for a period of 2 to 48 hours, and subsequent hydrolysing of the *tert*-butyl ester using, for example, trifluoroacetic acid. The *tert*-butyl-trifluorocrotonate can be prepared from the corresponding trifluorocrotonic acid by treatment with isobutylene in methylene chloride using sulfuric acid or trifluoromethane sulfonic acid as catalyst at about 25°C for a period of 12 to 24 hours. Alternatively, the said acid can be treated with N,N'-di(isopropyl)-*tert*-butoxyamidine at about 25°C for a period of 12 to 24

hours in a solvent such as methylene chloride.

In the particular case where Z in Formula 13 represents benzoyl or p-methoxybenzoyl, it is preferred that when p is 3, Z" in Formulae 30 and 31 represents 2-phenyldithian-2-yl or 2-(p-methoxyphenyl)dithian-2-yl respectively. Said Z" group can readily be converted in manner known *per se* into the required benzoyl or p-methoxybenzoyl group by treatment with 1 to 6 molar excess N-bromosuccinimide in aqueous acetonitrile or aqueous tetrahydrofuran at a temperature in the range 0° to 25°C for a period of 1 min. to 4 hours and optionally in the presence of a buffer such as calcium carbonate. The conversion can also be carried out in manner known *per se* (a) using mercuric chloride in aqueous tetrahydrofuran at a temperature in the range 0° to 60°C for a period of 1 to 14 hours or (b) using methyl iodide in aqueous acetonitrile at about 50°C for about 12 hours or (c) using trichloroisocyanuric acid in the presence of silver nitrate in aqueous acetonitrile at a temperature in the range 0° to 25°C for a period of 1 to 60 mins. Usually, the aqueous solvents used in all said methods of converting the phenyl dithianyl group into the benzoyl group will be about 4 parts solvent per part of water (by volume).

The phenyldithian-2-yl trifluoro compounds of Formula 30 can be prepared by treating in manner known per se the carbanion derived from the corresponding phenyl-dithiane with an alkyl, especially methyl, ethyl or tert-butyl, trifluorocrotonate, optionally in the presence of hexamethyl phosphoric acid triamide, subsequent addition of water (when $R_1$ is hydrogen) or an iodide of the formula $R_1I$ (when $R_1$ is not hydrogen) and then hydrolysis of the resultant trifluoromethyl-3-(2-phenyl-dithian-2-yl) propionic acid. The carbanion can be generated in tetrahydrofuran solution at a temperature in the range -70° to -30°C by treatment of the phenyl-dithiane with n-butyl lithium optionally in the presence of hexamethylphosphoric acid triamide. The crotonate in tetrahydrofuran can be added to the carbanion solution and the mixture maintained at a temperature in the range -70° to -30°C for a period of 1 to 90 minutes. The iodide or water is then added and, in the case of addition of the iodide, the solution maintained for about a further hour at a temperature in the range -70° to -25°C before adding water. When the product is an n-alkyl ester, it can be hydrolysed by treatment with aqueous alcohol containing sodium, potassium or lithium hydroxide at a temperature in the range 25° to 60°C for a period of 1 to 12 hours. When the product is a tert-butyl ester, the hydrolysis can

be carried out using trifluoroacetic acid.

In the particular case where Z in Formula 13 represents carboxycarbonyl, it is preferred that when p is 3 Z" in Formulae 30 and 31 represents 2-(*tert*-butoxycarbonyl)-dithian-2-yl, which can be converted into the required carboxycarbonyl group by hydrolysis to the 2-carboxydithian-2-yl and subsequent treatment by any of the methods described above for conversion of phenyldithianyl to benzoyl.

The butoxycarbonyldithianyl trifluoro compounds of Formula 30 can be prepared in manner known *per se* by an analogous synthesis to that of the phenyldithianyl compounds of Formula 30 described above but using lithium diisopropylamide to generate the carbanion from 2-*tert*-butoxycarbonyl dithiane.

In the particular case where Z in Formula 13 represents carboxyl, it is preferred that when p is 3 Z" in Formulae 30 and 31 represents 2-cyanodithian-2-yl or tris(phenylthio)methyl, which can be converted into the required carboxyl group by any of the methods described above for conversion of phenyldithianyl to benzoyl.

The cyanodithianyl trifluoro compounds of Formula 30 can be prepared in manner known *per se* by an analogous synthesis to that of the phenylthianyl compounds of

Formula 30 described above but using an anion derived from 2-cyanodithiane by treatment with lithium diisopropylamide. The cyanodithiane reactant can be prepared in manner known *per se* by dehydrating 2-carbamoyldithiane, for example, by treatment with phosphorous oxychloride, optionally in a solvent such as dichloroethylene, at a temperature in the range 50° to 100°C for a period of 1 to 12 hours. 2-carbamoyldithiane can be prepared in manner known *per se* from 2-methoxycarbonyldithiane by treatment with aqueous or alcoholic ammonia or from 2-carboxydithiane by treatment with thionylchloride to form the acid chloride which is then treated with aqueous ammonia.

The tris(phenylthio)methyl trifluoro compounds of Formula 30 can be prepared in manner known *per se* by an analogous synthesis to that of the phenyldithianyl compounds of Formula 30 described above but using the carbanion derived from triphenylthioorthoformate.

In the particular case where Z in Formula 13 represents formyl, it is preferred that when *p* is 3 Z" in Formulae 30 and 31 represents bis(phenylthio)methyl which can be converted into the required formyl group by any of the methods described above for conversion of phenyldithianyl to benzoyl. The formyl group can be converted to the acid by methods known *per se*.

The bis(phenylthio)methyl trifluoro compounds of Formula 30 can be prepared in manner known *per se* by, for example, treating the corresponding tris(phenylthio) methyl compounds of Formula 30 with a 2 to 6 fold excess of chromium (II) chloride in dimethylformamide-water (4:1) at about 100°C for a period of 1 to 3 hours. Alternatively, said tris(phenylthio)methyl compound can be treated with lithium propyl or butyl mercaptide in hexamethylphosphoric acid triamine at a temperature in the range 0° to 25°C for a period of 1 to 60 minutes to yield the desired bis(phenylthio) methyl compound.

In the particular case where Z in Formula 13 represents methylcarbonyl, it is preferred that when *p* is 3, Z" in Formulae 30 and 31 represents α-methoxyvinyl or α-ethoxyvinyl which can be converted in manner known *per se* into the required methylcarbonyl group. For example, the alkoxyvinyl compound of Formula 31 can be treated with trifluoroacetic acid at a temperature in the range 0° to 25°C for a period of 1 to 20 hours followed by treatment with water at about 25°C for 10 to 60 minutes. Alternatively, the alkoxyvinyl compound of Formula 31 can be treated with aqueous, especially 1N, hydrochloric acid in methanol, preferably in about equal parts by volume, at about 25°C for

BAD ORIGINAL

a period of 12 to 24 hours. In both reactions, the protecting group of the aminoacid carboxyl can be removed.

The α-alkoxy trifluoro compounds of Formula 30 can be prepared in manner known *per se* by treating the corresponding ethyl trifluorocrotonate with the cuprate reagent derived from α-alkoxyvinyl lithium at a temperature in the range -70° to 0°C for a period of 1 to 60 minutes and subsequently hydrolysis of the resultant trifluoromethyl-4-alkoxy-pent-4-enoic acid, ethylester with, for example, ethanolic hydroxide. The cuprate reagent can be prepared by the method described by Boechman *et al* (J. Chem. Soc. (London) Chem. Comm. 1975, 519).

Compounds of Formula 13 in which Z represents a carbonyl-terminated functional group, for example $-COR_{10}$, $-COR_2'$, $-CON\begin{matrix} R_{10} \\ R_{10} \end{matrix}$, $-CONHOR_{10}$ and

$$-CO-\overset{R_{10}}{\overset{|}{CH}}-R_{11},$$

where $R_2'$ is as defined in connection with Formula 13 and $R_{10}$ and $R_{11}$ are as defined in connection Formula 4, can be obtained from compounds of Formula 31 in which Z" represents 2-cyanodithian-2-yl or tris(phenylthio)methyl by conversion of said

group Z" into carboxyl and subsequent reaction of the resultant acid in manner known *per se*. In particular, synthesis from compounds of Formula 13 in which Z represents carboxyl can be conducted in the manner described above in connection with the preparation of compounds of Formula 15.

The compounds of Formula 1 in which X represents an ethynyl group (see following general Formula 32) can be prepared by acylating in manner known *per se* the corresponding protected amino acid of the formula $HR_2'$ (where $R_2'$ is as defined in connection with Formula 13) with the corresponding pent-4-ynoic acid of the following general Formula 33 to provide an N-acyl amino acid derivative of the following general Formula 34 which subsequently can be converted into a compound of Formula 32.

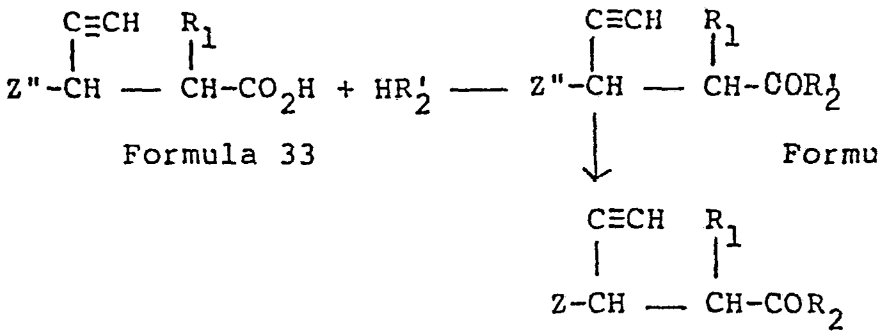

Formula 33

Formula 34

Formula 32

In Formulae 32, 33 and 34, $R_1$ and $R_2$ are as defined in connection with Formula 1, $R_2'$ is as defined in connection with Formula 15 and Z" represents the electron-withdrawing group Z if said group Z is inert under the reaction conditions employed or a protected form of said group Z. If Z" represents a protected form of the group Z, it subsequently will be converted in manner known *per se* into the group Z and said conversion can be carried out before or after the carboxyl group of the amino acid is freed.

When Z in Formula 32 represents cyano, it is unnecessary to use a protected form of the cyano group in the reactant of Formula 33 (i.e. Z" can represent cyano). However, it usually will be necessary to use a protected form of carbonyl-terminated functional groups represented by Z in Formula 32.

The acylation can be carried out in the manner described above in connection with the acylation of the compounds of Formulae 16 and 30.

In general terms, the preferred groups Z" will correspond to the required carbonyl-terminated group Z in the same manner as described above in connection with conversion of compounds of Formula 30 into compounds of Formula 13. Thus, Z" can represent (a) 2-phenyldithian-2-yl or 2-(*p*-methoxyphenyl) dithian-2-yl as protected forms of benzoyl and *p*-methoxy-benzyl; (b) 2-(*tert*-butoxycarbonyl)dithian-2-yl as a protected form of carboxycarbonyl; (c) 2-cyanodithian-2-yl or tris(phenylthio)methyl as protected forms of carboxyl; (d) bis(phenylthio) methyl as a protected form of formyl; and (e) α-methoxyvinyl or α-ethoxyvinyl as protected forms of methylcarbonyl. Said exemplified groups Z" can be converted into their corresponding groups Z in the manner described above in connection with the preparation of compounds of Formula 13. Further, compounds of Formula 32 in which Z represent a carbonyl-terminated functional group, for example $-COR_{10}$,

$$-COR_2', \quad -CON\begin{matrix} R_{10} \\ R_{10} \end{matrix}, \quad -CONHOR_{10} \quad \text{and} \quad -\overset{R_{10}}{\overset{|}{COCH}} - R_{11},$$

where $R_2'$ is as defined in connection with Formula 13 and $R_{10}$ and $R_{11}$ are as defined in connection with Formula 4, can be prepared from compounds of Formula 34 in which Z" represents carboxyl by analogous

methods to those described above in connection with analogous conversions of compounds of Formula 13.

The cyano compound of Formula 33 (i.e. Z" = cyano can be prepared in manner known *per se* by treating an alkyl (1 to 4 carbon atoms) ester of a 5-trimethylsilyl-pent-4-ynoic acid with a mixture of acetic acid and potassium or sodium cyanide in methanol or ethanol at a temperature in the range 0° to 25°C for a period of 1 to 48 hours and then hydrolysing the ester group by treatment with, for example, aqueous alcoholic lithium, sodium or potassium hydroxide at a temperature in the range 0° to 25°C for a period of 70 to 180 minutes.

The alkylesters of 5-trimethylsilyl-pent-4-ynoic acid can be prepared by conventional esterification of the corresponding free acid using, for example, diazomethane to obtain the methyl ester or alkanol and dicyclohexylcarbodiimide to obtain the higher esters.

5-Trimethylsilyl-pent-4-ynoic acids can be prepared in manner known *per se* by oxidation of the corresponding 5-trimethylsilyl-pent-2-ene-4-ynyl alcohol using, for example, Jones reagent (i.e. chromic anhydride in dilute sulfuric acid) at about 25°C for a period of 1 to 24 hours. Said alcohol can be prepared by trimethylsilylation in manner known *per se* of the corresponding pent-2-ene-4-ynyl alcohol by,

for example, treatment with an alkyl magnesium bromide, such as ethylmagnesium bromide, or an alkyl lithium, such as *n*-butyl lithium, in tetrahydrofuran solution at a temperature in the range -70° to 0°C for a period of 1 to 2 hours followed by addition of trimethylsilyl chloride. The resultant trimethylsiloxy derivative is then hydrolysed using, for example, aqueous hydrochloric acid at about 25°C for a period of 1 to 60 minutes.

The dithianyl, phenylthiomethyl and alkoxyvinyl compounds of Formula 33 can be prepared by analogous methods to those described above in connection with the preparation of analogous compounds of Formula 30 but using the corresponding alkyl 5-trimethylsilyl-pent-2-ene-4-ynate instead of the alkyl fluorocrotonate.

The compounds of Formula 1 inhibit angiotensin-converting enzyme and are useful in treating conditions in which said enzyme is implicated, especially hypertension in mammals. It is believed that the mechanisms involved in the cardiovascular effects of angiotensin-converting enzyme inhibitors include the following:-

(a) decreased peripheral resistance due both to inhibition of angiotensin II vasoconstriction and preservation of the vasodilator kinins such as

bradykinin;

(b) decreased plasma volume due to inhibition of aldosterone secretion, inhibition of release of the antidiuretic hormome and inhibition of the direct effects of angiotensin II on renal sodium readsorption; and

(c) potential direct vasodilator properties. The compounds of Formula 1 therefore are of use in the treatment of hypertension of all aetiologies, low output heart failure, shock and acute renal failure. Said compounds are believed to inhibit also enkephalinases and hence to be of use as analgesics in that they inhibit the conversion of the endogenous analagesic enkephaline. Further, the compounds of Formula 1 in which $R_1$ represents hydrogen are believed to inhibit delta-aminolevulinic acid dehydrase.

The compounds can be administered in various manners to achieve the desired effect. They can be administered alone or more usually in the form of pharmaceutical preparations to the patient being treated either orally or parenterally, for example, subcutaneously, intravenously or interperitoneally. The amount of the active compound ——————

administered will vary and can be any effective amount. Depending upon the patient, the condition being treated and the mode of administration, the quantity of active compound administered may vary over a wide range to provide from about 0.1 mg/mg (milligram per kilogram) to about 300 mg/kg of body weight of the patient per day. Unit dosage forms of these compounds can contain, for example, from about 5 mg to 2000 mg, especially from about 10 to 250 mg, of the compounds and may be administered, for example, from 1 to 4 times daily.

As used herein the term patient is taken to mean warm blooded animals, such as, mammals, for example, cats, dogs, rats, mice, guinea pigs, sheep, horses, bovine cows and humans.

The term "unit dosage form" is used herein to mean a physically discrete unit containing an individual quantity of the active ingredient in admixture with or otherwise in association with the carrier, said quantity being such that one or more units are normally required for a single therapeutic administration or that, in the case of severable units such as scored tablets, at least one fraction such as a half or quarter of a severable unit is required for a single therapeutic administration.

In the composition aspect of the invention

there are provided pharmaceutical formulations in which form the active compounds of the invention will normally be utilized. Such formulations are prepared in a manner well known *per se* in the pharmaceutical art and usually comprise at least one active compound of the invention in admixture or otherwise in association with a pharmaceutically acceptable carrier or diluent therefor. For making these formulations the active ingredient will usually be mixed with a carrier, or diluted by a carrier, or enclosed or encapsulated in a capsule, cachet, paper or other container. A carrier or diluent may be solid, semi-solid or liquid material which serves as a vehicle, excipient or medium for the active ingredient. Some examples of such diluents or carriers are lactose, dextrose, sucrose, sorbitol, mannitol, starches, gum acacia, calcium phosphate, liquid paraffin, oil of theobroma, alginates, tragacanth, gelatin, syrup B.P., methyl cellulose, polyoxyethylene sorbitan monolaurate, methyl- and propyl-hydroxybenzoate, talc, magnesium stearate or mineral oil.

The formulations of the invention may be adapted for enteral or parenteral use and may be administered to the patient in the form of tablets, capsules, suppositories, solutions, suspensions or

the like.

The solid unit dosage forms can be of the conventional type. Thus, the solid form can be a capsule which can be of the ordinary gelatin type containing an active compound of this invention and a carrier, for example, lubricant and inert fillers, such as, lactose, sucrose and corn starch. In another embodiment, the active compounds are tableted with conventional tablet bases such as lactose, sucrose or corn starch in combination with binders, such as, acadia, corn starch or gelatin, disintegrating agents, such as, corn starch, potato starch or alginic acid and a lubricant such as stearic acid or magnesium stearate.

For parenteral administration the compounds may be administered as injectable dosages of a solution or suspension of a compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable or synthetic origin, for example, peanut oil, soybean oil and mineral oil. In general, water, saline, aqueous dextrose and related solutions, ethanols and glycols,

such as, propylene glycol or polyethylene glycol are prepared liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials, such as, biodegradable polymers or synthetic silicones, for example, Silastic, silicone rubber manufactured by the Dow-Corning Corporation.

In the specific Examples included hereinbelow illustrative examples of suitable pharmaceutical formulations are described.

The present invention also includes the 3-ethynyl analogues of the compounds of Formula 29, which analogues have the following general Formula 35.

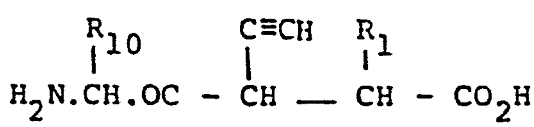

Formula 35

$R_1$ and $R_{10}$ in Formula 35 are as defined in connection with Formula 4.

The compounds of Formula 35 can be prepared by an analogous procedure to that described for the compounds of Formula 29 and both are also irreversible inhibitors of delta-aminolevulinic acid dehydrase.

Examples of preferred compounds of Formulae 29 and 35 are:-

3-glycyl-4,4,4-trifluoro-butanoic acid

3-glycyl-4,4-difluoro-butanoic acid

3-glycyl-4-monofluoro-butanoic acid

3-glycyl-pent-4-ynoic acid

3-glycyl-4,4,4-trifluoro-2-methyl-butanoic acid

3-glycyl-4,4-difluoro-2-methyl-butanoic acid

3-glycyl-4-monofluoro-2-methyl-butanoic acid

3-glycyl-2-methyl-pent-4-ynoic acid.

The references above to dose levels, modes of administration and pharmaceutical compositions apply also to the compounds of Formulae 29 and 35.

The invention is further illustrated by the following Examples. All references in the Examples to a "desired" compound mean the compound named in the immediately preceding heading of the relevant Example. Further, all references to a "step" mean the relevant step of the same Example unless an Example number is specifically stated.

EXAMPLE 1

2(tert butoxycarbonyl)butanedioic acid, 1-tert-butyl, 4-methyl ester

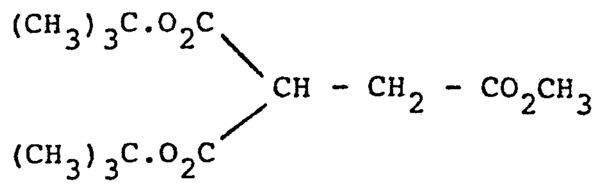

Di tert butyl malonate (100 mM, 21.6 g) is added, at room temperature and under nitrogen, to a suspension of sodium hydride (110 mM, 4.950 g of a 55% dispersion in oil) in tetrahydrofuran (280 ml). After stirring for 1 hour, a solution of α-bromo methylacetate (100 mM, 15.3 g) in tetrahydrofuran (20 ml) is added dropwise over a period of 15 minutes. Stirring is continued for 48 hours at room temperature. The mixture is then hydrolized and extracted twice with diethyl ether. The organic layer is dried over anhydrous magnesium sulfate and concentrated to dryness in vacuo. The desired triester compound is isolated by distillation under reduced pressure:

bp : 105-108°C (0.5 mm Hg)

Yield: 18.7 g (about 65%)

## EXAMPLE 2

**2-Difluoromethyl-2-tert-butoxycarbonyl butanedioic acid, 1-tert-butyl, 4-methyl ester**

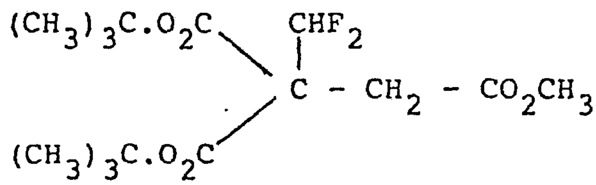

2-*tert*-butoxycarbonyl butanedioic acid, 1-*tert* butyl, 4-methyl ester (40 mM, 11.520 g) prepared in Example 1 is added, at room temperature and under nitrogen, to a suspension of sodium hydride (120 mM, 5.400 g of a 55% dispersion in oil) in tetrahydrofuran (200 ml). After stirring for one hour, a stream of chlorodifluoromethane is rapidly bubbled through the anion solution. Stirring is continued for 20 hours and the mixture is quenched with water and extracted twice with diethyl ether. The organic layer is dried over anhydrous magnesium sulfate, and concentrated to dryness *in vacuo*. The residual oil is crystallized from diethyl ether/petroleum ether mixture to yield the desired difluoromethylated triester:

mp : 49-50°C

Yield: 12.100 g (about 90%)

EXAMPLE 3

2-Difluoromethyl-2-*tert*-butoxycarbonyl butanedioic acid, 1-*tert*-butyl ester

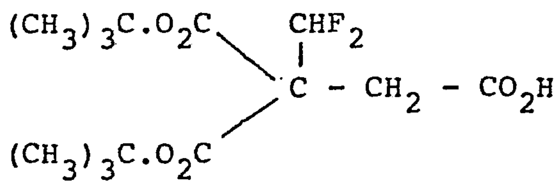

A solution of sodium hydroxide (15 mM, 0.6 g) in water (10 ml) is added at room temperature to a solution of 2-difluoromethyl-2-(*tert*-butoxy carbonyl)butanedioic acid, 1-*tert*-butyl 4-methyl ester (10 mM, 3.380 g) prepared in Example 2 in tetrahydrofuran (60 ml). After stirring for 20 hours at room temperature, water is added and the mixture is extracted with diethyl ether. The aqueous layer is acidified to pH 3 with 1 N HCl and twice extracted with diethyl ether. The organic layer is dried over anhydrous magnesium sulfate and concentrated to dryness *in vacuo* to yield a white solid (yield 3.200 g, about 98%) which was recrystallized from ether/pentane to provide the desired diester acid.

mp : 125°C

EXAMPLE 4

2-Difluoromethyl-2-[2L-(tert-butoxycarbonyl)-1-pyrrolidyl carbonyl-methyl]propanedioic acid, bis [tert-butyl] ester (otherwise N-[3-difluoromethyl-3,3-bis (tert-butoxycarbonyl)-propionyl]-L-proline), tert butyl ester

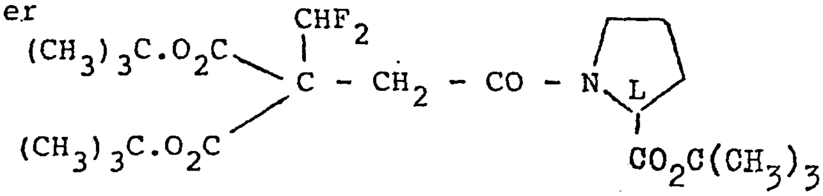

Dicyclohexylcarbodiimide (5.1 mM, 1.050 g) is added to a solution of L-proline tert-butyl ester (5.1 mM, 0.875 g) in methylene chloride (20 ml) at 0°C. 2-Difluoromethyl-2-(tert-butylcarbonyl)butanedioic acid, 1-tert-butyl ester (5.1 mM, 1.650 g) prepared in Example 3 is added and the mixture is stirred 1 hour at 0°C, and 72 hours at room temperature. The precipitate is filtered off, rinsed thoroughly with ethyl acetate and the filtrate concentrated to dryness in vacuo. The residual oil is triturated with diethyl ether and the insoluble material filtered off. The crude mixture is chromatographed on a silica gel column (150 g) with ethyl acetate/cyclohexane (1:4). The fractions containing the desired amide are concentrated to dryness and the residue is crystallized from ether pentane.

Yield: 1.800 g about 78%

mp: 92-93°C

EXAMPLE 5

2-Difluoromethyl-4-oxo-4-(2L-carboxy-1-pyrrolidyl) butanoic acid (otherwise N-(3-difluoromethyl-3-carboxy-propionyl)-L-proline

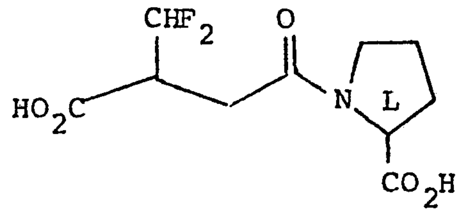

2-Difluoromethyl-2-[2L-*tert*-butoxycarbonyl)-1-pyrrolidyl carbonyl-methyl]propanedioic acid, bis [*tert*-butyl] ester (0.475 g, 1 mM) prepared in Example 4 is dissolved in trifluoroacetic acid (5 ml) at room temperature. After stirring for 1.5 hours at room temperature, the solvent is evaporated *in vacuo* yielding a white solid. After gentle heating at 40°C *in vacuo*, the oily residue is triturated with diethyl ether and evaporated to dryness several times to yield 0.280 g of the desired acid as a colorless oil.

NMR ($CDCl_3$ + ε $CF_3CO_2H$): δ ppm 6.02 (doublet of triplet) $J_{HF}$ = 53 Hz, $J_{HH}$ = 3Hz, $-CHF_2$.

EXAMPLE 6

2-Ethoxycarbonyl-butanedioic acid, 1-*tert*-butyl, 4-ethyl ester

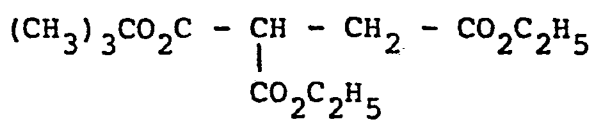

The required compound is prepared by treating

tert-butyl ethyl malonate with α-bromo-ethylacetate in substantially the manner described in Example 1. b.p. 95°C (0.5 mm Hg).

## EXAMPLE 7

**2-Difluoromethyl-2-ethoxycarbonyl-butanedioic acid, 1-tert-butyl-4-ethyl ester**

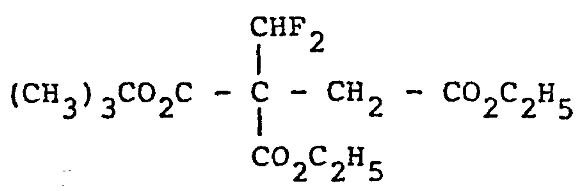

The required compound is prepared by treating 2-ethoxycarbonyl-butanedioic acid, 1-tert-butyl, 4 ethyl ester prepared in the preceding Example with chlorodifluoro-methane in substantially the manner described in Example 2. b.p. 96°C (0.2 mm Hg).

## EXAMPLE 8

**2-Difluoromethyl-2-ethoxycarbonyl-butanedioic acid, 4-ethyl ester**

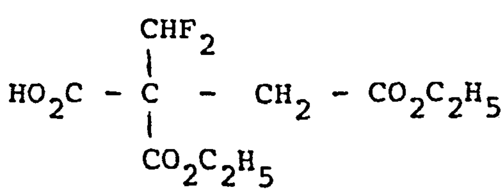

The required compound is prepared by treating

2-difluoromethyl-2-ethoxycarbonyl-butanedioic acid, 1-<u>tert</u>-butyl, 4 ethyl ester prepared in the preceding Example with trifluoroacetic acid at room temperature substantially in the manner described in Example 26 hereinafter. NMR ($CDCl_3$) $\delta$ ppm 6.35 (triplet) $J_{HF}$=55Hz, $-CHF_2$.

## EXAMPLE 9

**2-Difluoromethyl-2-ethoxycarbonyl-butanedioic acid-1-acid chloride-4-ethyl ester**

$$\begin{array}{ccccc} & & CHF_2 & & \\ & & | & & \\ ClOC & - & C & - & CH_2 - CO_2C_2H_5 \\ & & | & & \\ & & CO_2C_2H_5 & & \end{array}$$

2-Difluoromethyl-2-ethoxycarbonyl-butanedioic acid, 4-ethyl ester (5.2 g, 19.4 moles prepared in Example 8 is heated in 50 ml of thionyl chloride at 75°C for 2 hours. The excess of thionyl chloride is removed under reduced pressure and short path distillation (100-150°, 0.5 mm) affords 5.1 g of the expected acid chloride as an oil. I.R. (neat) 1800 $cm^{-1}$ (COCl).

## EXAMPLE 10

5-Bromo-3-ethoxycarbonyl-3-difluoromethyl-4-oxo pentanoic acid ethyl ester

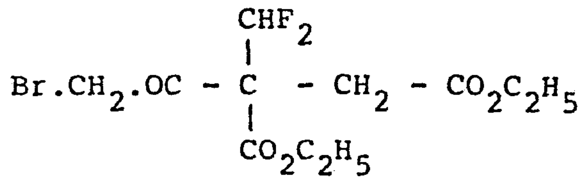

A solution of the acid chloride product of Example 9 (2.7 g ; 0.92 mmole) in 25 ml of dry diethyl ether is added at once to 100 ml of a 0.5 M solution of diazomethane in ether cooled with an ice bath. The mixture then is stirred for 2 hours at room temperature. After cooling with an ice bath, dry gaseous hydrobromic acid is passed through the reaction mixture until nitrogen evolution cases. The ether is removed under reduced pressure and the oily residue freed from hydrobromic acid by dissolution in benzene followed by evaporation under reduced pressure. The resulting oil is chromatographied over 75 g of silica gel and eluted with benzene to afford 1.7 g of the desired bromoketone contaminated with the corresponding chloroketone (about 25%). NMR ($CDCl_3$) $\delta$ ppm 6.3 (triplet) $J_{HF}$ = 56 Hz, $CHF_2$; 4.31 (singlet) $CH_2Br$.

EXAMPLE 11

3-Ethoxycarbonyl-3-difluoromethyl-5-phthalimido-4-oxo pentanoic acid ethyl ester

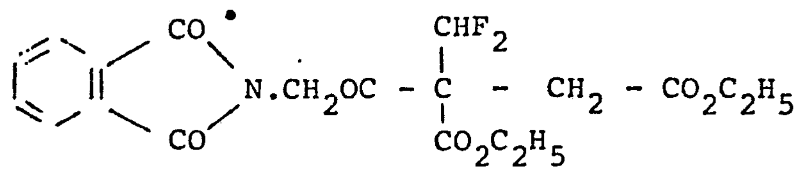

A mixture of the bromoketone product of Example 10 (1.29 g) containing 25% of chloroketone and potassium phthalamide (0.885 g ; 4.7 mmole) is stirred at room temperature in dimethyl formamide (20 mL) for 20 hours. The black mixture is diluted with 400 ml of water and then extracted with ether and usual treatment affords a viscous oil which is chromatographied over 70 g of silica gel. Elution with methylene chloride gives 0.75 g of the expected phthalimide derivative as a viscous oil. NMR ($CDCl_3$) $\delta$ ppm 6.4 (triplet) $J_{HF}$ = 53 Hz $CHF_2$; 4.86 (singlet) $CH_2N$.

EXAMPLE 12

5-Amino-3-difluoromethyl-4-oxo-pentanoic acid

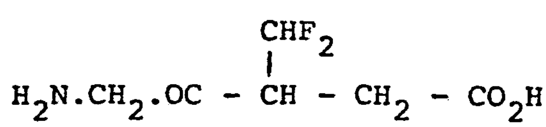

The phthalimido derivative of Example 11 is boiled in 6N hydrochloric acid for 24 hours to yield the desired aminoketonepentanoic acid. The solution is concentrated in vacuo and the residue extracted several times with hot chloroform, then dissolved in water and passed through an Amberlite cationic ionexchange column (polystyrene with sulfonic acid residues). Elution with 2M aqueousHCl affords the desired aminoketonopentanoic acid in the form of its hydrochloride.

EXAMPLE 13

5-Acetamido-3-difluoromethyl-4-oxo-pentanoic acid

$$CH_3CONH - CH_2 - OC - \overset{CHF_2}{CH} - CH_2 - CO_2H$$

The aminoacid derivative of Example 12 (2mM) is dissolved in 1M aqueous sodium hydroxide (4 mM) and the solution cooled to 0°C. Acetic anhydride (6 mM) and 0.5 M aqueous sodium hydroxide (2 mM) are added separately but simultaneously whilst the temperature is maintained at 0°C. The solution is maintained at 25°C for 4 hours and then acidified with 1M sulfuric acid and then extracted with ethyl acetate to give the desired acetamido compound.

EXAMPLE 14

N(5-Acetamido-3-difluoromethyl-4-oxo-pentanoyl) L-proline, 1-tert-butyl ester

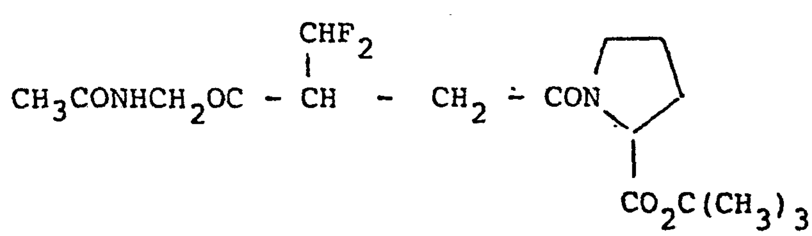

The acetamido compound of Example 13 is treated with L-proline tert-butyl ester in substantially the manner described in Example 4 to yield the required compound.

## EXAMPLE 15

**N-(5-Acetamido-3-difluoromethyl-4-oxo-pentanoyl) L-proline**

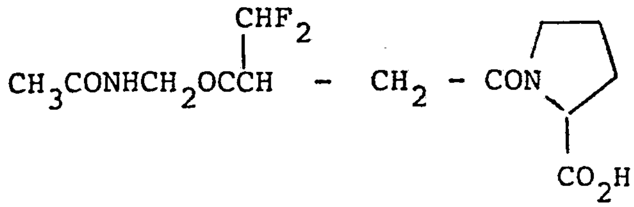

The required compound is obtained by treating the tert-butyl ester of Example 14 with trifluoroacetic acid in substantially the manner described in Example 26 hereinafter.

## EXAMPLE 16

**2-Difluoromethylbutanedioic Acid, 4-Monomethyl Ester**

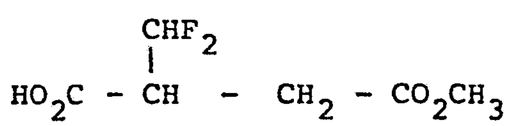

2-Difluoromethyl-2-(tert-butoxycarbonyl) butanedioic acid, 1-(tert-butyl)4-methyl ester (6.5 mM, 2.210 g) prepared in Example 2 is dissolved in trifluoroacetic acid (10 ml) at room temperature. After stirring for 1.5 hours at room temperature, the solvent is evaporated in vacuo yielding a white solid.

The crude disubstituted malonic acid is taken up in glacial acetic acid (20 ml) and the mixture is heated at 100°C for 1.5 hours. The solvent is evaporated _in vacuo_ yielding the desired product as a colorless oil which is purified by distillation.

Yield: 1.600 g (about 90%)

b.p. : 95°C / 0.5 mm Hg (Kugelrohr)

EXAMPLE 17

3-Difluoromethyl-4-Chloro-4-oxo-butanoic acid, methyl ester

$$ClOC - \overset{\displaystyle CHF_2 \atop |}{CH} - CH_2 - CO_2CH_3$$

2-Difluoromethylbutanedioic acid, 4-monomethyl ester (182 mg, 1mM) prepared in the preceding Example is dissolved in thionyl chloride (5 ml) and the mixture is heated at reflux for 2 hours. The solvent is evaporated _in vacuo_ yielding a yellowish oil.

NMR ($CDCl_3$) $\delta$ ppm 6.15 (doublet of triplet); $J_{HF}$ = 55 Hz, $J_{HH}$ = 4 Hz $-CHF_2$.

EXAMPLE 18

4,4-Difluoro-3-benzoylbutanoic acid, methyl ester

$$C_6H_5.OC - \overset{\displaystyle CHF_2 \atop |}{CH} - CH_2 - CO_2CH_3$$

A solution of lithium diphenylcuprate (0.5 mM) in diethylether (3 ml) is added, at -78°C, under nitrogen, to a solution of 3-difluoromethyl-4-chloro-4-oxo-butanoic acid, methyl ester (1 mM) prepared in the preceding Example in diethylether (5 ml). After

stirring for 1 hour at -78°C, the mixture is hydrolized with a saturated aqueous ammonium chloride solution and extracted twice with ether. The organic layer is dried over anhydrous magnesium sulfate and concentrated to dryness <u>in vacuo</u>. The 4,4-difluoro-3-benzoylbutanoic acid, methyl ester is isolated by HPLC (ether : cyclohexane mixtures). NMR ($CCl_4$) $\delta$ ppm 6.20 (doublet of triplet $J_{HF}$ = 54Hz, $J_{HH}$ = 3Hz, $-CHF_2$).

## EXAMPLE 19

<u>4,4-Difluoro-3-benzoylbutanoic acid</u>

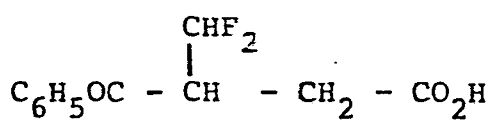

The methylester of Example 18 (10 mM) is dissolved in trimethylsilyliodide (5 mL) and heated at 100°C under nitrogen for 24 hours. Concentration <u>in vacuo</u> leaves a residue which is extracted with ethyl acetate and the organic phase is washed with water and dried with $MgSO_4$ to yield the expected acid.

## EXAMPLE 20

<u>N(4,4-difluoro-3-benzoyl-butyryl)-L-proline, tert-butyl ester</u>

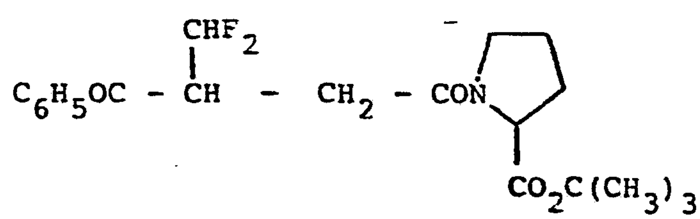

The required compound is prepared by treating

the acid of Example 19 with L-proline, tert-butyl ester in substantially the manner described in Example 4.

EXAMPLE 21

N(4,4-difluoro-3-benzoyl-butyryl)-L-proline

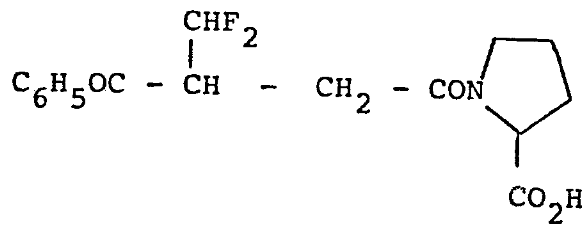

The required compound is prepared by treating the tert-butyl ester of Example 20 with trifluoroacetic acid in substantially the manner described in Example 26 as hereinafter.

EXAMPLE 22

2-Ethoxycarbonyl-3-methyl-butanedioic acid, bis tert-butyl ester

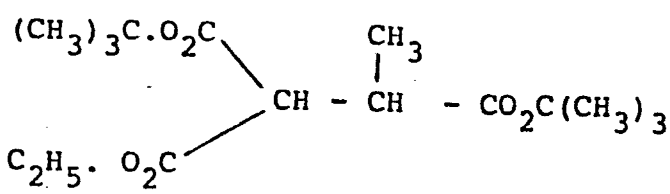

tert-Butyl ethyl malonate (100 mM, 18.800 g) is added, at room temperature, under nitrogen, to a suspension of sodium hydride (105 mM, 4.725 g of a 55% dispersion in oil) in tetrahydrofuran (250 ml); after stirring for 1.5 hours, a solution of α-bromo tert-butylpropionate (100 mM, 20.900 g) in tetrahydrofuran (15 ml) is added dropwise over a period of 15 minutes; stirring is continued for 20 hours. The mixture is then hydrolized and extracted twice with ether. The organic layer is dried over anhydrous magnesium sulfate and concentrated in vacuo. The desired triester is isolated by distillation under reduced pressure.

b.p.: 90-91°C / $4.10^{-2}$mm Hg

Yield: 25.2 g about 80%

EXAMPLE 23

2-Difluoromethyl-2-ethoxycarbonyl-3-methyl-butanedioic acid, bis tert-butyl ester

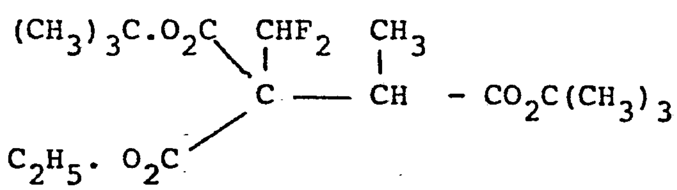

2-Ethoxycarbonyl-3-methylbutanedioic acid, bis tert-butyl ester (26.5 mM, 8.385 g) prepared in the preceding Example is added, at room temperature, under nitrogen, to a suspension of sodium hydride (29 mM, 1.305 g of a 55% dispersion in oil) in tetrahydrofuran (80 ml). After stirring for 0.5 hour, the solution

2.600 g after distillation, yield about 70%

b.p. 158-160°C / 0.1 mm Hg (Kugelrohr).

Product containing approximately 10% of $\alpha\beta$-unsaturated acid (oxidative decarboxylation product).

## EXAMPLE 25

**N-[3-difluoromethyl-3-ethoxycarbonyl-2-methyl-propionyl]-L-proline, *tert*-butyl ester**

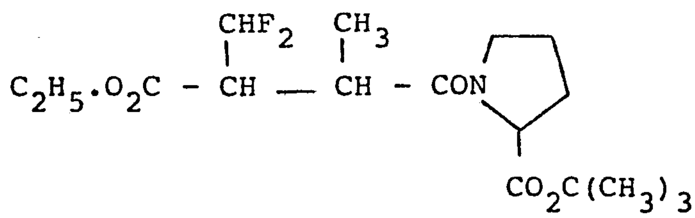

Dicyclohexylcarbodiimide (10 mM, 2.060 g) is added to a solution of L-proline [*tert*-butyl] ester (10 mM, 1.710 g) in methylene chloride (70 ml) at 0°C. 2-Difluoromethyl-3-methylbutandioic acid, 1-monoethyl ester (10 mM, 2.134 g) from the preceding Example is added and the mixture is stirred for 1 hour at 0°C, and 72 hours at room temperature. The precipitate is filtered off, rinsed thoroughly with methylene chloride and the filtrate is concentrated *in vacuo*. The residual oil is triturated with ether and the insoluble material filtered off. The crude mixture is chromatographed on a silica gel column (300 g) with ethyl acetate/cyclohexane (1:4). The fractions containing the desired material are concentrated to dryness to yield a colorless oil.

is rapidly saturated with a stream of chlorodifluoromethane. Stirring is continued for 20 hours, and the mixture is quenched with water, and extracted twice with ether. The organic layer is dried over anhydrous magnesium sulfate, and concentrated to dryness *in vacuo*.

The residual oil is distilled under reduced pressure yielding 6.700 g of the desired product (yield about 70%).

b.p. : 109-111°C /0.1 mm Hg

## EXAMPLE 24

**2-Difluoromethyl-3-methylbutanedioic acid, 1-monoethyl ester**

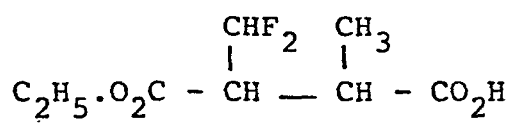

2-Difluoromethyl-2-ethoxycarbonyl-3-methylbutanedioic acid, bis[*tert*-butyl]ester (about 18 mM, 6.588 g) is dissolved in trifluoroacetic acid (15 ml) at room temperature. After stirring for 2 hours at room temperature, the solvent is evaporated *in vacuo* yielding a white solid.

The crude diacid is taken up in glacial acetic acid (40 ml) and the mixture is heated at 110°C for 15 hours. The solvent is evaporated *in vacuo* yielding a yellowish oil, purified by distillation under reduced pressure:

## EXAMPLE 26

N-(3-difluoromethyl-3-ethoxycarbonyl-2-methyl-propionyl)-L-Proline

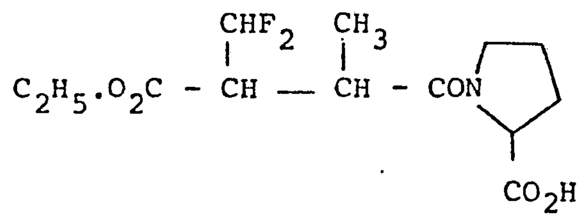

N-(3-difluoromethyl-3-ethoxycarbonyl-2-methyl-propionyl)-L-proline, tert-butyl ester (0.27 mM, 100 mg) prepared in the preceding Example is dissolved in trifluoroacetic acid (5 ml) at room temperature. After stirring for 2 hours at room temperature, the solvent is evaporated *in vacuo* yielding the desired product as a colorless oil. NMR ($CDCl_3$ + $\epsilon$ $CF_3CO_2H$) $\delta$ ppm 6.02 (doublet of triplet) $J_{HF}$ = 56Hz, $J_{HH}$ = 3Hz, $-CHF_2$.

## EXAMPLE 27

A) Preparation of:-
1-tert-butyl-3-benzyl-malonate

$$C_6H_5.CH_2.O_2C - CH_2 - CO_2C(CH_3)_3$$

Malonic acid monobenzylester (23.4 g, 0.121 mole), 50 ml of ether, 1.8 ml of concentrated sulfuric acid and 17 ml of liquid isobutylene are mixed in a pressure bottle. The mixture is shaken overnight at room temperature. The reaction mixture is poured into 60 ml of 6 N sodium hydroxide solution containing 60 g of ice. Extraction with diethylether and usual treatment affords an oil which gives after distillation (118°-120°C, 0.5 mm Hg) 10 g of the expected malonate.

The crude malonate is purified by chromatography on silica gel. NMR ($CDCL_3$) $\delta$ ppm 1.4 (singlet) $OC(CH_3)_3$; 3.15 (singlet) $-CH_2$; 3.66 (singlet) $-OCH_3$; 5.23 (singlet) $OCH_2$; 6.43 (triplet) $J_{HF}$ = 55Hz $CHF_2$; 7.33 (singlet) $C_6H_5$.

B) Preparation of:-

<u>N-(3-Difluoromethyl-3-carboxy-propionyl)-L-proline, 3-benzyl ester</u>

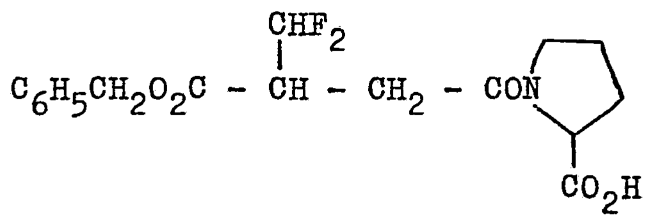

The procedure of Examples 1, 2, 3, 4 and 5 is repeated commencing with the malonate of step A to yield the desired benzyl ester.

## EXAMPLE 28

A) Preparation of:-

<u>1-benzyl-3-dibenzyl malonate</u>

$$(C_6H_5)_2CH.O_2C - CH_2 - COCH_2C_6H_5$$

Malonic acid monodibenzyl ester (100 mM), ethyl acetate (200 mL) and diphenyl diazomethane (150 mM) are heated at reflux temperature of ethyl acetate for 12 hours. The mixture is concentrated *in vacuo* leaving an oil which upon distillation yields 1-benzyl-3-dibenzyl malonate.

B) Preparation of:-

N-(3-Difluoromethyl-3-carboxy-propionyl)-L-proline, 3-benzyl ester

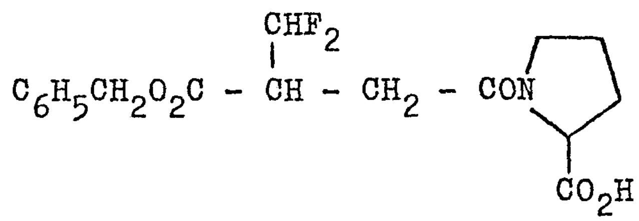

The procedure of Examples 1, 2, 3, 4 and 5 is repeated commencing with the malonate of step A to yield the desired benzyl ester.

EXAMPLE 29

A) Preparation of:-

1-benzyl-3-trityl malonate

$$(C_6H_5)_3C - O_2C - CH_2 - CO_2CH_2C_6H_5$$

Trityl bromide (52 mM) in benzene (100 mL) is added to a suspension of malonic acid monobenzyl ester sodium salt (52 mM) in anhydrous benzene (100 mL) under nitrogen atmosphere. The mixture is heated at reflux with rapid stirring for 5 hours and the hot solution is filtered and the filtrate concentrated to leave the required ester.

B) Preparation of:-

<u>N-(3-Difluoromethyl-3-carboxy-propionyl)-L-proline, 3-benzyl ester</u>

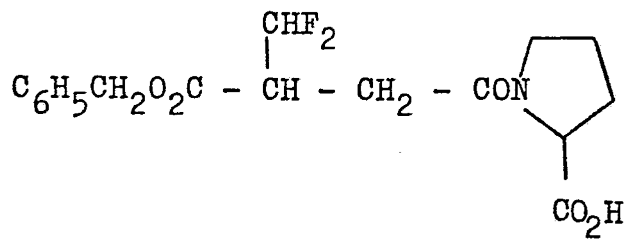

The procedure of Examples 1, 2, 3, 4 and 5 is repeated commencing with the malonate of step A to yield the desired benzyl ester.

EXAMPLE 30

2-Fluoromethylene-butanedioic acid, 4-methylester

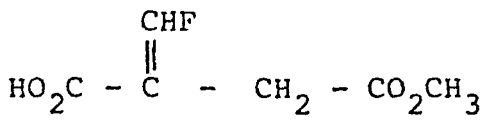

2-Difluoromethyl-2-carboxy-butanedioic acid, 4-methylester (0.28 g, 1.24 mmole) is dissolved in 2 ml of tetrahydrofuran, 1.3 ml of 2 N sodium hydroxide solution and 4 ml of distilled water are added and the mixture stirred during 10 minutes at room temperature. After dilution with water and acidification with 1.5 ml of N hydrochloric acid, the reaction mixture is extracted four times with diethylether. Usual treatment affords 0.19 g of expected 2-fluoromethylene butanedioic acid, 4-methylester. NMR ($CDCl_3$) $\delta$ ppm 3.33 (doublet) J = 3Hz $CH_2$; 7.63 (doublet) $J_{HF}$ = 80Hz C = CHF.

EXAMPLE 31

2-Fluoromethyl-butanedioic acid, 4-methylester

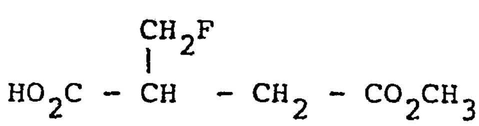

2-Fluoromethylene butanedioic acid, 4-methylester (0.19 g, 1.17 mmoles) prepared in the preceding Example is dissolved in 5 ml of ethyl acetate and 19 mg of 10% rhodium on charcoal is added. The mixture is hydrogenated at room temperature under atmospheric pressure. After 24 hours the hydrogen uptake is

completed. Filtration and removal of the solvent under reduced pressure affords the expected 2-fluoromethyl butanedioic acid, 4-methylester (0.18 g) as an oil.

## EXAMPLE 32

N-(3-methoxycarbonyl-2-difluoromethyl-propionyl)-L-Proline, *tert*-butyl ester

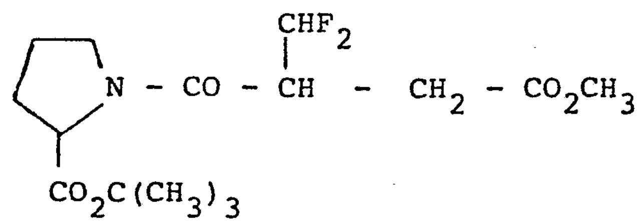

Dicyclohexylcarbodiimide (6.5 mM, 1.340 g) is added to a solution of L-proline *tert*-butyl ester (6.5 mM, 1.112g) in methylene chloride (25 ml) at 0°C. 2-Difluoromethylbutanedioic acid, 4-monomethyl ester (6 mM, 1.100 g) is added and the mixture is stirred for 1 hour at 0°C, and 72 hours at room temperature.

The precipitate is filtered off, rinsed thoroughly with methylene chloride and the filtrate is concentrated *in vacuo*. The residual oil is triturated with ether and the insoluble material filtered off. The crude mixture is chromatographed using the medium pressure silica gel chromatography technique described by Still *et al* with ethyl acetate/petroleum ether (4:6). The fractions containing the desired material are concentrated to dryness to yield the desired product as a colorless oil:

Yield 1.500 g (about 75%) NMR ($CDCl_3$) $\delta$ ppm 5.89 (doublet of triplet) $J_{HF}$ = 54 Hz $J_{HH}$ = 7Hz $CHF_2$.

EXAMPLE 33

N-(3-methoxycarbonyl-2-difluoromethyl-propionyl)-L-Proline

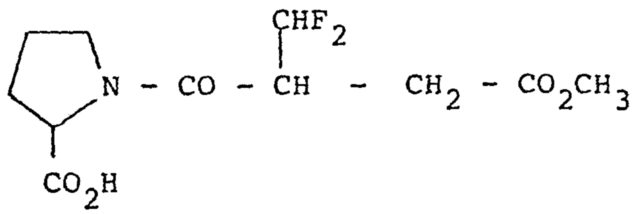

N-(3-methoxycarbonyl-2-difluoromethyl-propionyl)-L-proline tert-butyl ester (0.80 mM, 270 mg) prepared in the preceding Example is dissolved in trifluoroacetic acid (10 ml) at room temperature. After stirring for 2 hours at room temperature, the solvent is evaporated in vacuo, yielding a colorless oil. The acid is crystallized from ether/pentane. M.p.76-78°C.

EXAMPLE 34

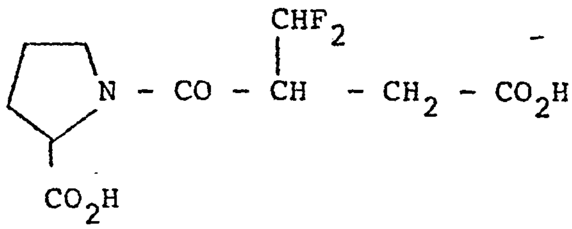

1-(3-Methoxycarbonyl-2-difluoromethyl-1-propionyl-L-proline, tert-butyl ester (0.78 mM, 260 mg) (Example 32) is dissolved in a mixture of 1 N hydrochloric acid and acetic acid (5:2) (7 ml). The mixture is heated at 95°C for 4 hours, and the solvent is then evaporated in vacuo, yielding a yellowish oil. NMR ($CDCl_3$ + ε $CF_3CO_2H$) δ ppm 6.07 (doublet of triplet) $J_{HF}$ = 54 Hz, $J_{HH}$ = 3Hz $CHF_2$.

EXAMPLE 35

3-Trifluoromethyl-3-tris(phenylthio)methylpropionic acid, ethylester

$$(C_6H_5S)_3C-\underset{}{\overset{CF_3}{\overset{|}{CH}}}-CH_2-CO_2C_2H_5$$

n-Butyl lithium (13.2 ml of a 1.4 M solution, 18.5 mMole) is added to trithiophenylorthoformate (6.3 g, 16.5 mMole) in tetrahydrofuran (THF, 100 ml) at -70°C. Ethyl 4,4,4-trifluorocrotonate (3.0 g, 16 mMole) is then added and the solution maintained at -30°C for 1 hour, then water is added and the product isolated by diethylether extraction. The ether solution was dried, concentrated and the residue crystallized from ethanol to afford 4.9 g of the required ester (m.p. 123°C).

EXAMPLE 36

3-Trifluoromethyl-3-tris(phenylthio)methylpropionic acid

$$(C_6H_5S)_3C-\overset{CF_3}{\overset{|}{CH}}-CH_2-CO_2H$$

The ester (1 g) prepared in the preceding Example in ethanol (100 ml) is treated with 25% aqueous sodium hydroxide (50 ml) at 25°C for 1 hour then concentrated under reduced pressure. The residue is acidified and extracted with dichloromethane, then the organic phase dried and concentrated to afford the required acid.

EXAMPLE 37

N-(3-Trifluoromethyl-3-tris(phenylthio)methylpropionyl)-L-proline, *tert*-butyl ester

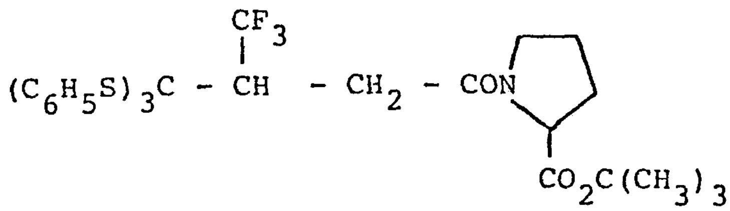

The acid (468 mg, 1 mMole) prepared in the preceding Example in dichloromethane (3 ml) is treated with L-proline *tert*-butyl ester (170 mg, 1 mMole) and dicyclohexyl carbodiimide (210 mg, 1 mMole) overnight at 25°C then filtered. The filtrate is diluted with dichloromethane and washed with 1N HCl and 1N sodium dicarbonate, then dried and concentrated to yield the required *tert*-butyl ester.

EXAMPLE 38

N-(3-Trifluoromethyl-3-tris(phenylthio)methylpropionyl)-L-proline

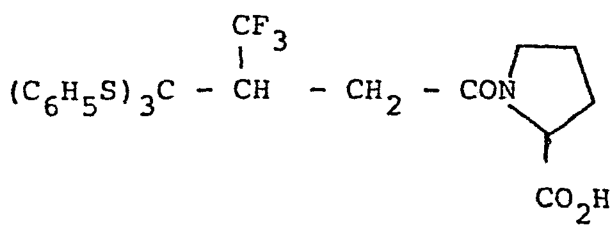

The residue at 0°C of Example 37 is treated with trifluoroacetic acid (2 ml) for 20 mins, the trifluoroacetic acid then being evaporated off to afford the required proline derivative.

EXAMPLE 39

N-(3-Trifluoromethyl-3-carboxy-propionyl)-L-proline

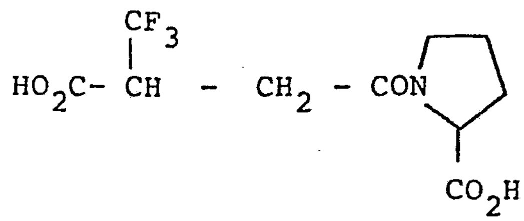

The trithiophenylorthoformate (600 mg) prepared in the preceding Example in acetonitrile (5 ml) is added to N-bromosuccinimide (1.1 g) in acetonitrile (8 ml) and water (2 ml) at 0°C. After 40 minutes at 0°C aqueous bisulfite is then added and the mixture extracted with ether. The ether solution is washed well with water, then dried and evaporated to afford the required diacid.

EXAMPLE 40

N-(3-Trifluoromethyl-3-carboxy-propionyl)-L-proline, tert-butyl ester

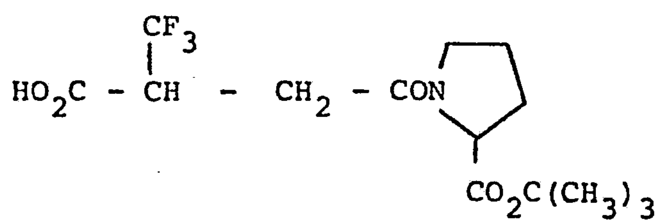

The trithiophenylorthoformate (600 mg) prepared as in Example 37 in acetonitrile (5 ml) is added to N-bromosuccinimide (1.1 g) in acetonitrile (8 ml) and water (2 ml) at 0°C. After 40 minutes at 0°C aqueous bisulfite is then added and the mixture extracted with

ether. The ether solution is washed well with water, then dried and evaporated to afford the required acid.

EXAMPLE 41

N-(3-Trifluoromethyl-3-methoxycarbonyl-propionyl)-L-proline, tert-butyl ester

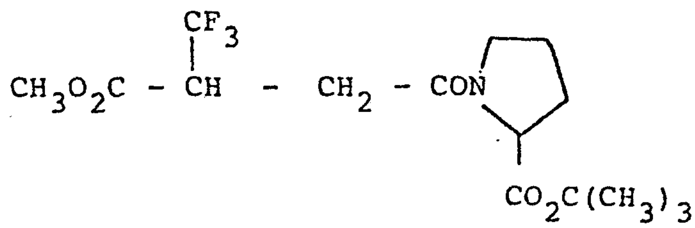

The acid prepared in Example 40 is treated with excess ethereal diazomethane, for 10 minutes then concentrated to afford the required methyl ester.

EXAMPLE 42

N-(3-Trifluoromethyl-3-methoxycarbonyl-propionyl)-L-proline

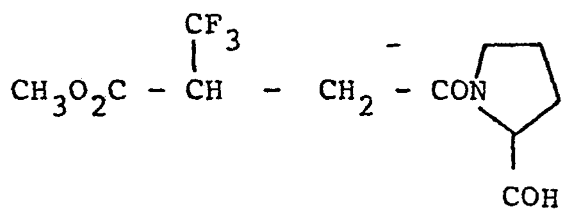

Treatment of the methyl ester prepared in Example 41 with trifluoroacetic acid (2 ml) at 0°C for 20 minutes followed by evaporation affords the required half ester.

EXAMPLE 43

3-Trifluoromethyl-3(2-phenyldithian-2-yl) propionic acid, ethyl ester

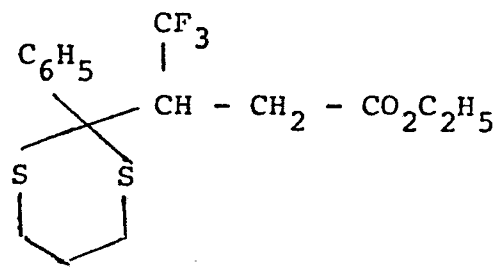

A solution of 2-phenyl dithiane (2.4 g, 12.1 mMole), and hexamethylphosphorous triamide (2.2 ml, 12.1 mMole) in tetrahydrofuran (50 ml) at -70°C is treated with n-butyl lithium (8.6 ml of a 1.4 m solution). Ethyl-4,4,4-trifluorocrotonate (1.86 g, 11 mMole) in tetrahydrofuran (5 ml) is then added and the reaction quenched with water after 1 minute at -70°C. The product is purified by chromatography on silica gel to afford an oil, b.p. 180°C/0.1 mm (Kugelrohr).

EXAMPLE 44

3-Trifluoromethyl-3-(2-phenyldithian-2-yl) propionic acid

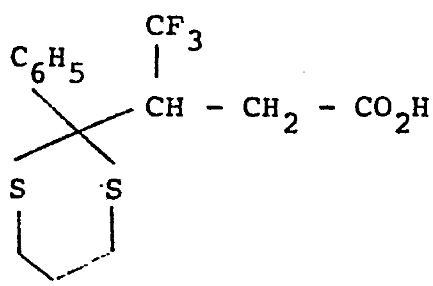

The ester (3.1 g) prepared in Example 43 in

ethanol (20 ml) is treated with 50% aqueous sodium hydroxide (5 ml) for 2 hours at 25°C and then concentrated under reduced pressure. The residue is acidified and then extracted with chloroform. The chloroform solution is dried and concentrated and the residue recrystallized from dichloromethane-heptane to yield the required acid m.p. 156-157°C.

EXAMPLE 45

N(3-Trifluoromethyl-3-(2-phenyldithian-2-yl)-propionyl)-L-proline, tert-butyl ester

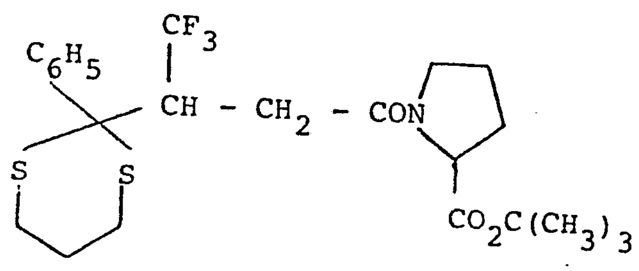

The acid (336 mg, 1 mMole) prepared in Example 44 in dichloromethane (3 ml) is treated with L-proline tert-butyl ester (170 mg, 1 mMole) and dicyclohexylcarbodiimide (210 mg, 1 mMole) overnight at 25°C then filtered. The filtrate is diluted with dichloromethane and washed with 1N HCl and 1N sodium bicarbonate, then dried and concentrated to yield the required ester.

EXAMPLE 46

N-(3-Trifluoromethyl-3-(2-phenyldithian-2-yl)-propionyl)-L-proline

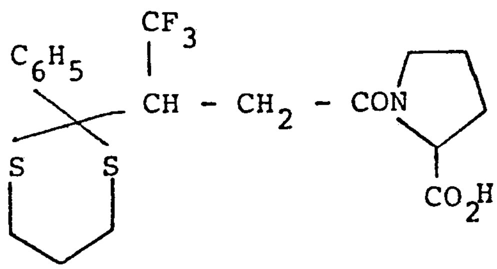

The residue from Example 45 at 0°C is treated with trifluoroacetic acid (2 ml) for 20 minutes, the trifluoroacetic acid then being evaporated off to afford the required proline derivative.

EXAMPLE 47

N-(3-Trifluoromethyl-3-benzoyl-propionyl)-L-proline

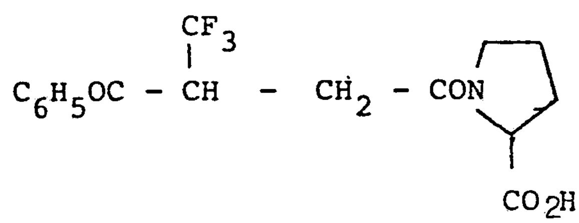

The acid (600 mg) prepared in Example 46 in acetonitrile (5 ml) is added to N-bromosuccinimide (1.1 g) in acetonitrile (8 ml) and water (2 ml) at 0°C. After 40 minutes at 0°C aqueous bisulfite is then added and the mixture extracted with ether. The ether solution was washed well with water, then dried and evaporated to afford the keto acid.

EXAMPLE 48

3-Trifluoromethyl-3-(2-tert-butoxycarbonyl-dithian-2-yl)-propionic acid, ethyl ester

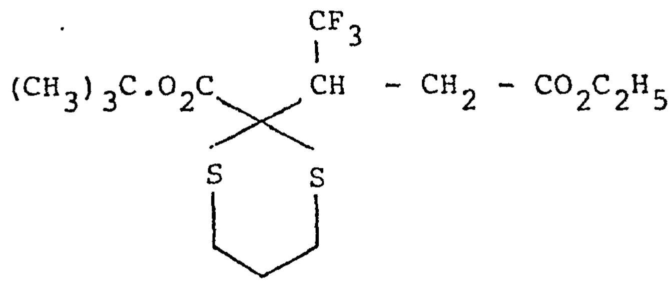

A solution of 2-tert-butoxycarbonyl-dithiane (2.6 g, 12.1 mMole), and hexamethylphosphorus triamide (2.2 ml, 12.1 mMole) in tetrahydrofuran (50 ml) at -70°C is treated with n-butyl lithium (8.6 ml of a 1.4 m solution). Ethyl 4,4,4-trifluorocrotonate (1.86 g, 11 mMole) in tetrahydrofuran (5 ml) is then added and the reaction quenched with water after 30 minutes at -70°C. The product was purified by chromatography on silica gel to afford an oil.

EXAMPLE 49

3-Trifluoromethyl-3-(2-tert-butoxycarbonyl-dithian-2-yl)-propionic acid

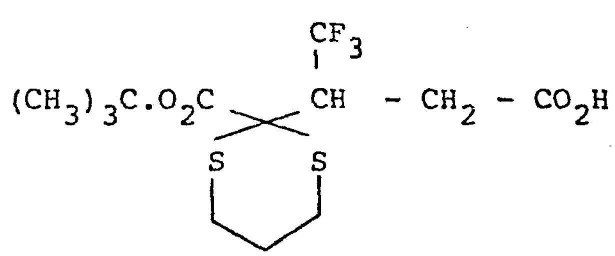

The ester (3.1 g) prepared in Example 48 in

ethanol (20 ml) is treated with 50% aqueous-sodium hydroxide (5 ml) for 2 hours at 25°C and then concentrated under reduced pressure. The residue is acidified and then extracted with chloroform. The chloroform solution is dried and concentrated and the residue recrystallized from dichloromethane-heptane to yield the required ester acid.

EXAMPLE 50

N-(3-Trifluoromethyl-3-(2-*tert*-butoxycarbonyl-dithian-2-yl)-propionyl)-L-proline, *tert*-butyl ester

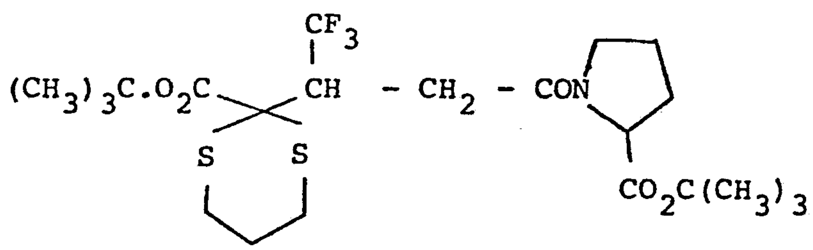

The acid (360 mg, 1 mMole) prepared in Example 49 in dichloromethane (3 ml) is treated with L-proline *tert*-butyl ester (170 mg, 1 mMole) and dicyclohexyl carbodiimide (210 mg, 1 mMole) overnight at 25°C and then filtered. The filtrate is diluted with dichloromethane and washed with 1N HCl and 1N sodium bicarbonate and then dried and concentrated to yield the desired diester.

EXAMPLE 51

N-(3-Trifluoromethyl-3(2-carboxy-dithian-2-yl)-propionyl)-L-proline

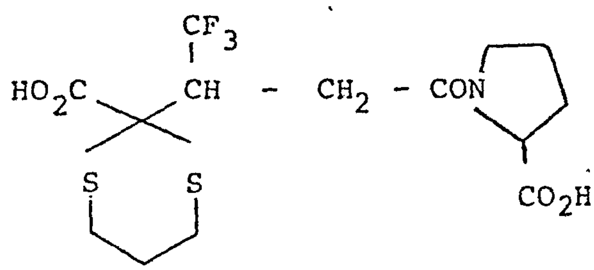

The residue of Example 50 at 0°C was treated with trifluoroacetic acid (2 ml) for 20 minutes, the trifluoroacetic acid then being evaporated off to afford the required proline derivative.

EXAMPLE 52

N-(3-Trifluoromethyl-3-carboxycarbonyl-propionyl)-L-proline

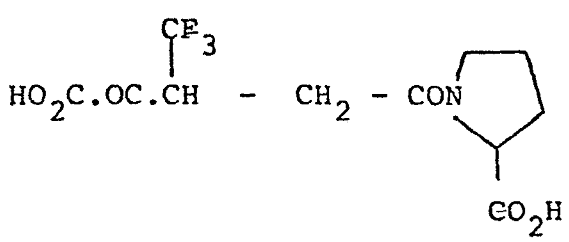

The acid(600 mg) prepared in Example 51 is added to N-bromosuccinimide (1.1 g) in acetonitrile (8 ml) and water (2 ml) at 0°C. After 40 minutes at 0°C, aqueous bisulfite is then added and the mixture extracted with ether. The ether solution is washed well with water and then dried and evaporated to afford the required diacid.

EXAMPLE 53

3-(Trifluoromethyl-4-methoxy-pent-4-enoic acid, ethyl ester

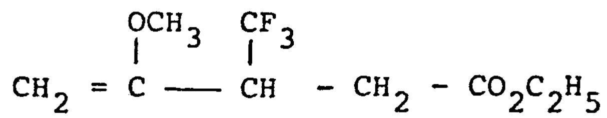

*tert*-butyl lithium (10 mMole, 6.25 ml of a 1.6 m solution in *n*-pentane) is added dropwise to a solution of methyl vinyl ether (92 g, 16 mMole) in tetrahydrofuran (100 ml) at -70°C. The mixture is allowed to warm to -5°C and then added to a suspension of copper iodide (5 mMole) in tetrahydrofuran (20 ml) at -70°C. The mixture is maintained at -30°C for 1 hour and then cooled to -70°C and treated with ethyl-4,4,4-trifluorocrotonate (1.68 g, 10 mMole) in tetrahydrofuran (10 ml). After 1 hour at -70°C, the mixture is treated with water and the product isolated by diethylether extraction.

EXAMPLE 54

3-Trifluoromethyl-4-methoxy-pent-4-enoic acid

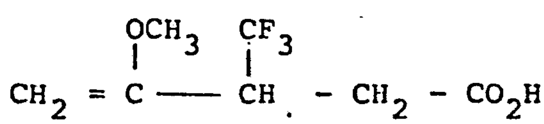

The ester (2 g) prepared in Example 53 in ethanol (20 ml) is treated with 25% aqueous sodium hydroxide (5 ml) for 2 hours at 25°C and then concentrated under reduced pressure. Careful acidification to pH 6.5, followed by extraction with chloroform affords the desired acid (1.5 g).

EXAMPLE 55

N-(3-Trifluoromethyl-4-methoxy-pent-4-enoyl)-L-proline, *tert*-butyl ester

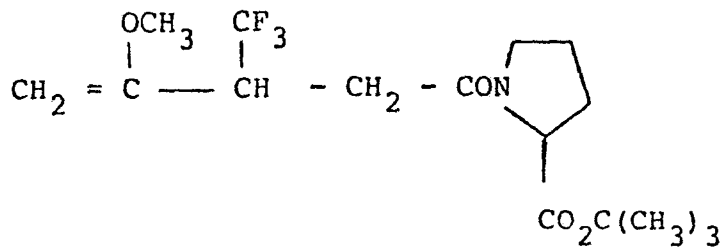

The acid (198 mg, 1 mMole) prepared in Example 54 in dichloromethane (3 ml) is treated with L-proline *tert*-butyl ester (170 mg, 1 mMole) and dicyclohexyl carbodiimide (210 mg, 1 mMole) overnight at 25°C and then filtered. The filtrate is diluted with dichloromethane and washed with 1N HCl and 1N sodium bicarbonate and then dried and concentrated to yield the required ester.

EXAMPLE 56

N-(3-Trifluoromethyl-3-acetyl-propionyl)-L-proline

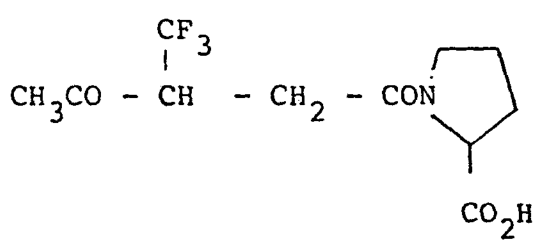

The residue of Example 55 is treated with trifluoroacetic acid (2 ml) for 20 minutes, followed by water (2 ml) for a further 20 minutes. The aqueous trifluoroacetic acid is then evaporated to afford the required proline derivative.

EXAMPLE 57

3-Trifluoromethyl-3-bis(phenylthio)methyl propionic acid, ethyl ester

$$(C_6H_5S)_2CH - \overset{\displaystyle CF_3}{\overset{|}{CH}} - CH_2 - CO_2C_2H_5$$

Methyllithium (0.33 ml of a 1.6 M solution, 0.53 mMole is added to n-butylmercaptan (100 mg, 1.0 mMole) in hexamethylphosphorous triamide (HMPA) (2 ml). 3-Trifluoromethyl-3-tris(phenylthio)methyl propionic acid, ethyl ester prepared in Example 35 (250 mg, 0.5 mMole) in HMPA (1 ml) is then added and the solution stirred at 25°C for 15 minutes and then aqueous ammonium chloride is added and the mixture extracted with ether. The ether solution is washed well with water, dried and evaporated to afford the crude thioacetal which can be purified by chromatography on silica gel.

EXAMPLE 58

3-Trifluoromethyl-3-bis(phenylthio)methyl propionic acid

$$(C_6H_5S)_2CH - \overset{\displaystyle CF_3}{\overset{|}{CH}} - CH_2 - CO_2H$$

The ester (100 mg) prepared in Example 57 in ethanol (5 ml) containing aqueous 25% sodium hydroxide (2 ml) is stirred at 25°C for 2 hours then acidified and the required acid isolated by diethyl ether extraction.

## EXAMPLE 59

**N-(3-Trifluoromethyl-3-bis(phenylthio)methyl-propionyl)-L-proline, *tert*-butyl ester**

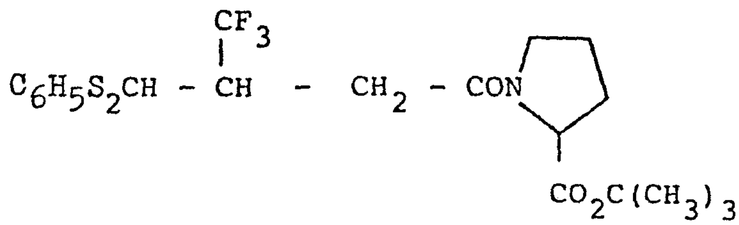

The acid (360 mg, 1 mMole) prepared in Example 58 in dichloromethane (3 ml) is treated with L-proline *tert*-butyl ester (170 mg, 1 mMole) and dicyclohexylcarbodiimide (210 mg, 1 mMole) overnight at 25°C and then filtered. The filtrate is diluted with dichloromethane and washed with 1N HCl and 1N sodium bicarbonate and then dried and concentrated.

## EXAMPLE 60

**N-Trifluoromethyl-3-bis(phenylthio)methyl-propionyl)-L-proline**

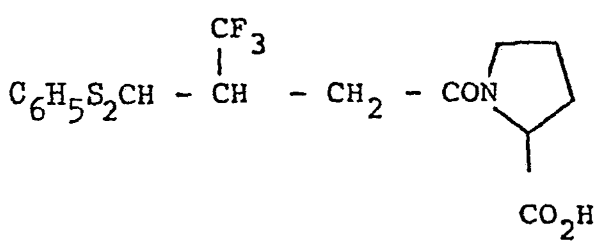

The residue of Example 59 at 0°C is treated with trifluoroacetic acid (2 ml) for 20 minutes, the trifluoroacetic acid then being evaporated off to afford the required proline derivative.

EXAMPLE 61

N-(3-Trifluoromethyl-3-formyl-propionyl)-L-proline

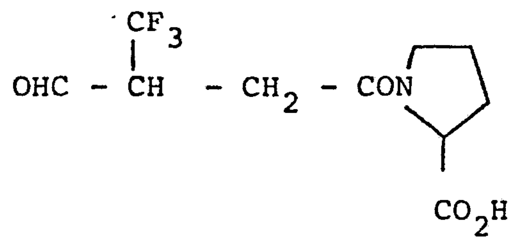

The thioacetal (600 mg) in acetonitrile (5 ml) is added to N-bromosuccinimide (1.1 g) in acetonitrile (8 ml) and water (2 ml) at 0°C. After 40 minutes at 0°C aqueous bisulfite is then added and the mixture extracted with ether. The ether solution is washed well with water, then dried and evaporated to afford the aldehyde acid.

EXAMPLE 62

A) Preparation of:-

N-(3-Trifluoromethyl-3-bis(phenylthio)methyl-propionyl)-L-proline, *tert*-butyl ester

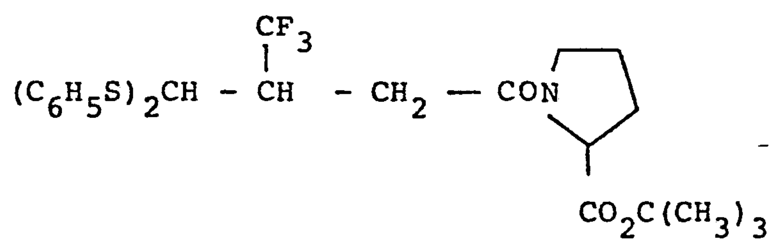

A solution of chromous chloride (600 mg, 7 mMole) and N-(3-trifluoromethyl-3-tris(phenylthio)methyl-propionyl)-L-proline, *tert*-butyl ester prepared in Example 37 (1 mMole) in dimethylformamide (12 ml) and water (1.2 ml) is heated for 3 hours. On cooling the mixture is poured into water and extracted with diethyl

ether. The ether solution is washed well with water and then dried and concentrated to afford the required thioacetal derivative.

B) Preparation of:-

N-(3-Trifluoromethyl-3-formyl-propionyl)-L-proline

The procedure of Example 39 is repeated commencing with the ester of step A to yield the desired compound.

## EXAMPLE 63

Tert-butyl-4,4,4-trifluorocrotonate

$$\begin{array}{l} CF_3 \\ | \\ CH = CH - CO_2C(CH_3)_3 \end{array}$$

A solution of equimole amounts of trifluorocrotonic acid and N,N'-di(isopropyl)-*tert*-butoxy amidine in methylene chloride is stirred overnight at 25°C and then filtered. The filtrate is fractionally distilled to afford the required *tert*-butyl ester.

## EXAMPLE 64

3-Trifluoromethyl-3-cyano-propionic acid, *tert*-butyl ester

$$\begin{array}{l} \quad\;\; CF_3 \\ \quad\;\; | \\ NC - CH - CH_2 - CO_2C(CH_3)_3 \end{array}$$

To a stirred solution of the ester (1.9 g, 10 mMole) prepared in Example 63 in dimethoxyethane (10 ml) at 0°C is added potassium cyanide (1.2 g, 20 mMole) in water (5 ml). After 3 hours at 0°C, the mixture is stirred at room temperature for 48 hours, then diluted with aqueous sodium chloride and extracted with ether. The ether solution is dried and concentrated to afford the required nitrile.

EXAMPLE 65

3-Trifluoromethyl-3-cyano-propionic acid

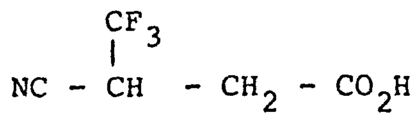

The tert-butyl ester (0.5 g) prepared in Example 64 at 0°C is treated with trifluoroacetic acid (2 ml) for 20 minutes and then the trifluoroacetic acid evaporated off under reduced pressure to yield the free acid.

EXAMPLE 66

N-(3-Trifluoromethyl-3-cyano-propionyl)-L-proline tert-butyl ester

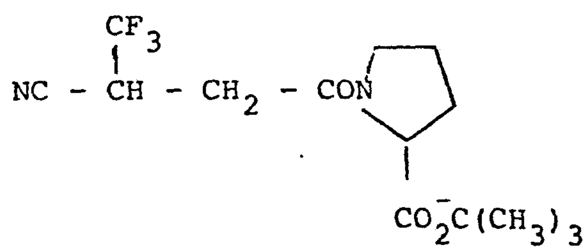

The acid (167 mg, 1 mMole) prepared in Example 65 in dichloromethane (3 ml) is treated with L-proline tert-butyl ester (170 mg, 1 mMole) and dicyclohexylcarbodiimide (210 mg, 1 mMole) overnight at 25°C and then filtered. The filtrate is diluted with dichloromethane and washed with 1N HCl and 1N sodium bicarbonate and then dried and concentrated to yield the required ester.

EXAMPLE 67

N-(3-Trifluoromethyl-3-cyano-propionyl)-L-proline

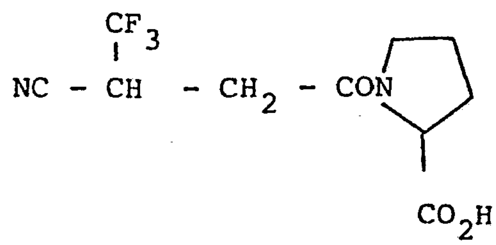

The residue from Example 66 at 0°C is treated with trifluoroacetic acid (2 ml) for 20 minutes, the trifluoroacetic acid then being evaporated off to afford the required proline derivative.

EXAMPLE 68

(a) 2-Carbamoyl-dithiane

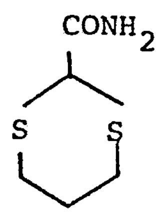

2-Methoxycarbonyl-dithiane (19.7 g) in ethanol (100 ml) at 0°C is saturated with anhydrous ammonia, allowed to stand for 1 week at 25°C and the precipitate (15.9 g) collected to yield the required amide.

(b) 2-Cyano-dithiane

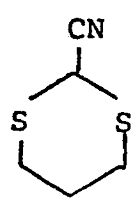

The amide (13.1 g, 80.4 mMole) prepared in Example 68a in dichloroethane (100 ml) containing sodium

chloride (15 g) and phosphorous oxychloride (12.2 g, 80 mMole) is heated at reflux overnight. On cooling, the mixture is filtered and the filtrate concentrated. The residue is dissolved in ether, washed with sodium bicarbonate solution then dried and concentrated. The residue is crystallized from methanol.

EXAMPLE 69

3-Trifluoromethyl-3(2-cyanodithian-2-yl)-propionic acid, ethyl ester

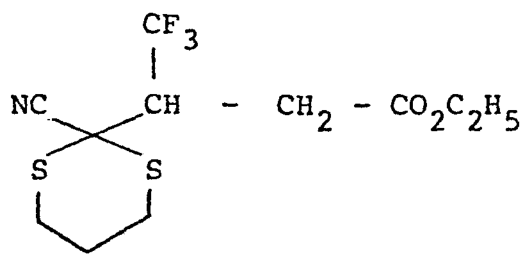

The nitrile (290 mg, 2 mMole) prepared in Example 68 in tetrahydrofuran (2 ml) is added to a solution of lithium diisopropylamide (prepared from diisopropylamine 2 mMole and n-Butyl lithium, 2 mMole) in tetrahydrofuran (10 ml) at -78°C. Ethyl-4,4,4-trifluorocrotonate (336 mg, 2 mMole) in tetrahydrofuran (2 ml) is then added and the solution maintained at -78°C for 30 minutes then quenched with water, and the products isolated by ether extraction. The product is then purified by chromatography on silica gel, being eluted with 20% diethyl ether-pentane.

EXAMPLE 70

3-Trifluoromethyl-3-(2-cyanodithian-2-yl)propionic acid

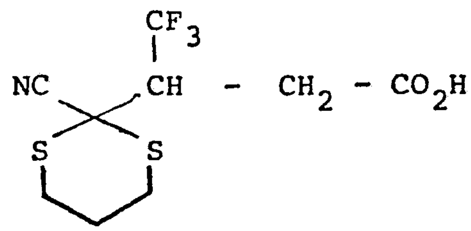

The ester (1 g) prepared in Example 69 in methanol (20 ml) containing 25% aqueous sodium hydroxide (5 ml) is stirred at 25°C for 1 hour and then concentrated at reduced pressure. The residue is acidified and extracted with ether. The ether solution is dried and concentrated to afford the acid.

EXAMPLE 71

N-(3-Trifluoromethyl-3-(2-cyanodithian-2-yl)propionyl)-L-proline, *tert*-butyl ester

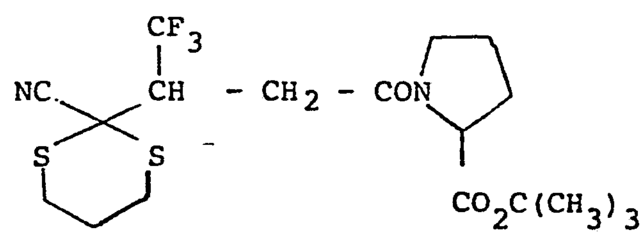

The acid (285 mg, 1 mMole) prepared in Example 70 in dichloromethane (3 ml) is treated with L-proline *tert*-butyl ester (170 mg, 1 mMole) and dicyclohexyl carbodiimide (210 mg, 1 mMole) overnight at 25°C and then filtered. The filtrate is diluted with dichloromethane and washed with 1N HCl and 1N sodium bicarbonate and then dried and concentrated to yield the required ester.

EXAMPLE 72

N-(3-Trifluoromethyl-3-(2-cyanodithian-2-yl)propionyl)-L-proline

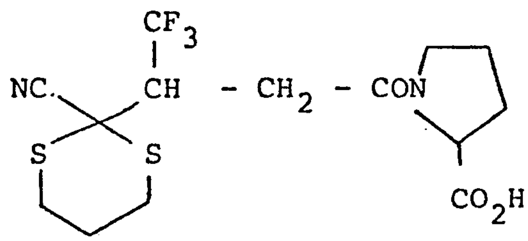

The residue of Example 71 is treated with trifluoroacetic acid (2 ml) for 20 minutes, the trifluoroacetic acid then being evaporated off to afford the required proline derivative.

EXAMPLE 73

N-(3-Trifluoromethyl-3-carboxy-propionyl)-L-proline, *tert*-butyl ester

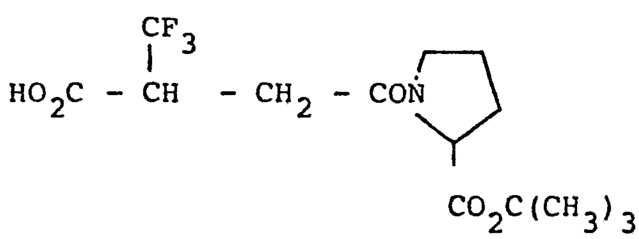

The nitrile (400 mg) prepared in Example 71 is added to N-bromosuccinimide (1.1 g) in acetonitrile (8 ml) and water (2 ml) at 0°C. After 40 minutes at 0°C, aqueous bisulfite is added and the mixture extracted with ether. The ether solution is washed well with water, then dried and evaporated to afford the required acid.

EXAMPLE 74

N-(3-Trifluoromethyl-3-methoxycarbonyl)-propionyl)-L-proline, *tert*-butyl ester

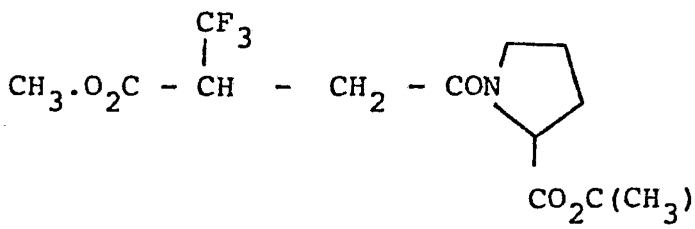

The acid of Example 73 is treated with excess ethereal diazomethane followed by evaporation to afford the required methyl ester.

EXAMPLE 75

N-(3-Trifluoromethyl-3-methoxycarbonyl-propionyl)-L-proline

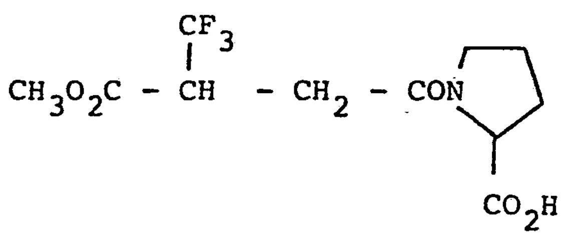

The methyl ester of Example 74 at 0°C is treated with trifluoroacetic acid (2 ml) for 20 minutes and then the trifluoroacetic acid evaporated off under reduced pressure to yield the desired half ester.

EXAMPLE 76

5-Trimethylsilyl-pent-2-ene-4-ynoic acid, methyl ester

$$(CH_3)_3Si - C \equiv C - CH = CH - CO_2CH_3$$

Ethylmagnesium bromide (0.6 mole, 400 ml of a 1.5 M solution in tetrahydrofuran) is added to pent-2-ene-4-ynyl alcohol (24.6 g, 0.3 mole) in tetrahydrofuran (1 l) at 0°C. After 1 hour at 25°C the mixture is recooled to 0°C and chlorotrimethylsilane added (65.1 g, 0.6 mole) in tetrahydrofuran (50 ml). The mixture is stirred for a further hour at 25°C then HCl (1N) added until the pH is acidic. The mixture is extracted with ether, the ether solution washed with aqueous sodium chloride, then dried and evaporated. The residue can be distilled to afford the pure alcohol b.p. 65-70°C, 0.1 mm, but is usually used in the next step without purification. Acetone (100 ml) is added followed by Jones reagent in excess, and the mixture stirred overnight at 25°C and then concentrated under reduced pressure. The residue is diluted with water and extracted with ether. The ether solution is washed well with water then dried and concentrated. The residue is then treated with excess diazomethane in ether, then concentrated. The residue is distilled b.p. 80-95°C/0.1 mm to afford the required ester as an oil (21.5 g).

Jones reagent is chromic anhydride in dilute sulfuric acid.

EXAMPLE 77

5-Trimethylsilyl-3-tris(phenylthio)methyl-pent-4-ynoic acid, methyl ester

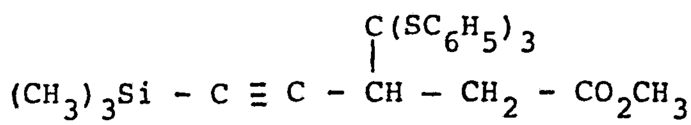

*n*-Butyl lithium (25 mMole, 16.1 ml of a 1.55 M solution) is added to trithiophenylorthoformate (8.5 g, 25 mMole) in tetrahydrofuran (200 ml) at -70°C. After 10 minutes the ester (4.55 g, 25 mMole) prepared in Example 76 in tetrahydrofuran (20 ml) is added and the solution placed at -30°C for 1 hour and then quenched with water. The product is isolated by ether extraction and chromatographed on silica gel. Elution with 20% ether-pentane affords the required ester as a crystalline product (5.5 g).

EXAMPLE 78

3-Tris(phenylthio)methyl-pent-4-ynoic acid

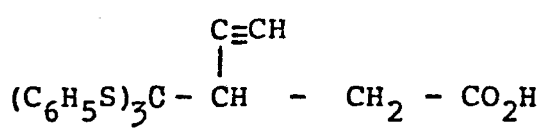

The ester (0.5 g) prepared in Example 77 in ethanol (5 ml) and 25% NaOH (5 ml) is stirred at room temperature for 1 hour then concentrated and acidified, then extracted with chloroform solution. The chloroform solution is dried and evaporated to afford the required acid which is recrystallized from dichloromethane-heptone.

EXAMPLE 79

N-(3-Tris(phenylthio)methyl-pent-4-ynoyl)-L-proline, tert butyl ester

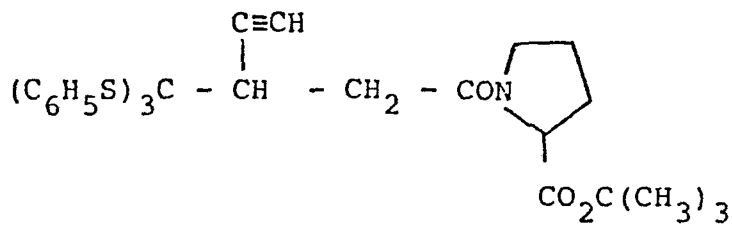

The acid (424 mg, 1 mMole) prepared in Example 78 in dichloromethane (3 ml) is treated with L-proline tert butyl ester (170 mg, 1 mMole) and dicyclohexyl carbodiimide (210 mg, 1 mMole) overnight at 25°C then filtered. The filtrate is diluted with dichloromethane and washed with 1N HCl and 1N sodium bicarbonate, then dried and concentrated to yield the required ester.

EXAMPLE 80

N-(3-Tris(phenylthio)methyl-pent-4-ynoyl)-L-proline

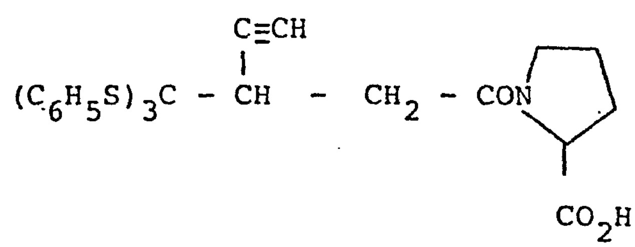

The residue of Example 79 at 0°C is treated with trifluoroacetic acid (2 ml) for 20 minutes, the trifluoroacetic acid then being evaporated off to afford the required proline derivative.

EXAMPLE 81

N-(3-carboxy-pent-4-ynoyl)-L-proline

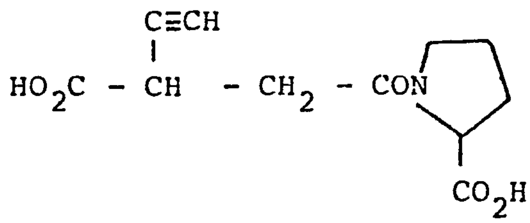

The acid (600 mg) prepared in Example 81 in acetonitrile (5 ml) is added to N-bromosuccinimide (1.1 g) in acetonitrile (8 ml) and water (2 ml) at 0°C. After 40 minutes at 0°C aqueous bisulfite is then added and the mixture extracted with ether. The ether solution is washed well with water, then dried and evaporated to afford the required diacid.

EXAMPLE 82

N-(3-carboxy-pent-4-ynoyl)-L-proline, *tert* butyl ester

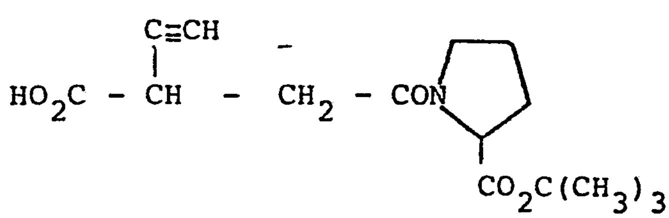

The ester (600 mg) prepared in Example 79 in acetonitrile (5 ml) is added to N-bromosuccinimide (1.1 g) in acetonitrile (8 ml) and water (2 ml) at 0°C. After 40 minutes at 0°C, aqueous bisulfite is then added and the mixture extracted with ether. The ether solution is washed well with water, then dried and evaporated to afford the required acid.

EXAMPLE 83

N-(3-Methoxycarbonyl-pent-4-ynoyl)-L-proline, tert butyl ester

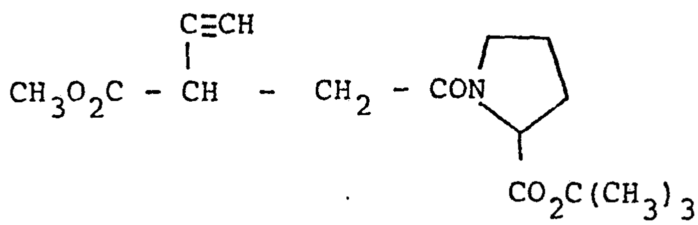

The acid prepared in Example 82 is treated with excess etheral diazomethane followed by evaporation to afford the required methyl ester.

EXAMPLE 84

N-(3-Methoxycarbonyl-pent-4-ynoyl)-L-proline

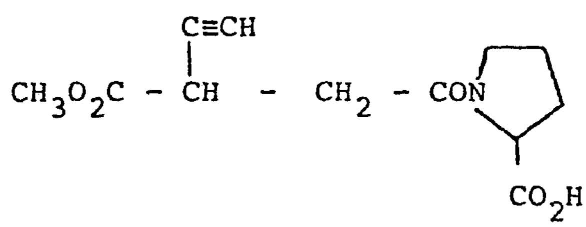

The ester prepared in Example 83 is treated at 0°C with trifluoroacetic acid (2 ml) for 20 minutes. The trifluoroacetic is then evaporated off under reduced pressure to afford the required half ester.

EXAMPLE 85

5-Trimethylsilyl-3-cyano-pent-4-ynoic acid, methyl ester

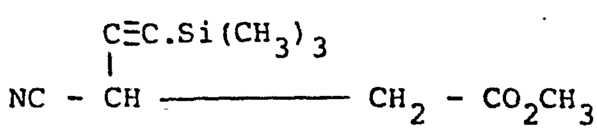

To a stirred solution of the ester (1.0 g, 10 mMole) prepared in Example 76 and acetic acid (60 g,

10 mMole) in ethanol (10 ml) at 0°C is added KCN (1.2 g, 20 mMole) in water (5 ml). After 3 hours at 0°C the mixture is stirred at room temperature for 48 hours, then diluted with aqueous sodium chloride and extracted with ether. The ether solution is dried and concentrated to afford the nitrile ester.

EXAMPLE 86

3-Cyano-pent-4-ynoic acid

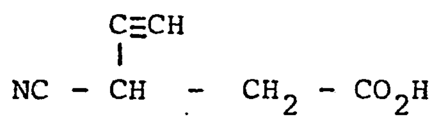

The ester (0.5 g) prepared in Example 85 in ethanol (5 ml) and 25% NaOH (5 ml) is stirred at reflux temperature for 1 hour and then concentrated and extracted with chloroform solution. The chloroform solution is dried and evaporated to afford the required acid which is recrystallized from dichloromethane-heptane.

EXAMPLE 87

N-(3-Cyano-pent-4-ynoyl)-L-proline, tert butyl ester

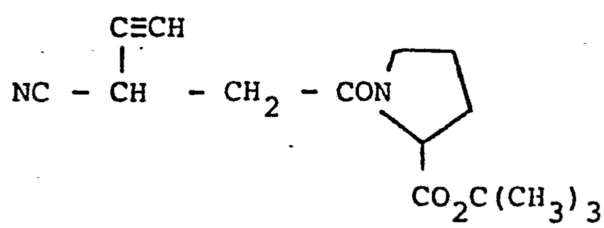

The acid (123 ml, 1 mMole) prepared in Example 86 in dichloromethane (3 ml) is treated with L-proline tert butyl ester (170 mg, 1 mMole) and dicyclohexyl

carbodiimide (210 mg, 1 mMole) overnight at 25°C and then filtered. The filtrate is diluted with dichloromethane and washed with 1N HCl and 1N sodium bicarbonate, then dried and concentrated to yield the required ester.

EXAMPLE 88

N-(3-Cyano-pent-4-ynoyl)-L-proline

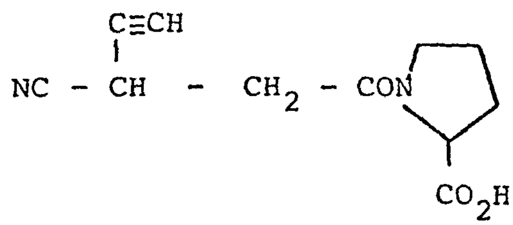

The residue of Example 87 at 0°C is treated with trifluoroacetic acid (2 ml) for 20 minutes, the trifluoroacetic acid then being evaporated off to afford the required proline derivative.

EXAMPLE 89

3-(2-Cyano-dithian-2-yl)-pent-4-ynoic acid, methyl ester

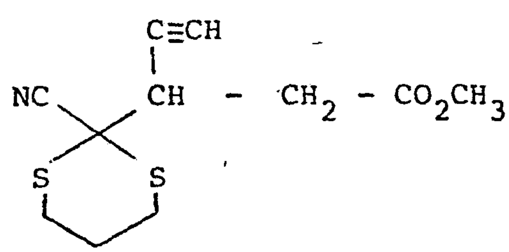

2-Cyano-dithiane (290 mg, 2 mMole) in tetrahydrofuran (2 ml) is added to a solution of lithium diisopropyl amide (prepared from diisopropyl amine,

2 mMole, and n-Butyl lithium, 2 mMole) in tetrahydrofuran (10 ml) at -78°C. The ester (264 mg, 2 mMole) prepared in Example 76 in tetrahydrofuran (2 ml) is then added, the solution maintained at -78°C for 30 minutes and then quenched with water. The product is isolated by ether extraction and then purified by chromatography on silica gel, being eluted with 20% diethylether-pentane.

## EXAMPLE 90

3-(2-Cyanodithian-2-yl)-pent-4-ynoic acid

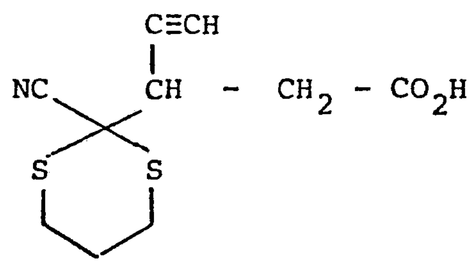

The ester (1 g) prepared in Example 89 in methanol (20 ml) containing 5% aqueous sodium hydroxide (5 ml) is stirred at 25°C for 1 hour and then concentrated at reduced pressure. The residue is acidified and extracted with diethylether. The ether solution is dried and concentrated to afford the required acid.

EXAMPLE 91

N-(3-(2-Cyanodithian-2-yl)pent-4-ynoyl)-L-proline, tert-butyl ester

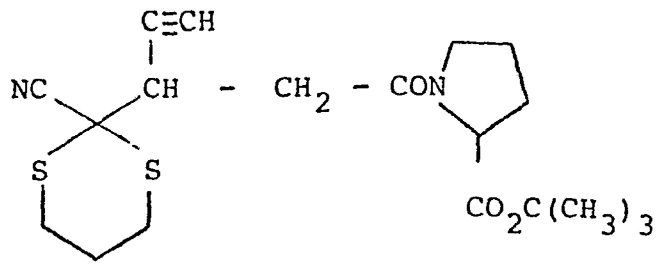

The acid (241 mg, 1 mMole) prepared in Example 90 in dichloromethane (3 ml) is treated with L-proline tert butyl ester (170 mg, 1 mMole) and dicyclohexyl carbodiimide (210 mg, 1 mMole) overnight at 25°C and then filtered. The filtrate is diluted with dichloromethane and washed with 1N HCl and 1N sodium bicarbonate, then dried and concentrated to yield the desired ester.

EXAMPLE 92

N-(3-(2-Cyanodithian-2-yl)pent-4-ynoyl)-L-proline

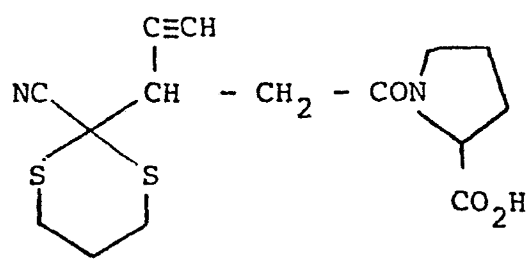

The residue of Example 91 is treated with trifluoroacetic acid (2 mml) for 20 minutes, the trifluoroacetic acid then being evaporated off to afford the required proline derivative.

EXAMPLE 93

N-(3-Carboxy-pent-4-ynoyl)-L-proline, tert butyl ester

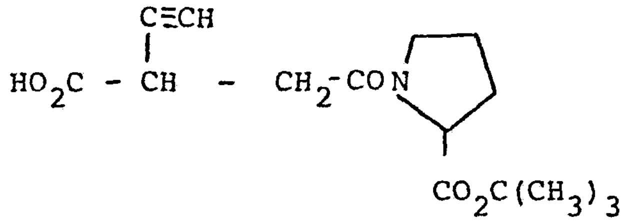

The dithiane nitrile (400 mg) prepared in Example 91 in acetonitrile (5 ml) is added to N-bromosuccinimide (1.1 g) in acetonitrile (8 ml) and water (2 ml) at 0°C. After 4 minutes at 0°C, aqueous bisulfite is added and the mixture extracted with ether. The ether solution is washed well with water, then dried and evaporated to afford the required acid.

EXAMPLE 94

N-(3-Methoxycarbonyl-pent-4-ynoyl)-L-proline, tert butyl ester

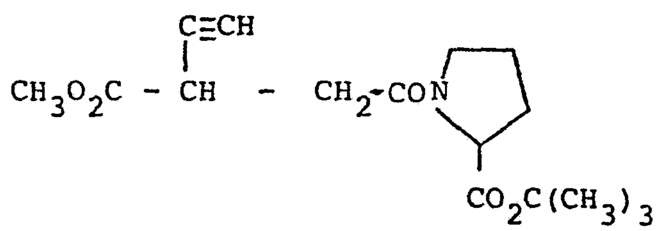

The acid prepared in Example 93 is treated with excess etheral diazomethane followed by evaporation to afford the required methyl ester.

EXAMPLE 95

N-(3-Methoxycarbonyl-pent-4-ynoyl)-L-proline

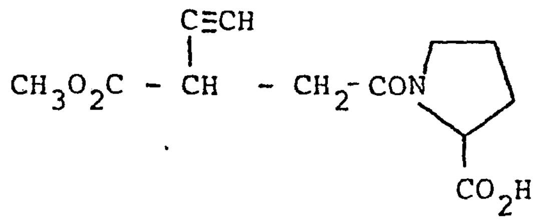

The ester of Example 94 at 0°C, is treated with trifluoroacetic acid (2 ml) for 20 minutes. The trifluoroacetic acid is evaporated off under reduced pressure to afford the required half ester.

EXAMPLE 96

A) Preparation of:-

3-Difluoromethyl-3-ethoxy carbonyl-4-oxo butanoic acid ethyl ester

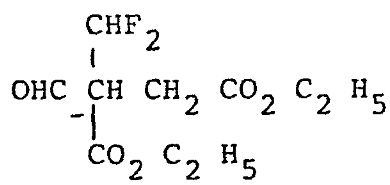

2-difluoromethyl-2-ethoxy carbonyl butanedioic acid-1-acid chloride-4-ethyl ester (0.57 g, 2 mmoles) prepared in Example 9 dissolved in 2 ml of dry tetrahydrofuran is added to a well stirred suspension of hydrogen-equilibrated palladium 10% on carbon (20 mg) in 6 ml of dry tetrahydrofuran containing 2,6-dimethylpyridine (0.215 g, 2 mmoles). The mixture is stirred for two hours and then filtered. The filtrate is condensed and then chromatographed on silica gel to

yield 3-difluoromethyl-3-ethoxy-carbonyl-4-oxo butanoic acid ethyl ester.

B) Preparation of:-

N-3-(Trifluoromethyl-3-formyl-propionyl)-L-proline

The procedure of Examples 12, 4 and 5 is repeated commencing with the diester of step A to yield the desired compound.

EXAMPLE 97

5-Amino-3-difluoromethyl-4-oxo-hexanoic acid

$$\begin{array}{l} \quad\quad\;\; CH_3 \quad\; CHF_2 \\ \quad\quad\;\; | \quad\quad\;\;\; | \\ NH_2\text{-}CH\text{-}CO\text{-}CH\ CH_2\ COOH \end{array}$$

Isocyano methyl propionate (10.22 g, 22 mmoles) and 1,5-diazabicyclo [5.4.0] undec-5-ene (0.6 ml) is added to 2-difluoromethyl-2-ethoxy carbonyl butanedioic acid-1-acid chloride-4-ethyl ester (0.57, 2 mmoles) prepared in Example 9 dissolved in 10 ml of dry tetrahydrofuran. The mixture is stirred 48 hours at room temperature. The reaction mixture is poured into 50 ml of normal hydrochloric acid. Extraction with ether and usual treatment affords the expected isonitrile which is hydrolyzed by reflux in 6 N hydrochloric acid and methanol to give 5-amino-3-difluoromethyl-4-oxo hexanoic acid.

EXAMPLE 98

N-(5-Acetamido-3-difluoromethyl-4-oxo-hexanoyl)-L-proline

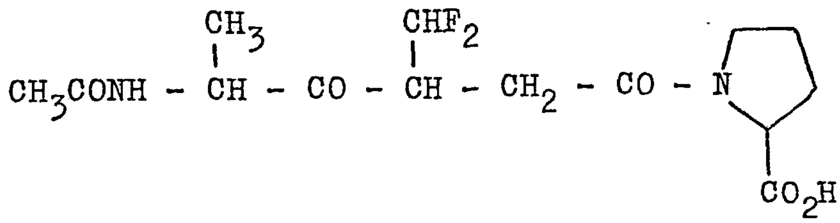

The procedure of Examples 13, 14 and 15 is repeated to yield the desired compound from the acid of Example 97.

In the following Examples relating to pharmaceutical compositions, the term "active compound" is used to indicate the compound N-(3-difluoromethyl-3-carboxy-propionyl)-L-proline. This compound may be replaced in these compositions by any other inhibitor of the invention, for example by N-(3-carboxy-pent-4-ynoyl)-L-proline. Adjustments in the amount of medicament may be necessary or desirable depending upon the degree of activity of the medicament as is well known in the art.

EXAMPLE 99

An illustrative composition for hard gelatin capsules is as follows:-

| | | |
|---|---|---|
| (a) | active compound | 20 mg |
| (b) | talc | 5 mg |
| (c) | lactose | 90 mg |

The formulation is prepared by passing the dry powders of (a) and (b) through a fine mesh screen and mixing them well. The powder is then filled into hard gelatin capsules at a net fill of 115 mg per capsule.

EXAMPLE 100

An illustrative composition for tablets is as follows:-

| | | |
|---|---|---|
| (a) | active compound | 20 mg |
| (b) | starch | 43 mg |
| (c) | lactose | 45 mg |
| (d) | magnesium stearate | 2 mg |

The granulation obtained upon mixing the lactose with the compound (a) and part of the starch and granulated with starch paste is dried, screened, and mixed with the magnesium stearate. The mixture is compressed into tablets weighing 110 mg each.

EXAMPLE 101

An illustrative composition for an injectable suspension is the following 1 ml ampul for an intramuscular injection:-

| | | Weight per cent |
|---|---|---|
| (a) | active compound | 1.0 |
| (b) | polyvinylpyrrolidone | 0.5 |
| (c) | lecithin | 0.25 |
| (d) | water for injection to make | 100.0 |

The materials (a)-(d) are mixed, homogenized, and filled into 1 ml ampuls which are sealed and autoclaved 20 minutes at 121°C. Each ampul contains 10 mg per ml of novel compound (a).

EXAMPLE 102

| | mg/suppository |
|---|---|
| Active Compound | 50 |
| Oil of Theobroma | 950 |

The medicament is powdered and passed through a B.S. No. 100 Sieve and triturated with molten oil of Theobroma at 45°C to form a smooth suspension. The mixture is well stirred and poured into moulds each of nominal 1G capacity, to produce suppositories.

## CLAIMS

1. A compound of the following general Formula 1:-

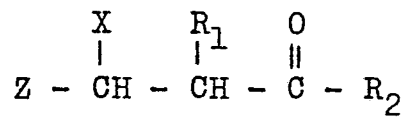

Formula 1

wherein:-

Z represents a cyano or a carbonyl-terminated electron-withdrawing group;

X represents ethynyl, monofluoromethyl, difluoromethyl or trifluoromethyl;

$R_1$ represents hydrogen, $C_1$-$C_7$ alkyl, or phenyl-$C_1$-$C_7$ alkyl; and

$R_2$ represents an aminocarboxylic acid residue obtained by removal of a hydrogen atom from the amino group thereof,

and salts, amides and esters thereof.

2. A compound as claimed in Claim 1 wherein X represents ethynyl.

3. A compound as claimed in Claim 1 wherein X represents monofluoromethyl, difluoromethyl or trifluoromethyl.

4. A compound as claimed in any one of the preceding Claims wherein Z represents cyano.

5. A compound as claimed in any one of Claims 1 to 4 wherein Z represents cyano or a carbonyl-terminated group of the formula -COR wherein:-

R represents

$-R_{10}$, $-OR_{10}$, 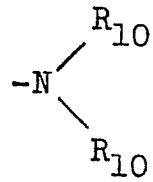, $-NHOR_{10}$, $-SR_{10}$,

$-R_2$, - 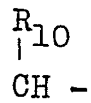 - $R_{11}$, or $-CO_2H$

$R_2$ is as defined in Claim 1;

$R_{10}$ represents hydrogen, $C_1$-$C_7$ alkyl, phenyl-$C_1$-$C_7$ alkyl, phenyl or p-methoxyphenyl;

$R_{11}$ represents

halogen, $-OR_{10}$, 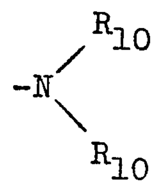, $-O.CO.R_{10}$,

$-SR_{10}$, tosyloxy, or $-NH.CO.R_{12}$;

$R_{12}$ represents $C_1$-$C_7$ alkyl, phenyl-$C_1$-$C_7$ alkyl, phenyl, - 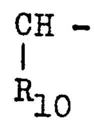 - $NH.CO.R_{12}$, 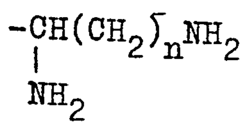

or 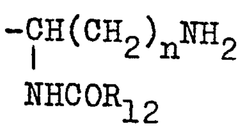 ; and

n represents 3 or 4.

6. A compound as claimed in any one of the preceding Claims having the following general Formula 2:-

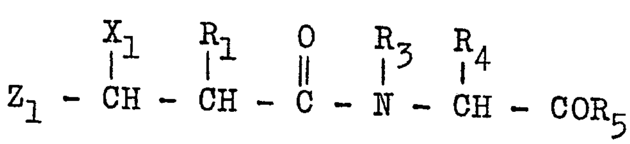 Formula 2

wherein:-

$Z_1$ represents cyano or a carbonyl-terminated electron-withdrawing group;

$X_1$ represents ethynyl or mono-, di- or tri-fluoromethyl;

$R_1$ represents hydrogen, $C_1$-$C_7$ alkyl or phenyl-$C_1$-$C_7$ alkyl;

$R_3$ and $R_4$ represent hydrogen or the side chain of a naturally occurring mammalian amino acid of the formula $\overset{R_3}{\overset{|}{HN}} - \overset{R_4}{\overset{|}{CH}} - CO_2H$; and

$R_5$ represents hydroxy, $C_1$-$C_7$ alkoxy, primary amino or

$- \overset{R_3}{\overset{|}{N}} - \overset{R_4}{\overset{|}{CH}} - COR_5$ in which $R_3$, $R_4$ and $R_5$ are as defined above,

and pharmaceutically acceptable salts thereof. -

7. A compound as claimed in any one of Claims 1 to 5 having the following general Formula 5:-

$$Z_1 - \overset{X_1}{\overset{|}{CH}} - \overset{R_1}{\overset{|}{CH}} - \overset{O}{\overset{\|}{C}} - \overset{R_3'}{\overset{|}{N}} - \overset{R_4'}{\overset{|}{CH}} - COR_5' \qquad \text{Formula 5}$$

wherein:-

$Z_1$ represents cyano or an electron-withdrawing carbonyl-terminated functional group;

$X_1$ represents ethynyl or mono-, di- or tri-fluoromethyl;

$R_1$ represents hydrogen, $C_1$-$C_7$ alkyl or

phenyl-$C_1$-$C_7$ alkyl;

$R_3'$ represents hydrogen and $R_4'$ represents hydrogen or $C_1$-$C_4$ alkyl and optionally substituted by indole, by imidazole or by phenyl, or

$R_3'$ and $R_4'$ together represent alkylene or alkenylene, said alkylene and alkenylene having 2, 3 or 4 chain carbon atoms and being optionally substituted by hydroxy, by $C_1$-$C_7$ alkyl or, in the hydrocarbon chain, by a sulfur atom; and

$R_5'$ represents hydroxy, $C_1$-$C_7$ alkoxy, primary amino, or

$$\begin{array}{ccc} & R_3' & & R_4' \\ & | & & | \\ - & N & - & CH & - COR_5' \end{array}$$

in which $R_3'$, $R_4'$ and $R_5'$ are as defined above,

and pharmaceutically acceptable salts thereof.

8. A compound as claimed in any one of the preceding Claims wherein RCO- represents $-COR_{10}'$, -CONHOH or $-COCH(R_{10}")NHCOR_{10}'$ wherein $R_{10}'$ represents $C_1$-$C_7$ alkyl, phenyl or phenyl-$C_1$-$C_7$ alkyl and $R_{10}"$ represents $C_1$-$C_7$ alkyl or phenyl-$C_1$-$C_7$ alkyl.

9. A compound as claimed in Claim 8 wherein $R_{10}'$ represents $C_1$-$C_4$ alkyl, phenyl or benzyl and $R_{10}"$ represents $C_1$-$C_4$ alkyl or benzyl.

10. A compound as claimed in any one of the preceding Claims having the following general Formula

8:-

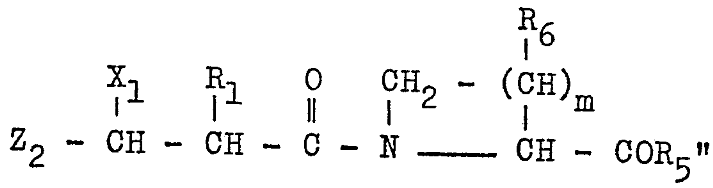 Formula 8

wherein:-

$Z_2$ represents cyano, $-COR_{10}'$, -CO.NH.OH or 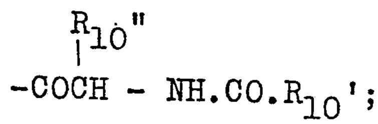

$X_1$ represents ethynyl or mono-, di- or tri-fluoromethyl;

$R_1$ represents hydrogen, $C_1$-$C_7$ alkyl or phenyl-$C_1$-$C_7$ alkyl;

$R_5''$ represents hydroxy, $C_1$-$C_7$ alkoxy or primary amino;

<u>m</u> represents 1, 2 or 3;

each $R_6$ independently represents hydrogen, hydroxy or $C_1$-$C_7$ alkyl or, when <u>m</u> represents 2 or 3, $R_6$ in each of two adjacent carbon atoms together represent a second valency bond between those carbon atoms;

$R_{10}'$ represents $C_1$-$C_7$ alkyl, phenyl-$C_1$-$C_7$ alkyl or phenyl; and

$R_{10}''$ represents $C_1$-$C_7$ alkyl or phenyl-$C_1$-$C_7$ alkyl,

and pharmaceutically acceptable salts thereof.

11. A compound as claimed in Claim 10 having the following general Formula 11:-

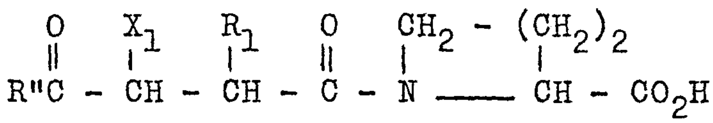 Formula 11

wherein:-

$X_1$ represents ethynyl or mono-, di- or tri-fluoromethyl;

R" represents 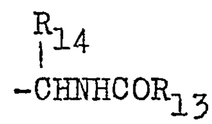 or -NHOH

$R_1$ represents hydrogen, $C_1$-$C_4$ alkyl or phenyl-$C_1$-$C_4$ alkyl,

$R_{13}$ represents $C_1$-$C_7$ alkyl or phenyl, and

$R_{14}$ represents benzyl or sec.butyl (i.e. $-CH(CH_3)CH_2CH_3$)

and pharmaceutically acceptable salts thereof.

12. A pharmaceutical composition comprising a compound as claimed in any one of the preceding Claims as an active ingredient in admixture or otherwise associated with a pharmaceutically acceptable diluent or carrier.

13. A compound as claimed in any one of Claims 1 to 11 for use in inhibiting angiotensin-converting enzyme, enkephalinase or, when $R_1$ represents hydrogen, delta-aminolevulinic acid dehydrase in a patient.

14. A compound of the following general Formula 21:-

$$C_6H_5CH_2 - CO - CH - CO_2 - A \qquad \text{Formula 21}$$

wherein A represents tert-butyl, dibenzyl or trityl.

15. A compound of the following general Formula 36:-

$$H_2N-\overset{R_{10}}{\overset{|}{C}}H.OC-\overset{X_1}{\overset{|}{C}}H-\overset{R_1}{\overset{|}{C}}H-COR_9 \qquad \text{Formula 36}$$

wherein:-

$X_1$ represents ethynyl or mono-, di- or tri-fluoromethyl;

$R_1$ represents hydrogen, $C_1$-$C_7$ alkyl or phenyl-$C_1$-$C_7$ alkyl,

$R_9$ represents hydroxy, $C_1$-$C_7$ alkoxy or amino, and

$R_{10}$ represents hydrogen, $C_1$-$C_7$ alkyl, phenyl-$C_1$-$C_7$ alkyl, phenyl or p-methoxyphenyl.

16. A pharmaceutical composition comprising a compound according to Claim 15 as an active ingredient in admixture or otherwise associated with a pharmaceutically acceptable diluent or carrier.

17. A compound as claimed in Claim 10 for use in inhibiting delta-aminolevulinic acid dehydrase in a patient.